# EUROPEAN PATENT APPLICATION

(11) **EP 4 527 834 A1**
(43) Date of publication of application: **26.03.2025**
(21) Application number: 23807528.7
(22) Date of filing: 11.05.2023
(51) Int. Cl.: C07D 303/34, C07D 405/14, C08G 59/02

(54) **GLYCIDYL ETHER GROUP-CONTAINING COMPOUND, CURABLE RESIN COMPOSITION, CURED OBJECT, AND LAMINATE**

(30) Priority: 17.05.2022 JP 2022080768
(71) Applicant: DIC Corporation, Tokyo 174-8520 (JP)
(72) Inventor: SUZUKI, Etsuko, Sakura-shi, Chiba 285-8668 (JP); ASANO, Yuu, Sakura-shi, Chiba 285-8668 (JP); OTSU, Masato, Sakura-shi, Chiba 285-8668 (JP); ARITA, Kazuo, Sakura-shi, Chiba 285-8668 (JP)
(74) Representative: TBK
(86) International application number: PCT/JP2023/017675
(87) International publication number: WO 2023/223924

(57) **Abstract**

There is provided a compound which, while being a curable resin, can easily exhibit repairability and remoldability when being in the form of a cured product, a curable resin composition obtained using the same, and a cured product thereof. A glycidyl ether group-containing compound is used, in which a structural unit A having one or more glycidyl ether groups and a structural unit B different from the structural unit A are linked in A-B-A, and the structural unit A and the structural unit B are bonded by a reversible bond having a dissociation temperature of 120°C or higher. This reversible bond is preferably an anthracene-type addition-type structure formed by a Diels-Alder reaction or a disulfide bond sandwiched between aromatic rings.

## Description

### TECHNICAL FIELD

The present invention relates to a glycidyl ether group-containing compound having a specific structure, a curable resin composition containing the same, a cured product, and a laminate containing a layer formed of the cured product.

### BACKGROUND ART

Cured products obtained from epoxy resins have excellent heat resistance, mechanical strength, electrical properties, adhesive properties, and the like, and are essential materials in various fields such as electrical and electronics, paints, and adhesives.

On the other hand, cured products using thermosetting resins such as epoxy resins have low long-term reliability. For example, when a cured product of an epoxy resin deteriorates due to oxidation, cracks may occur.

In addition, the cured product obtained by curing a thermosetting resin such as an epoxy resin is not soluble in solvents (insoluble) and do not melt even at high temperatures (unmeltable), thus is poor in recyclability or reusability, and the cured product becomes waste after use, making it a challenge to reduce waste and reduce the burden on the environment.

Therefore, a cured product using an epoxy resin or the like is required to solve the problems of prolonging life and waste reduction, and it is considered effective to add easy disassembly, and repairability and remoldability to the cured product to solve these problems.

Against this background, a method has been disclosed in which a compound having thermal degradability is blended in advance with a reactive adhesive component, and then, after use, the adhesive strength is reduced by a certain amount of heating to enable disassembly (see, for example, Patent Document 1).

Further, even if cracks or delamination occur in an encapsulant using an epoxy resin or the like, a method of making a self-healing encapsulant by using microcapsule particles encapsulating a first thermosetting resin and a second thermosetting resin precursor material is disclosed (see, for example, Patent Document 2).

In addition to the above, there has been much research on the use of reversible bonds such as dynamic covalent bonding and supramolecular bonding in a cured product to impart repairability and remoldability.

### CITATION LIST

### PATENT LITERATURE

Patent Document 1:JP-A-2013-256557
Patent Document 2:JP-A-2017-041496

### SUMMARY OF INVENTION

### TECHNICAL PROBLEM

In the technology provided in Patent Document 1, the adhesive is disposed of after disassembly, and although the base material to be adhered to is recyclable, the overall recyclability of the adhesive is insufficient. Further, the technology disclosed in Patent Document 2 has a certain degree of self-healing property, but is not a solution from the viewpoint of reuse, and the problem of waste when the adhesive is no longer needed remains. In addition, since it is necessary to ensure the molecular mobility of the raw materials involved in the reversible bond, there is a problem that the raw materials to be used are limited to gel-like substances with poor mechanical strength, and improvements are currently required in all of these areas. Therefore, the object of the present invention is to provide a compound that can easily achieve repairability and remoldability in a cured product while being a curable resin, and a curable resin composition using the compound, and a cured product made from the compound.

### SOLUTION TO PROBLEM

As a result of diligent study, the present inventors found that the above problems can be solved by using a glycidyl ether group-containing compound having a specific structure and using the compound as a curable resin composition, and completed the invention.

That is, the present invention includes the following aspects.
[1] A glycidyl ether group-containing compound in which a structural unit A having one or more glycidyl ether groups and a structural unit B different from the structural unit A are linked in A-B-A, in which
   the structural unit A and the structural unit B are bonded by a reversible bond having a dissociation temperature of 120°C or higher.
[2] The glycidyl ether group-containing compound according to [1], in which
   the reversible bond is a covalent reversible bond.
[3] The glycidyl ether group-containing compound according to [2], in which
   the reversible bond is any one of an addition-type structure by a Diels-Alder reaction, a disulfide bond, an ester bond, a boronic acid ester bond, a hemiaminal bond, an imine bond, an acylhydrazone bond, an olefin metathesis reaction, an alkoxyamine skeleton, and an amide bond, all of which have a dissociation temperature of 120°C or higher.
[4] The glycidyl ether group-containing compound according to [2] or [3], in which
   the reversible bond is an anthracene-type addition-type structure by a Diels-Alder reaction or a disulfide bond sandwiched between aromatic rings.
[5] The glycidyl ether group-containing compound according to any one of [1] to [4], in which
   the structural unit B has an alkylene chain or an alkylene ether chain.
[6] The glycidyl ether group-containing compound according to [5], in which
   the alkylene chain has 4 to 16 carbon atoms.
[7] The glycidyl ether group-containing compound according to any one of [1] to [6], in which
   the structural unit B further has a reversible bond that is the same as the reversible bond having a dissociation temperature of 120°C or higher, which is a linkage site between the structural unit A and the structural unit B.
[8] A glycidyl ether group-containing compound represented by the following general formula: 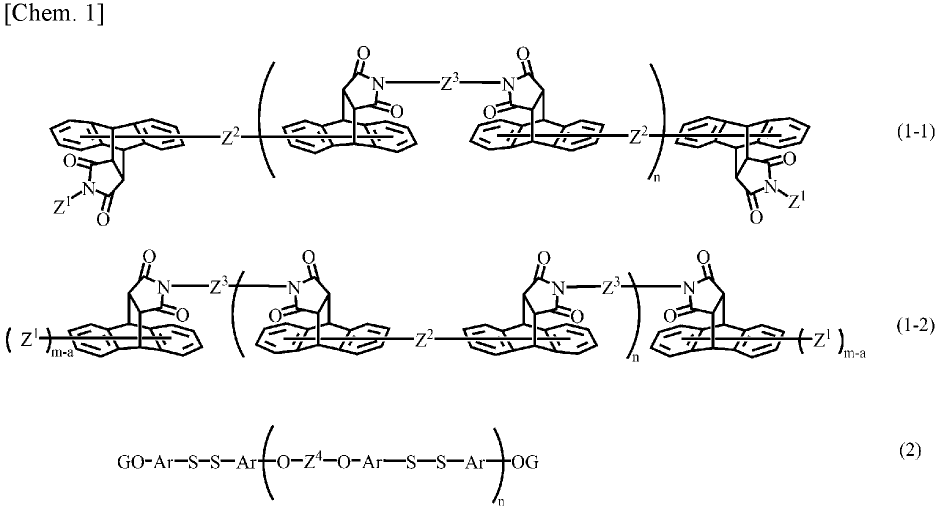 [in the formula (2), Ar's are each independently a structure containing an unsubstituted aromatic ring or an aromatic ring having a substituent, and the anthracene-derived structure in formulas (1-1) and (1-2) may have a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group as a substituent. G is a glycidyl group or a 2-methylglycidyl group, in the formula, m-a is an integer of 1 to 10, and n is an average value of repetition number of 0 to 10, Z¹ is any of the structures represented by the following formula (3), Z² is any of the structures represented by the following formula (4), Z³ is any of the structures represented by the following formula (5), Z⁴ is any of the structures represented by the following formula (6) or (7), and the multiple structures present in one molecule may be identical or different from each other, 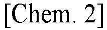 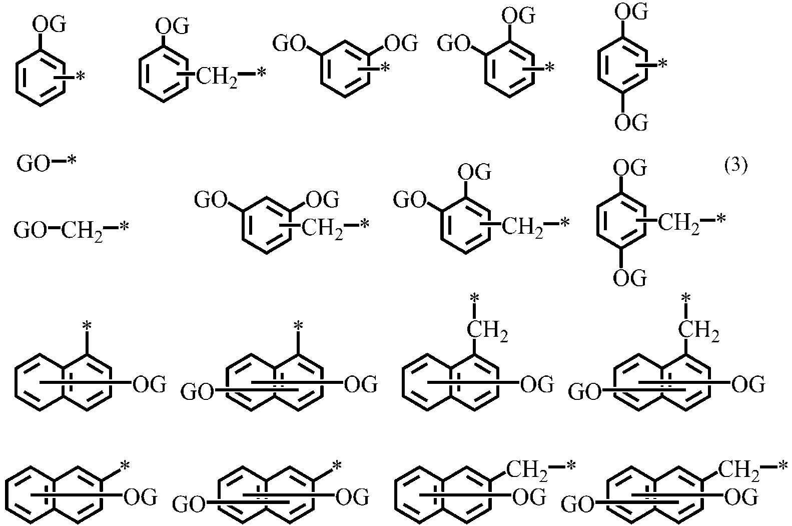
   [the aromatic ring in the formula (3) may be substituted or unsubstituted, and * denotes a binding point,
   G is the same as above, and
   the -OG on the naphthalene ring in the formula indicates that the glycidyl ether group may be bonded at any point], 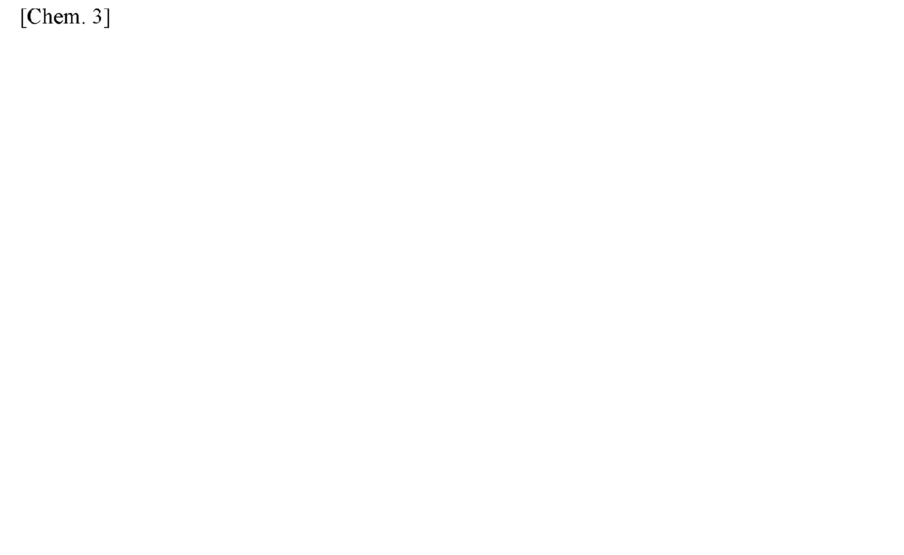 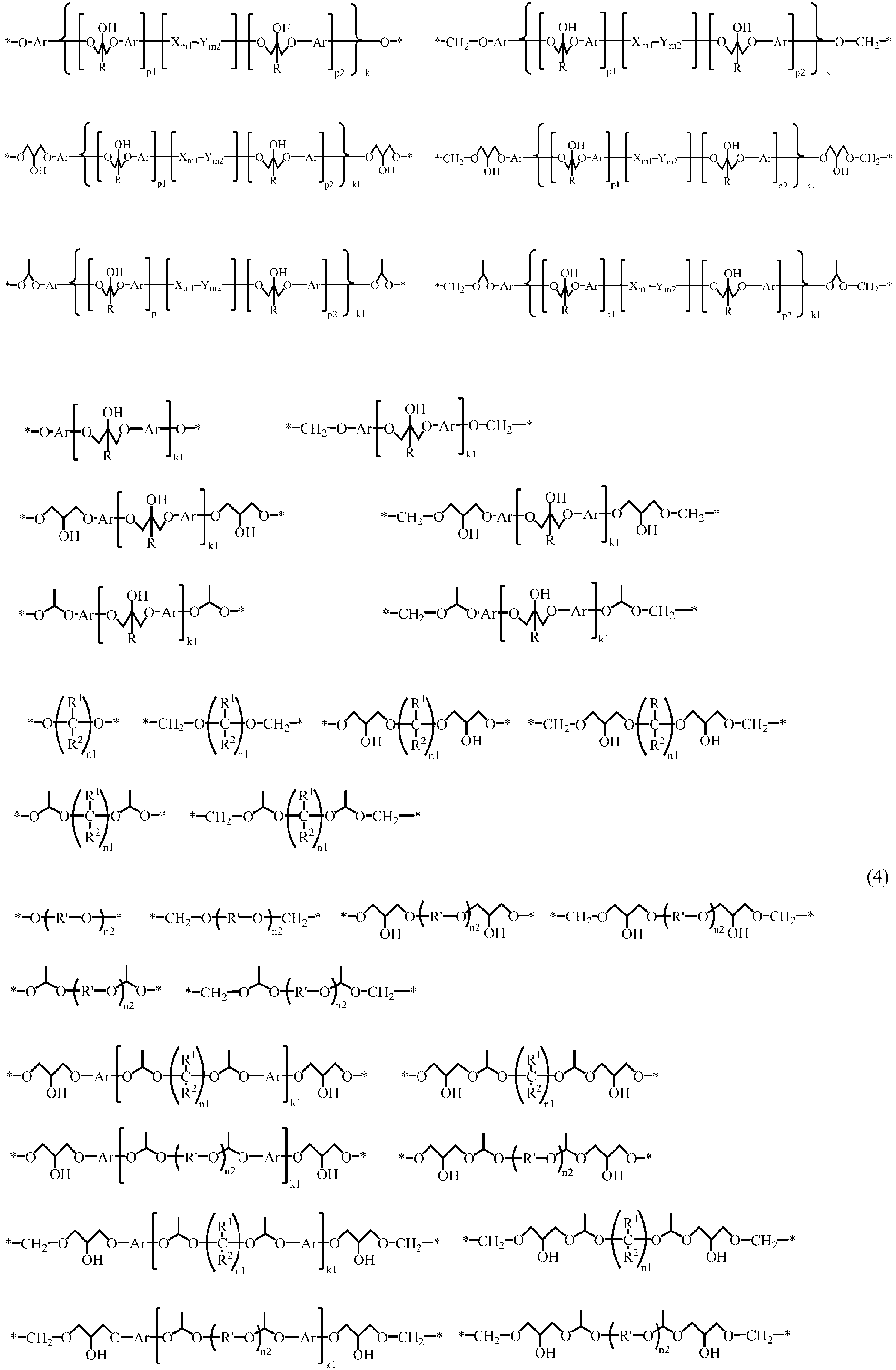
   [in the formula (4),
   Ar's are each independently a structure containing an unsubstituted aromatic ring or an aromatic ring having a substituent,
   R¹ and R² are each independently a hydrogen atom, a methyl group, or an ethyl group,
   R is a hydrogen atom or a methyl group,
   R' is a divalent hydrocarbon group having 2 to 12 carbon atoms,
   n1 is an integer of 2 to 16, and n2 is an average number of repeating units of 2 to 30,
   k1 is an average value of repetition number, from 0.5 to 10,
   p1 and p2 are each independently 0 to 5, and
   X is a structural unit represented by the following formula (4-1), and Y is a structural unit represented by the following formula (4-2), 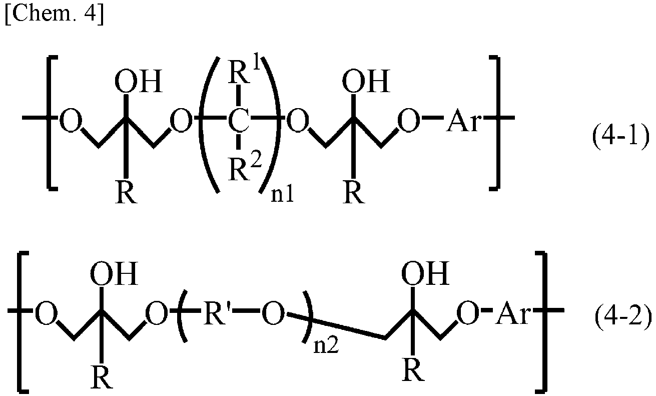
   [in the formulas (4-1) and (4-2), Ar, R, R¹, R², R', n1, and n2 are the same as above],
   m1 and m2 are an average value of repetition number, each independently 0 to 25, and m1 + m2 ≥ 1.
   provided that a bond between the structural unit X represented by the formula (4-1) and the structural unit Y represented by the formula (4-2) may be random or blocked, and the respective total numbers of structural units X and Y present in one molecule are m1 and m2, respectively], 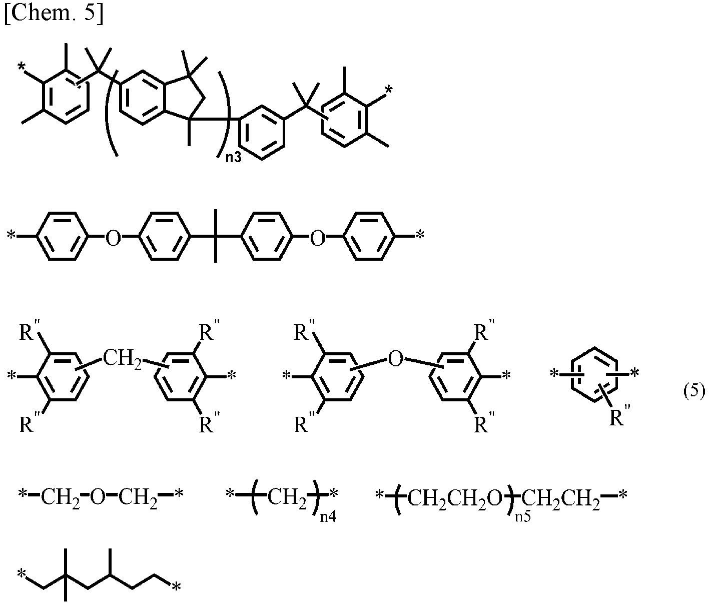
   [in the formula (5), n3 and n5 are an average value of repetition number and are each 0.5 to 10, n4 is an integer of 1 to 16, and R"'s are each independently a hydrogen atom, a methyl group, or an ethyl group], 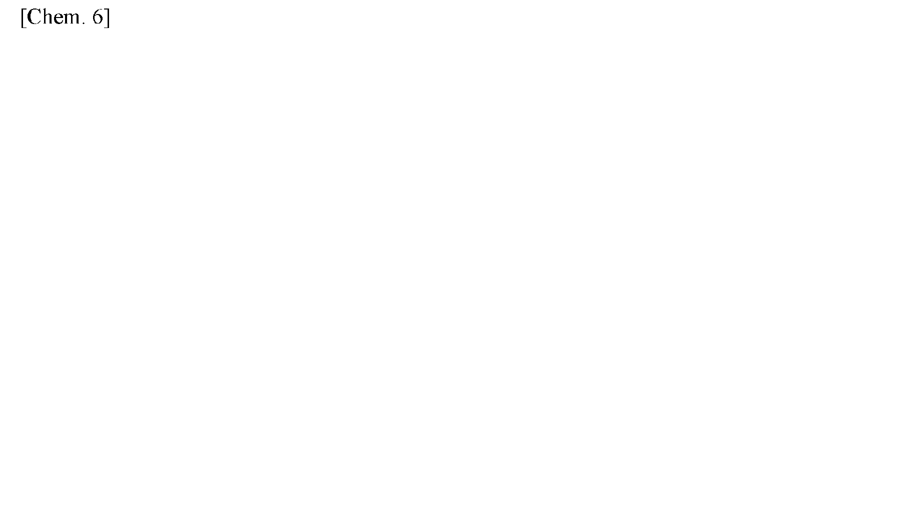 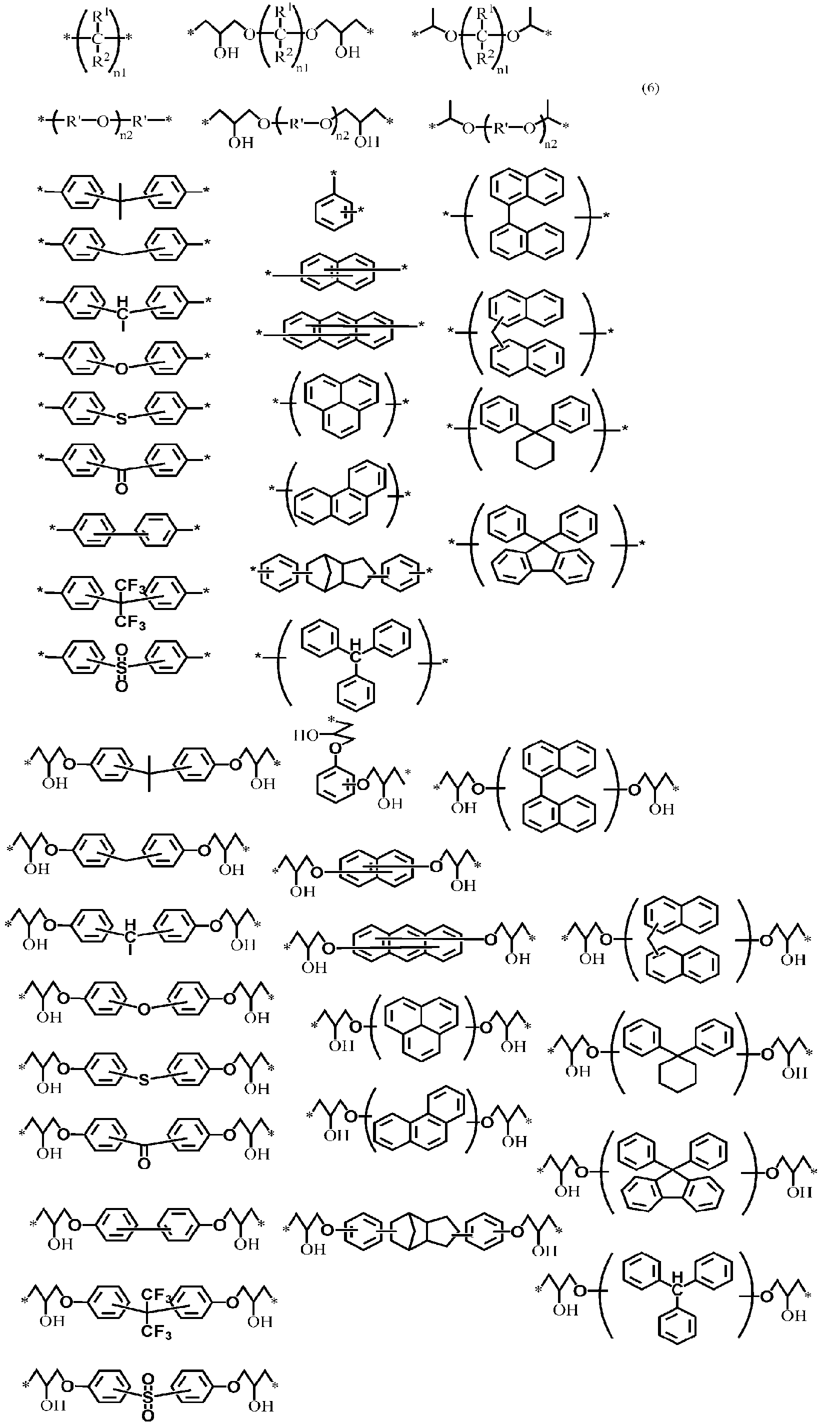 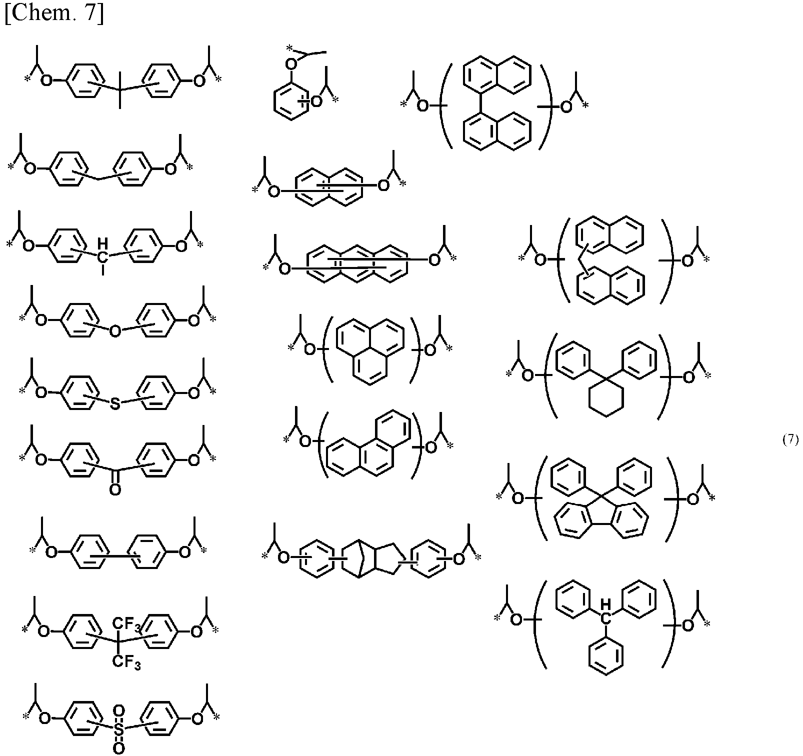
   [in the formulas (6) and (7), R¹, R², R', n1, and n2 are the same as above].
[9] A curable resin composition containing, as essential components:
   the glycidyl ether group-containing compound according to any one of [1] to [8]; and
   a compound (I) having reactivity with the glycidyl ether group-containing compound.
[10] The curable resin composition according to [9], in which the compound (I) having reactivity with the glycidyl ether group-containing compound is a hydroxyl group-containing compound.
[11] The curable resin composition according to [10], in which the compound (I) having reactivity with the glycidyl ether group-containing compound is a hydroxyl group-containing compound having a reversible bond.
[12] The curable resin composition according to [11], in which the hydroxyl group-containing compound having a reversible bond is a hydroxyl group-containing compound represented by the following general formula: 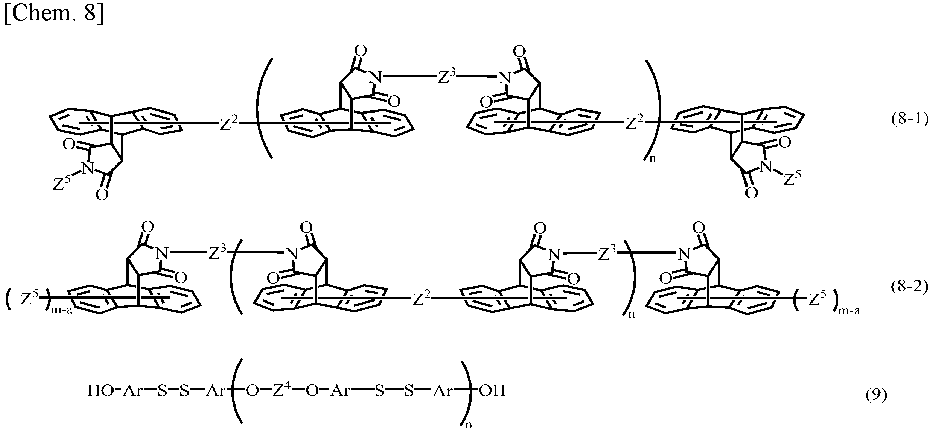
   [in the formula (9), Ar's are each independently a structure containing an unsubstituted aromatic ring or an aromatic ring having a substituent, and the anthracene-derived structure in the formulas (8-1) and (8-2) may have a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group as a substituent,
   in the formula, m-a is an integer of 1 to 10, and n is an average value of repetition number of 0 to 10. Z⁵ is any of the structures represented by the following formula (10), Z² is any of the structures represented by the following formula (11), Z³ is any of the structures represented by the following formula (12), Z⁴ is any of the structures represented by the following formula (13) or (14), and the multiple structures present in one molecule may be identical or different from each other, 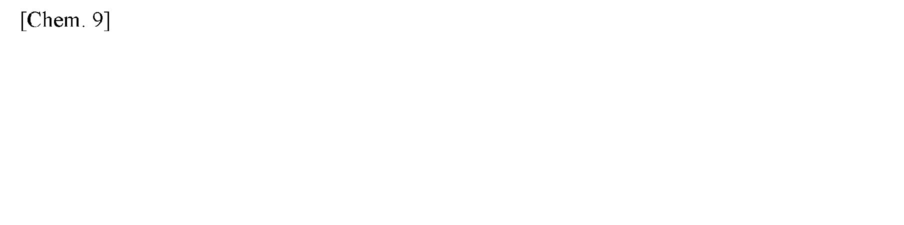 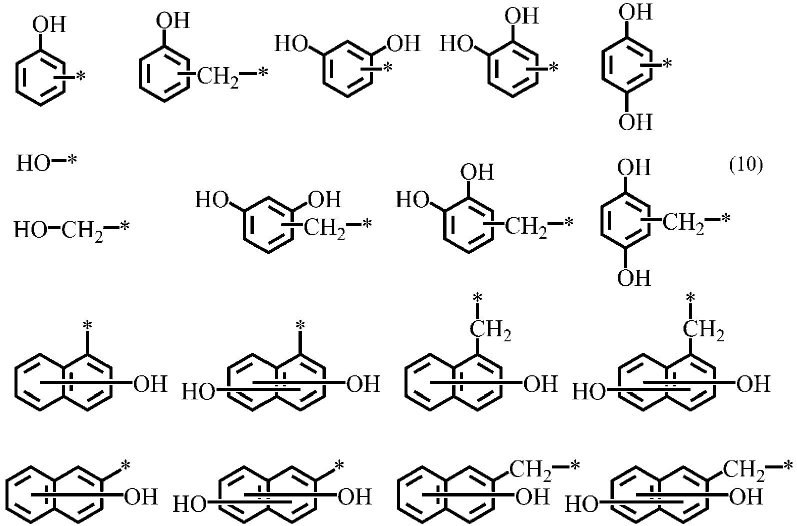
   the aromatic ring in the formula (10) may be substituted or unsubstituted, and * denotes a binding point, the hydroxyl group on the naphthalene ring in the formula indicates that the hydroxyl group may be bonded at any point], 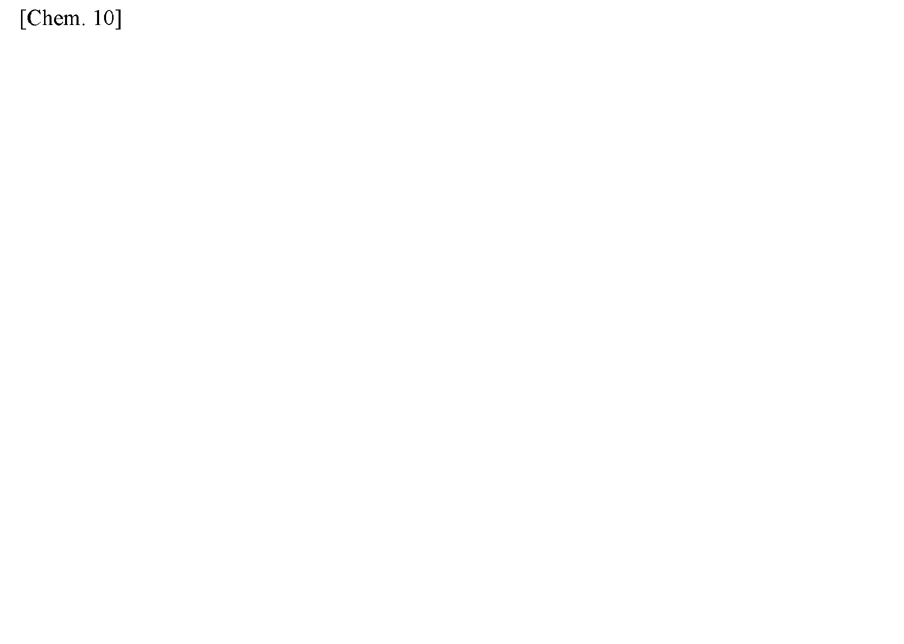 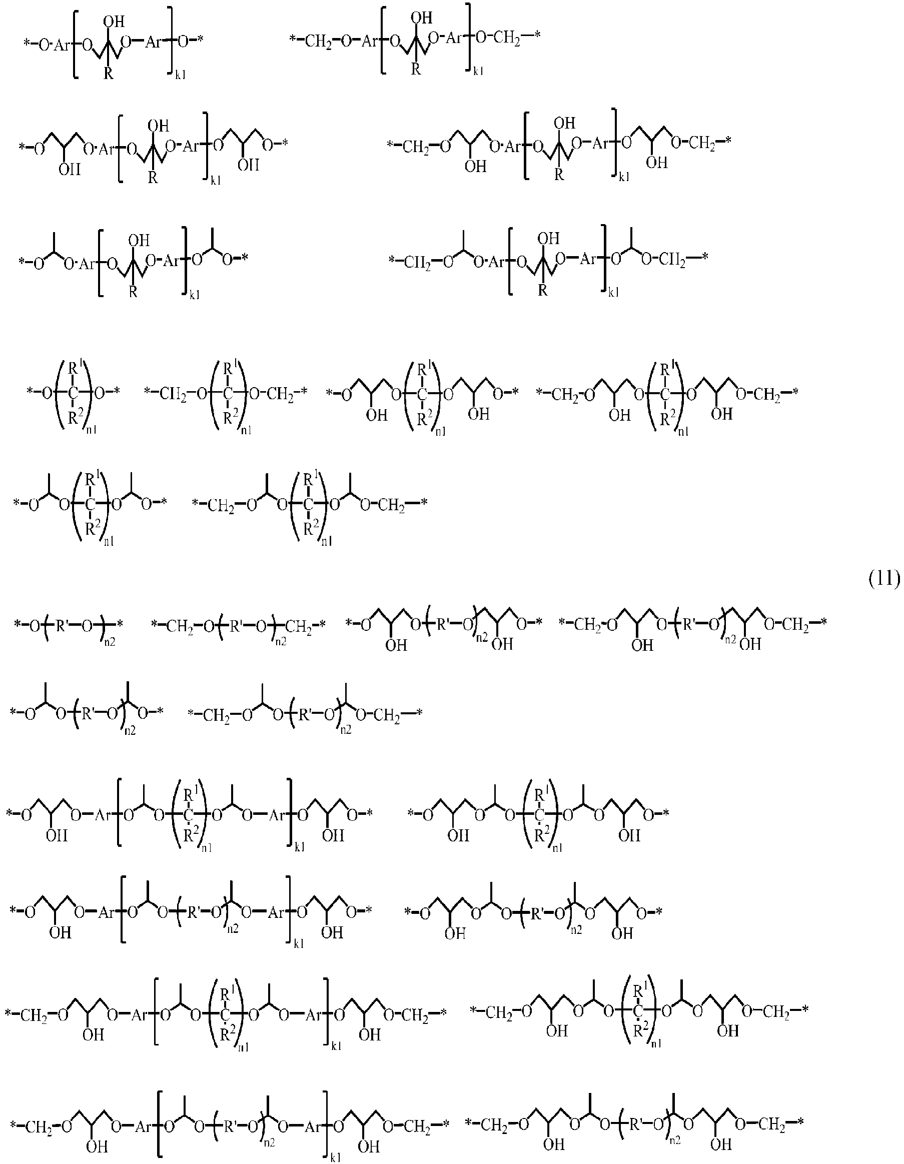
   [in the formula (11),
   Ar is the same as above,
   Ar's are each independently a structure containing an unsubstituted aromatic ring or an aromatic ring having a substituent,
   R¹ and R² are each independently a hydrogen atom, a methyl group, or an ethyl group,
   R is a hydrogen atom or a methyl group,
   R' is a divalent hydrocarbon group having 2 to 12 carbon atoms,
   n1 is an integer of 2 to 16, and n2 is an average number of repeating units of 2 to 30,
   k1 is an average value of repetition number, from 0.5 to 10,
   p1 and p2 are each independently 0 to 5, and
   X is a structural unit represented by the following formula (11-1), and Y is a structural unit represented by the following formula (11-2), 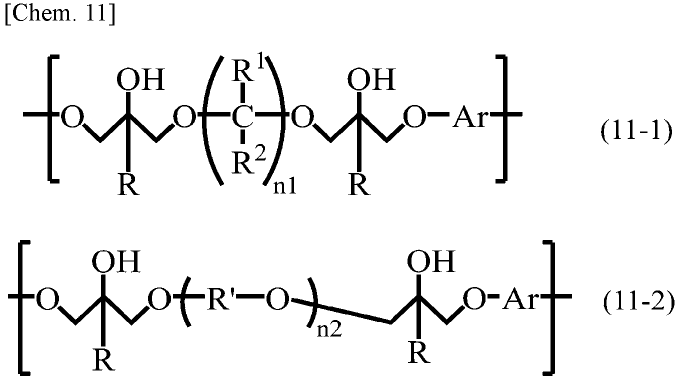
   [in the formulas (11-1) and (11-2), Ar, R, R¹, R², R', n1, and n2 are the same as above],
   m1 and m2 are an average value of repetition number, each independently 0 to 25, and m1 + m2 ≥ 1,
   Provided that a bond between the structural unit X represented by the formula (11-1) and the structural unit Y represented by the formula (11-2) may be random or blocked, and the respective total numbers of structural units X and Y present in one molecule are m1 and m2, respectively], 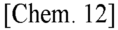 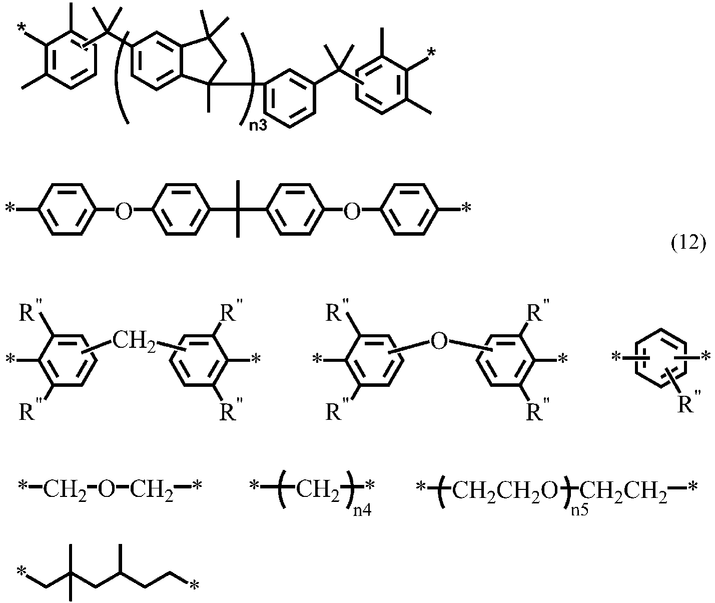
   [In the formula (12), n3 and n5 are an average value of repetition number and are each 0.5 to 10, n4 is an integer of 1 to 16, and R"'s are each independently a hydrogen atom, a methyl group, or an ethyl group], 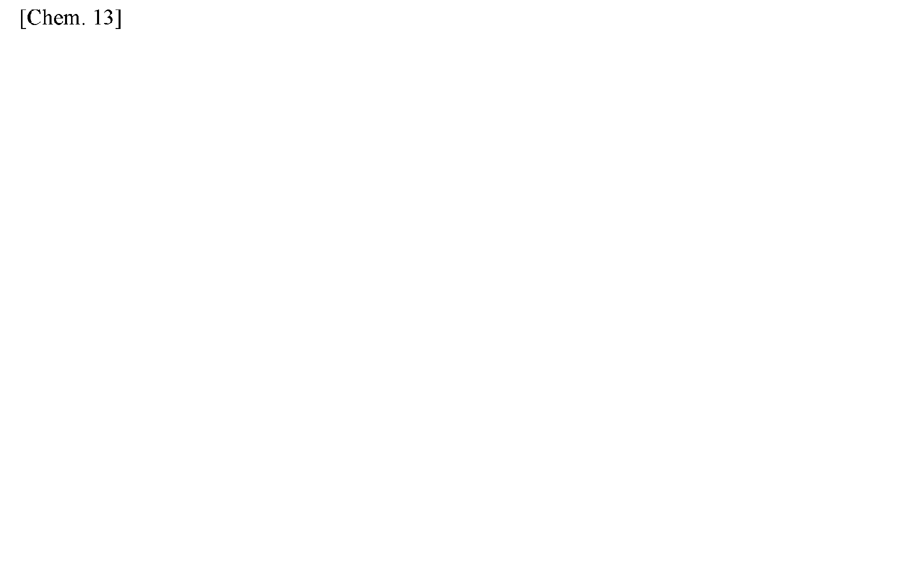 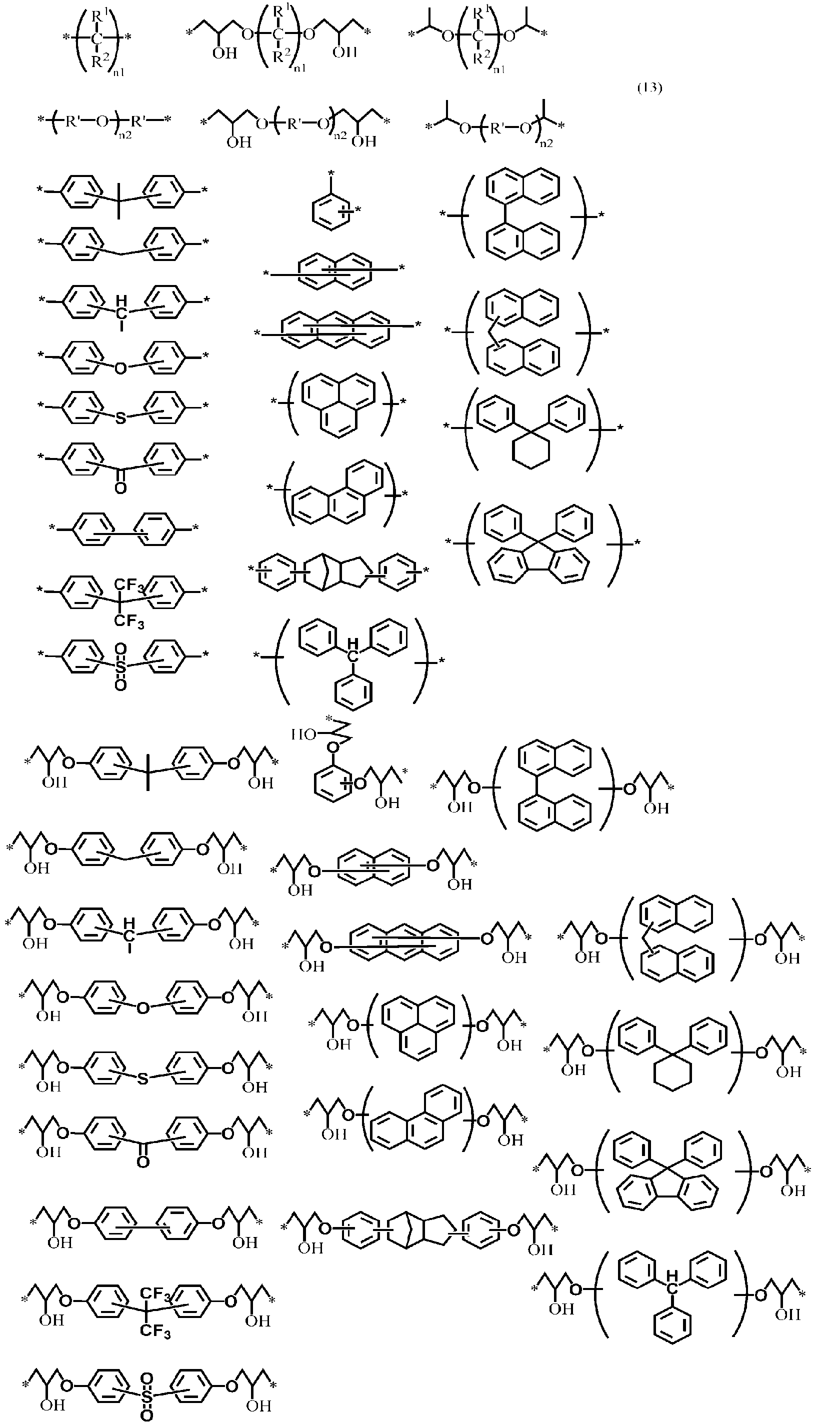 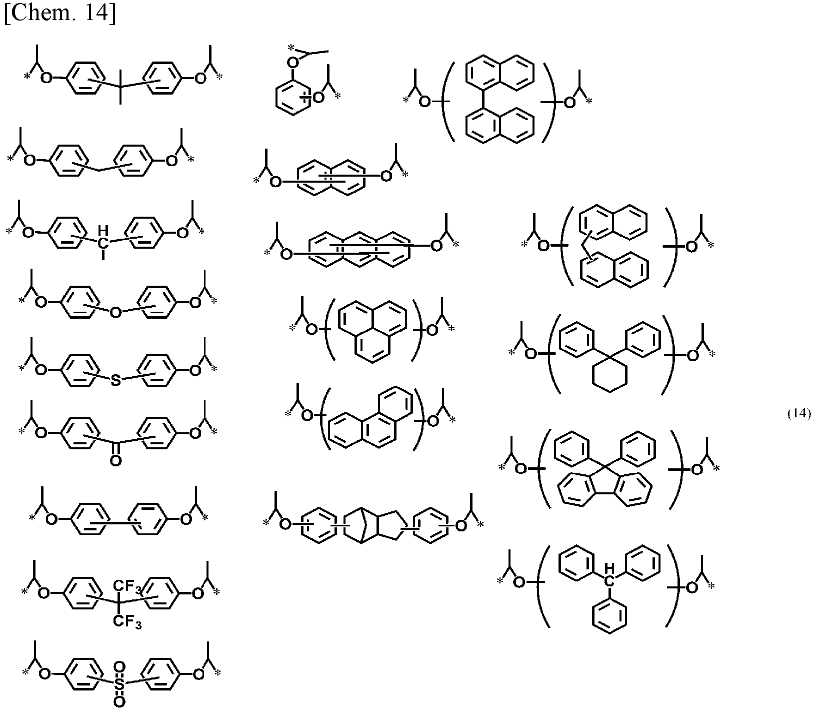
   [in the formulas (13) and (14), R¹, R², R', n1, and n2 are the same as above].
[13] The curable resin composition according to any one of [9] to [12], further containing:
   an epoxy resin with an epoxy equivalent weight of 100 to 10,000 g/eq, other than the glycidyl ether group-containing compound according to any one of [1] to [8].
[14] The curable resin composition according to [13], in which
   the epoxy resin is represented by the following formula (15) and has an epoxy equivalent weight of 500 to 10000 g/eq: 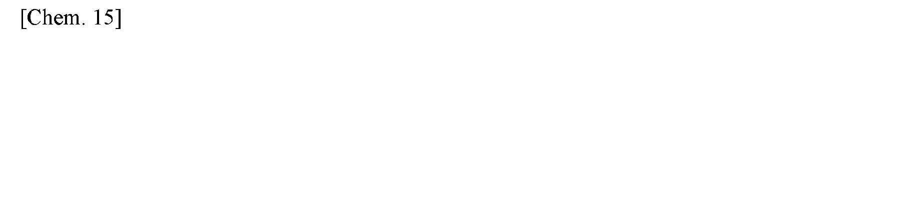 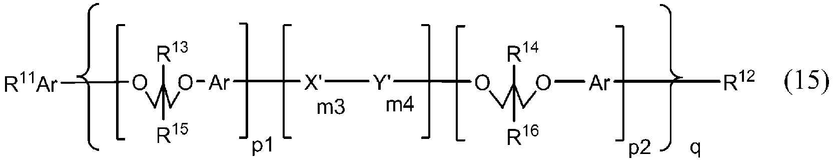
   [in the formula (15), Ar's are each independently a structure containing an unsubstituted aromatic ring or an aromatic ring having a substituent,
   X' is a structural unit represented by the following formula (15-1), and Y' is a structural unit represented by the following formula (15-2), 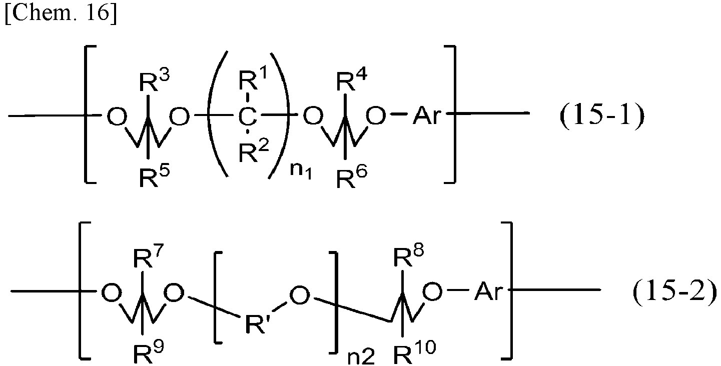
   [in the formulas (15-1) and (15-2), Ar is the same as above,
   R¹ and R² are each independently a hydrogen atom, a methyl group, or an ethyl group,
   R' is a divalent hydrocarbon group having 2 to 12 carbon atoms,
   R³, R⁴, R⁷, and R⁸ are each independently a hydroxyl group, a glycidyl ether group, or a 2-methylglycidyl ether group,
   R⁵, R⁶, R⁹, and R¹⁰ are each independently a hydrogen atom or a methyl group,
   n₁ is an integer of 4 to 16, and
   n₂ is an average number of repeating units of 2 to 30]
   R¹¹ and R¹² are each independently a glycidyl ether group or a 2-methyl glycidyl ether group,
   R¹³ and R¹⁴ are each independently a hydroxyl group, a glycidyl ether group, or a 2-methyl glycidyl ether group,
   R¹⁵ and R¹⁶ are hydrogen atoms or methyl groups,
   m3, m4, p1, p2, and q are an average value of repetition number,
   m3 and m4 are each independently 0 to 25 and m3 + m4 ≥ 1,
   p1 and p2 are each independently 0 to 5, and
   q is 0.5 to 5,
   provided that a bond between the structural unit X' represented by the formula (15-1) and the structural unit Y' represented by the formula (15-2) may be random or blocked, and the respective total numbers of structural units X and Y present in one molecule are m3 and m4, respectively).
[15] The curable resin composition according to any one of [9] to [14], in which
   a concentration of the reversible bond to a total mass of a curable component in the curable resin composition is 0.10 mmol/g or more.
[16] The curable resin composition according to any one of [9] to [15], in which
   the curable resin composition is a self-healing composition or a composition for a remolding material.
[17] A cured product obtained by curing the curable resin composition according to any one of [9] to [16].
[18] A laminate containing:
   a base material; and
   a layer containing the cured product according to [17].
[19] A heat-resistant member containing:
   the cured product according to [17].
[20] A method for producing a glycidyl ether group-containing compound, the method including:
   synthesizing a glycidyl ether group-containing compound represented by the formulas (1-1) and (1-2) in situ during a process of curing a compound (I) having reactivity with the glycidyl ether group-containing compound, by using an intermediate of a conjugated diene or a parent diene intermediate represented by the following general formulas (1-1)' and (1-2)': 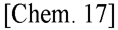 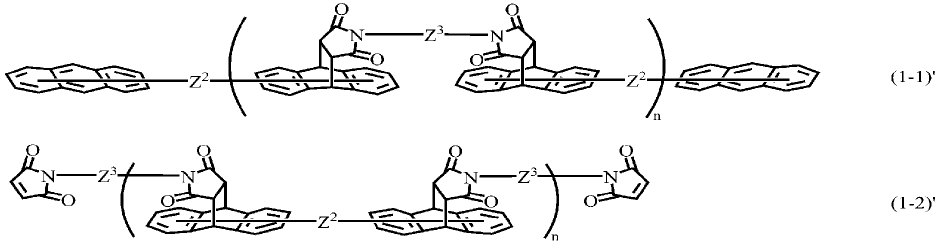 [in the formula, n, Z², and Z³ are the same as above].
[21] A cured product obtained by subjecting, to a curing reaction, the formula (1-1)', a maleimide having a glycidyl ether group, and a compound (I) having reactivity with a glycidyl ether group-containing compound as essential raw materials.
[22] A cured product obtained by subjecting a curing reaction, the formula (1-2), an anthracene having a glycidyl ether group, and a compound (I) having reactivity with the glycidyl ether group-containing compound as essential raw materials.

### ADVANTAGEOUS EFFECTS OF INVENTION

According to the present invention, a cured product made of a curable resin composition can be given repairability and remoldability, which can contribute to extending the service life of the cured product itself and reducing waste.

### DESCRIPTION OF EMBODIMENTS

Hereinafter, embodiments of the present invention will be described in detail. It should be understood that the present invention is not limited to the following embodiments, and design changes, improvements, and the like are appropriately added based on ordinary knowledge of those skilled in the art without departing from the gist of the present invention.

A glycidyl ether group-containing compound according to an embodiment of the present invention is a glycidyl ether group-containing compound in which a structural unit A having one or more glycidyl ether groups and a structural unit B different from the structural unit A are linked in A-B-A, and the structural unit A and the structural unit B are bonded by a reversible bond having a dissociation temperature of 120°C or higher. The glycidyl ether group in the present invention does not refer only to a glycidyl ether group without a substituent, but also includes a glycidyl ether group with a substituent on a carbon atom.

With such a configuration, the glycidyl ether group-containing compound is incorporated into the crosslinked structure by a curing reaction based on the glycidyl ether group thereof. On the other hand, by being reversible even after becoming a cured product, the structural unit B in particular can be present apart from the crosslinked structure, and thus has high molecular mobility even in the cured product. For this reason, when the cured product is subjected to impact such that the cured product cracks or is pulverized, the reversible bonds are likely to break. On the other hand, the reversible bonds can be reversibly remolded even in low temperature ranges, including room temperature, and can exhibit functions such as repairability and remoldability. The structural unit B is present away from the crosslinked structure and thus exhibits particularly high molecular mobility, indicating low-temperature repairability and low-temperature remolding. For example, even when the cured product obtained by using the glycidyl ether group-containing compound of the present invention is pulverized, it is possible to easily repair the cured product based on the reversible bond by placing the cured product at low temperatures, including room temperature, or in a warm or heated state, and it is also possible to remold the cured product after having the cured product pulverized.

The reversible bond having a dissociation temperature of 120°C or higher include a covalent bond or a non-covalent bond, and a covalent bond is preferable from the viewpoint of durability of the cured product. On the other hand, a non-covalent bond is preferable from the viewpoint of short repair and remolding times after pulverizing the cured product.

Examples of the covalent bond is not particularly limited, but include an addition-type structure by a Diels-Alder reaction, a disulfide bond, an ester bond, a boronic acid ester bond, a hemiaminal bond, an imine bond, an acylhydrazone bond, an olefin metathesis reaction, an alkoxyamine skeleton, and an amide bond. Among these, an anthracene-type (reversible bond consisting of an anthracene structure and a maleimide structure) addition-type structure by the Diels-Alder reaction and a disulfide bond sandwiched by aromatic rings are preferable from the viewpoint of heat resistance and hydrolysis resistance of the cured product.

Examples of the non-covalent bond is not particularly limited, but include a van der Waals force, an ionic bond, a clathrate bond of cyclodextrin, and a hydrogen bond such as a ureidopyrimidinone unit and a polyether thiourea.

The method using anthracene with a reactive functional group on the ring and maleimide with a reactive functional group is preferable for introducing the anthracene-type addition-type structure by the Diels-Alder reaction into the compound, because the production process is simple. A specific reversible bond partial structure can be represented by the following chemical formula. A reversible bond can be introduced into the compound by bonding with other structural units based on the R portion in the following formula in the maleimide-derived structure or various reactive functional groups on the ring in the anthracene-derived structure.

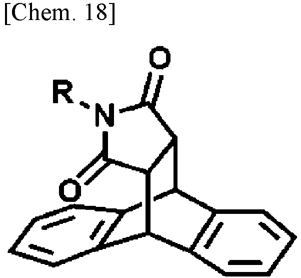

The Diels-Alder reaction is an addition reaction between a conjugated diene and a parent diene to form a 6-membered ring. Since the Diels-Alder reaction is an equilibrium reaction, a Retro-Diels-Alder reaction occurs at a predetermined temperature, resulting in dissociation (decrosslinking). At this time, when the temperature at which the Retro-Diels-Alder reaction occurs (dissociation temperature) is low, decrosslinking occurs in a high temperature range, resulting in a decrease in the crosslinking density of the cured product and a decrease in mechanical strength. Therefore, in the present invention, it is necessary that the Diels-Alder reaction units are a combination having high thermal stability, with a dissociation temperature of 120°C or higher. For example, the Diels-Alder reaction unit consisting of an anthracene structure and a maleimide structure has a high dissociation temperature of 250°C or higher and maintains the crosslinked structure without dissociation at least at 200°C or so, which is excellent for thermal stability. Therefore, the decrease in crosslinking density of the cured product can be suppressed and good mechanical strength can be maintained. When the obtained cured product is subjected to mechanical energy such as scratches or an external force, the C-C bond of the Diels-Alder reaction unit has lower bonding energy than that of a normal covalent bond, and thus the C-C bond of the Diels-Alder reaction unit will be preferentially broken. However, in the temperature range lower than the dissociation temperature, it is believed that the equilibrium of the C-C bond of the Diels-Alder reaction unit moves toward the binding direction, thus forming the adduct (Diels-Alder reaction unit) again and allowing damage repair and remolding.

Examples of the compound containing the disulfide bond include the following compounds. Similarly, by bonding various compounds via a site other than the disulfide binding site, for example, a hydroxyl group, an amino group, and a vinyl group, it is possible to incorporate the disulfide binding site into the glycidyl ether group-containing compound. Similarly, at this disulfide binding site, when the cured product is, for example, broken by an external force, the disulfide bond is preferentially broken, however, below the dissociation temperature, the equilibrium moves toward the binding direction, thus forming an S-S bond again and allowing damage repair and remolding.

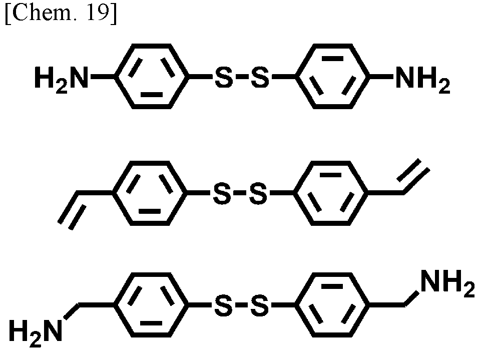

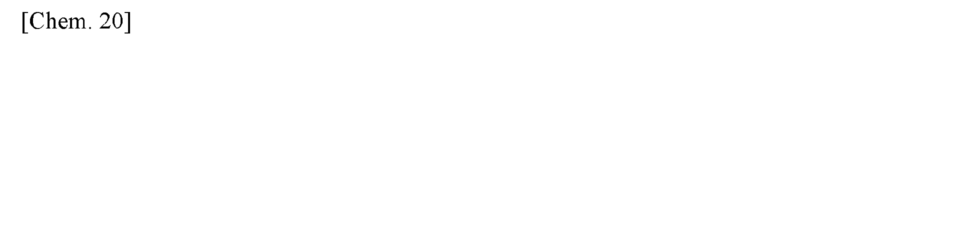

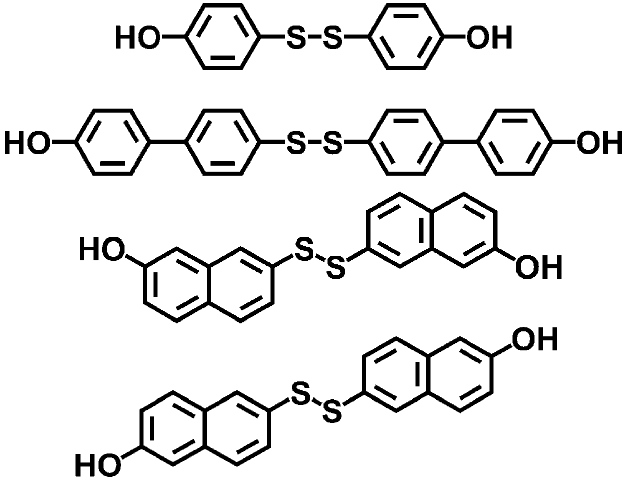

Examples of the compound containing the alkoxyamine skeleton include the following compounds. Similar to the above, the reversible bond can be incorporated into the glycidyl ether group-containing compound by binding with other compounds via a terminal vinyl group (methacryloyl group).

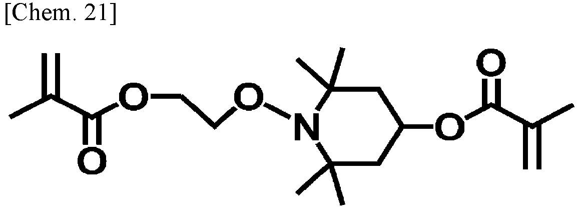

Although the the reversible bond described above will be present in at least two sites in the target glycidyl ether group-containing compound, it is preferable to have multiple reversible bonds of the same type as those between A and B in the structural unit B from the viewpoints of obtaining a structure with higher molecular mobility and facilitating adjustment of properties such as mechanical strength of the cured product.

In addition, for the same reason as above, the molecular weight as the structural unit B is preferably have a certain size or more, for example, the average molecular weight thereof (Mw) is preferably 28 or more. When there are reversible bonds in the structural unit B, the molecular weight between the reversible bonds is preferably 28 or more. A crosslinkable functional group similar to the glycidyl ether group in the structural unit A may be present in the structural unit B, but from the viewpoint of more easily expressing the effect of the present invention, it is preferable to have no crosslinkable (curable) functional group.

The structural unit B preferably contains an alkylene chain or an alkylene ether chain, from the viewpoint of enabling the cured product to exhibit greater flexibility or better conformability to a base material when the glycidyl ether group-containing compound of the present invention is used, for example, as a structural adhesive, and in this case, the alkylene chain more preferably contains 2 to 30 carbon atoms, and most preferably contains 4 to 16 carbon atoms. The alkylene ether chain is not particularly limited, but is preferably an alkylene ether chain having 2 to 12 carbon atoms, and the average value of repetition number is preferably in the range of 2 to 30.

The glycidyl ether group in the structural unit A may be any glycidyl ether group that can readily react with other functional groups, but from the viewpoint of availability of raw materials and curability, it is preferable to use a glycidyl ether group without a substituent on a carbon atom. Alternatively, the number of glycidyl ether groups in the structural unit A is not particularly limited, but from the viewpoints of industrial availability of raw materials and ease of adjustment of crosslinking density when a cured product is obtained, the number of glycidyl ether groups is preferably in the range of 1 to 3, and more preferably 1 to 2.

The average molecular weight (Mw) of the glycidyl ether group-containing compound is not particularly limited, but is preferably 500 or more and preferably 50,000 or less, from the viewpoint of achieving both of mechanical strength and flexibility, and repairability and remoldability when used as a cured product. In addition, when there are multiple reversible bonds other than between A and B, for example in the structural unit B, it is more preferable that the molecular weight per reversible bond be in the range of 300 to 10000 from the viewpoint of the remoldability of the cured product.

The glycidyl ether group-containing compound according to an embodiment of the present invention is a compound represented by the following general formula.

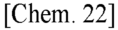

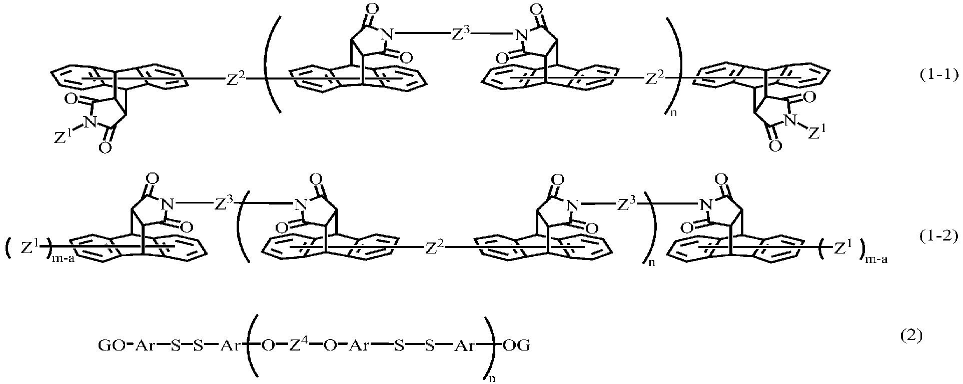

Ar's in the formula (2) are each independently a structure containing an unsubstituted aromatic ring or an aromatic ring having a substituent, and the anthracene-derived structure in the formulas (1-1) and (1-2) may have a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group as a substituent. G is a glycidyl group or a 2-methylglycidyl group. In the formula, m-a is an integer of 1 to 10, and n is an average value of repetition number of 0 to 10. Z¹ is any of the structures represented by the following formula (3), Z² is any of the structures represented by the following formula (4), Z³ is any of the structures represented by the following formula (5), Z⁴ is any of the structures represented by the following formula (6) or (7), and the multiple structures present in one molecule may be identical or different from each other, 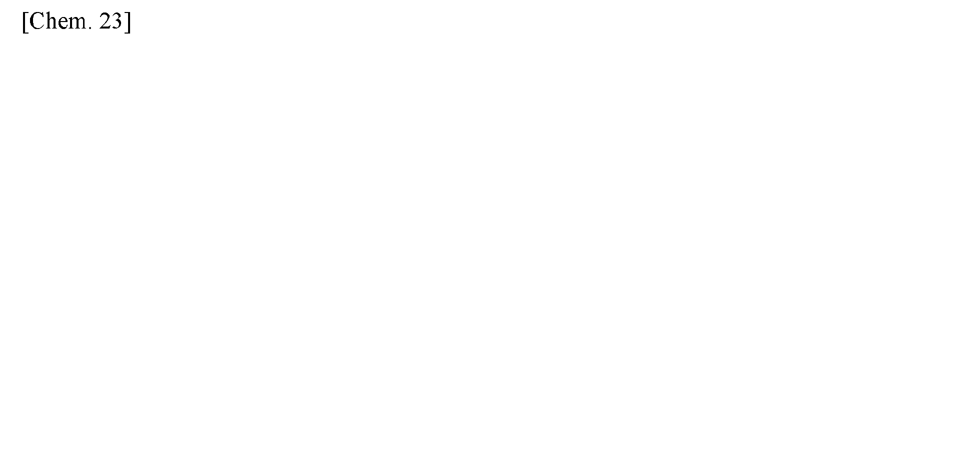 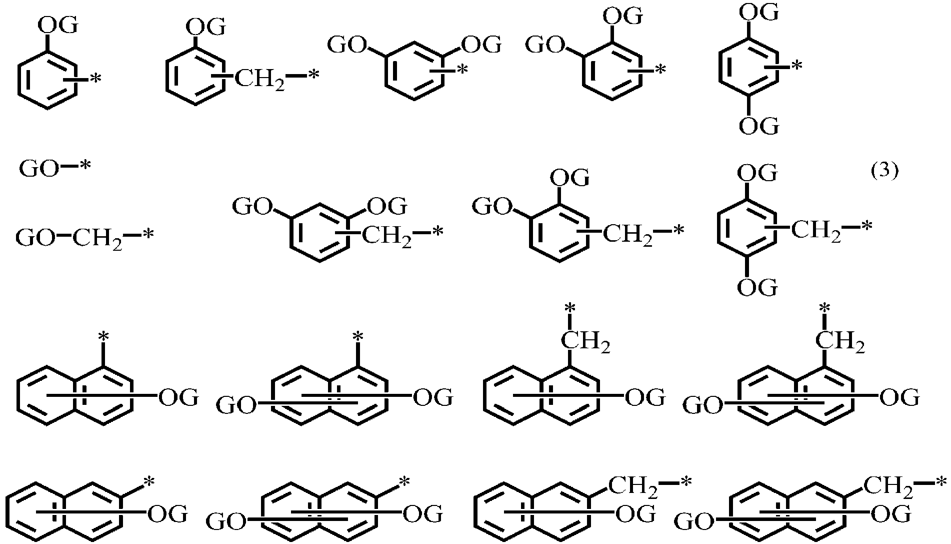
[the aromatic ring in the formula (3) may be substituted or unsubstituted, and * denotes a binding point,
G is the same as above, and
the -OG on the naphthalene ring in the formula indicates that the glycidyl ether group may be bonded at any point.] 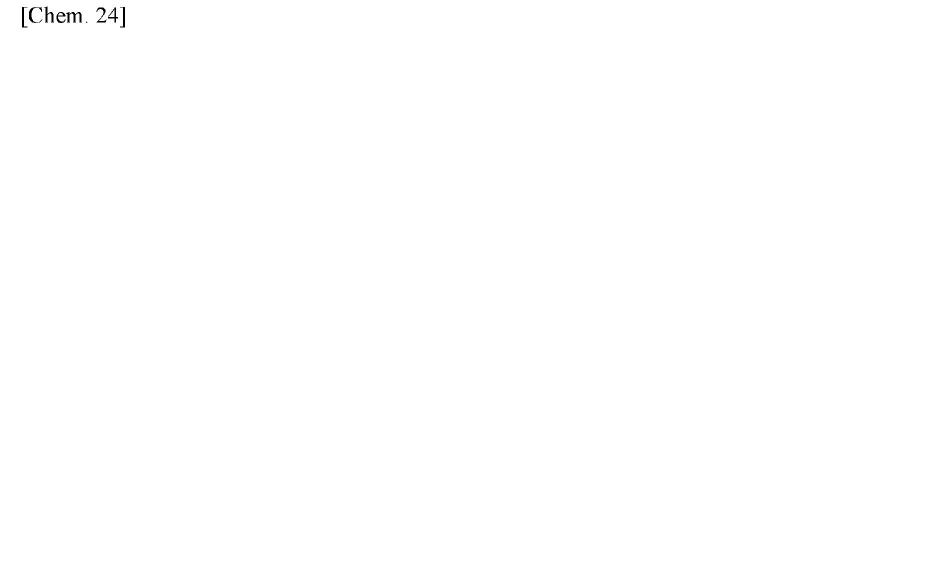 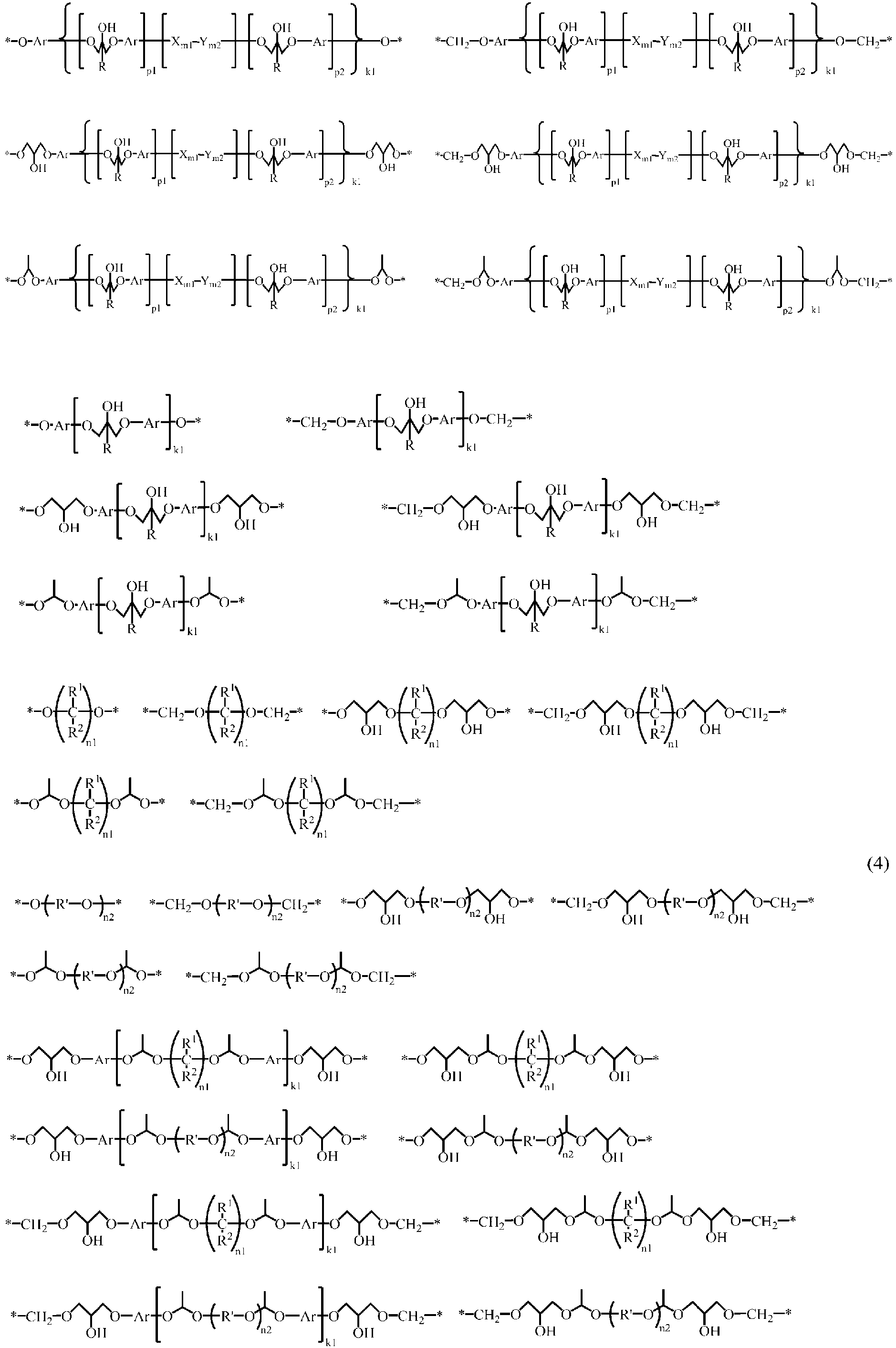
In the formula (4),
Ar's are each independently a structure containing an unsubstituted aromatic ring or an aromatic ring having a substituent,
R¹ and R² are each independently a hydrogen atom, a methyl group, or an ethyl group,
R is a hydrogen atom or a methyl group,
R' is a divalent hydrocarbon group having 2 to 12 carbon atoms,
n1 is an integer of 2 to 16, and n2 is an average number of repeating units of 2 to 30,
k1 is an average value of repetition number, from 0.5 to 10,
p1 and p2 are each independently 0 to 5,
X is a structural unit represented by the following formula (4-1) and Y is a structural unit represented by the following formula (4-2), 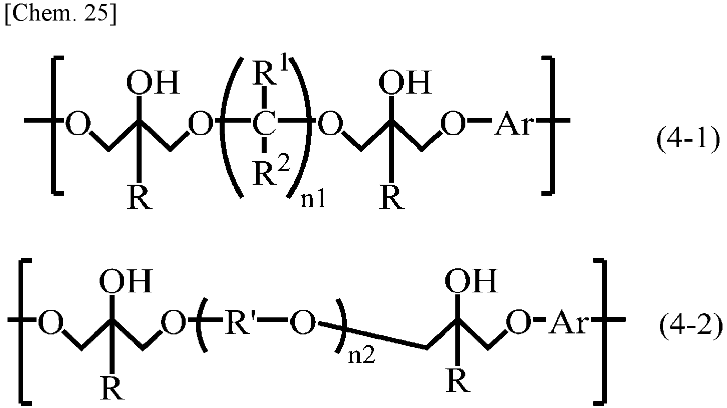
[in the formulas (4-1) and (4-2), Ar, R, R¹, R², R', n1, and n2 are the same as above.]
m1 and m2 are an average value of repetition number, each independently 0 to 25, and m1 + m2 ≥ 1.
provided that a bond between the structural unit X represented by the formula (4-1) and the structural unit Y represented by the formula (4-2) may be random or blocked, and the respective total numbers of structural units X and Y present in one molecule are m1 and m2, respectively.] 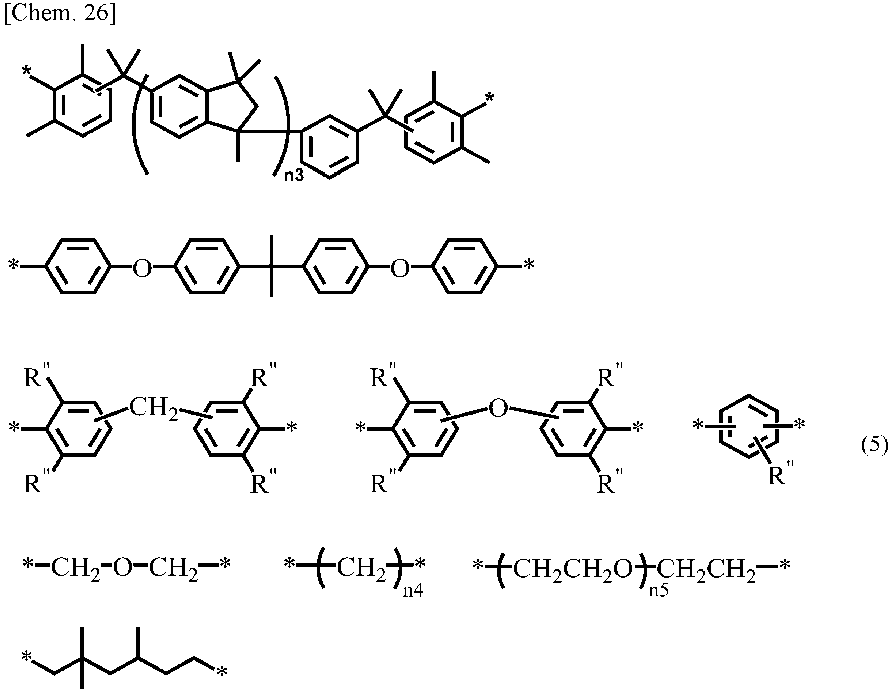
[in the formula (5), n3 and n5 are an average value of repetition number and are each 0.5 to 10, n4 is an integer of 1 to 16, and R"'s are each independently a hydrogen atom, a methyl group, or an ethyl group.] 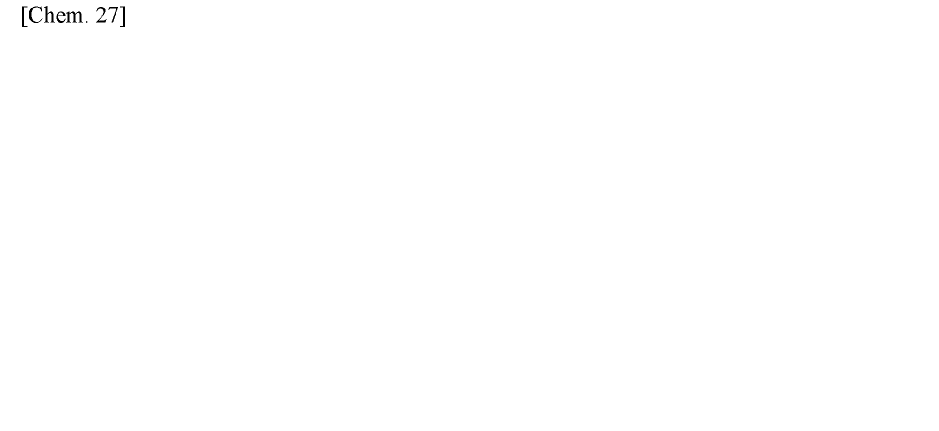 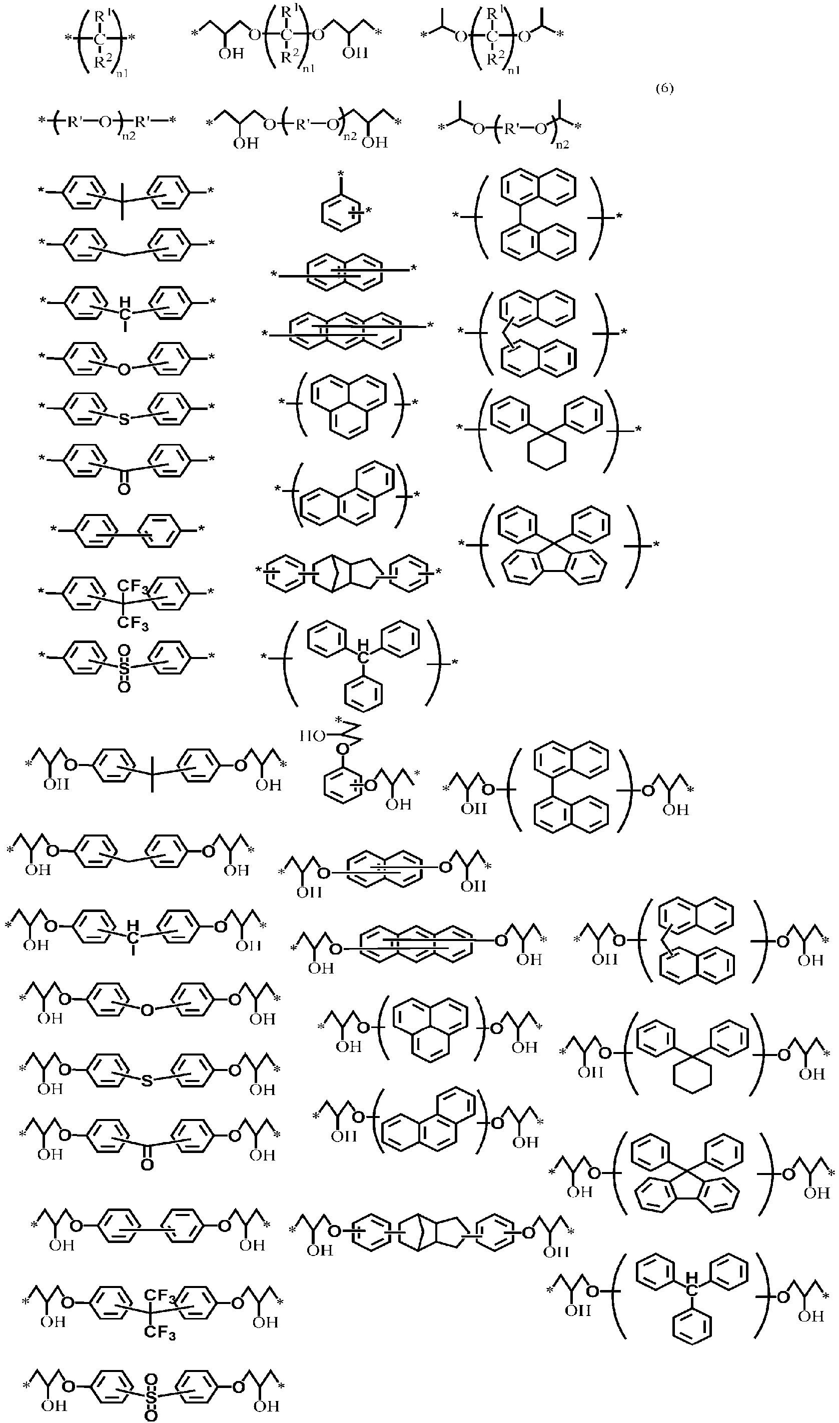 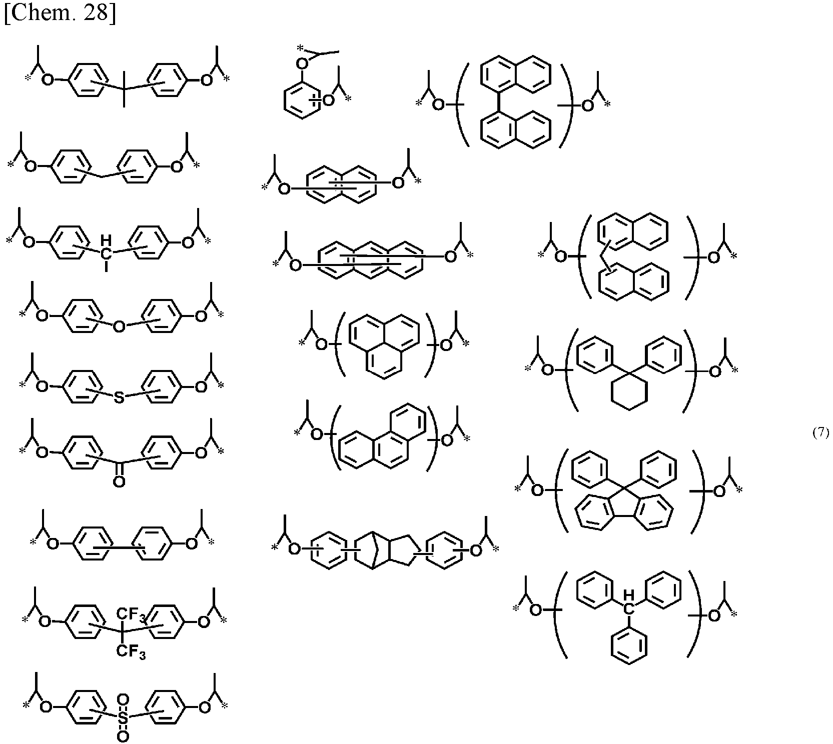
[in the formulas (6) and (7), R¹, R², R', n1, and n2 are the same as above].

The general formulas (1-1) and (1-2) have a reversible bond formed by anthracene and maleimide at the terminal within the molecule. The terminal maleimide structure in the general formula (1-1) and the terminal anthracene structure in the general formula (1-2) each have one or more Z1's which are any structure represented by the general formula (3), and this glycidyl ether or 2-methyl glycidyl ether group contributes to the curing reaction in the curable resin composition described later. m-a is the number of Z1's in the anthracene-derived structure and is an integer of 1 to 10, but is preferably in the range of 1 to 4, and more preferably 1 or 2 from the viewpoints of industrial availability of raw materials, ease of control of the curing reaction, and the like.

The general formula (2) has a disulfide bond within the molecule, and the molecular terminal has a structure containing an aromatic ring having one or more Z1's which are any structure represented by the general formula (3). The glycidyl ether or 2-methyl glycidyl ether group contributes to the curing reaction in the curable resin composition described later.

Z1 in the formula is a structural unit having a glycidyl ether group or a 2-methylglycidyl ether group represented by the general formula (3), but among these, those with the following structural formula are preferable from the viewpoint of raw material availability and reactivity. G is a glycidyl ether group or a 2-methyl glycidyl ether group.

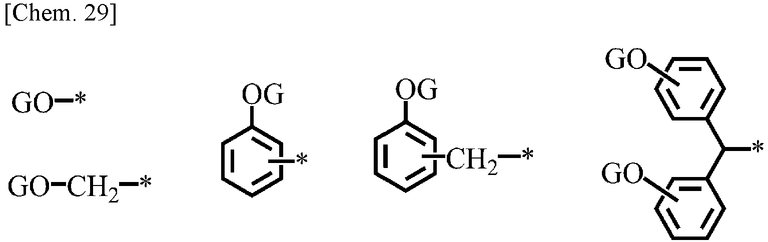

In the general formulas (1-1) and (1-2), the site linking the maleic acid-derived structures is Z3 and the site linking the anthracene-derived structures is Z2, and the site linking the oxygen atoms in the general formula (2) is Z4, and each of the structures is any structure represented by the general formulas (4), (5), (6) and (7).

n in the general formulas (1-1), (1-2) and (2) is an average value of repetition number and is 0 to 10, preferably in the range of 0 to 5.

Ar in these structural formulas is an aromatic ring that may have a substituent, and is not particularly limited. Examples of the aromatic ring include a benzene ring, a naphthalene ring, an anthracene ring, a phenanthrene ring, and a fluorene ring. Examples of the substituent include a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an arakyl group, and an aryl group. It is preferable that the substituent on Ar does not cause a curing reaction when used as the curable resin composition described later, since the effect of the present invention is more easily exhibited.

Among these, Ar is preferably any structure represented by the following structural formula. 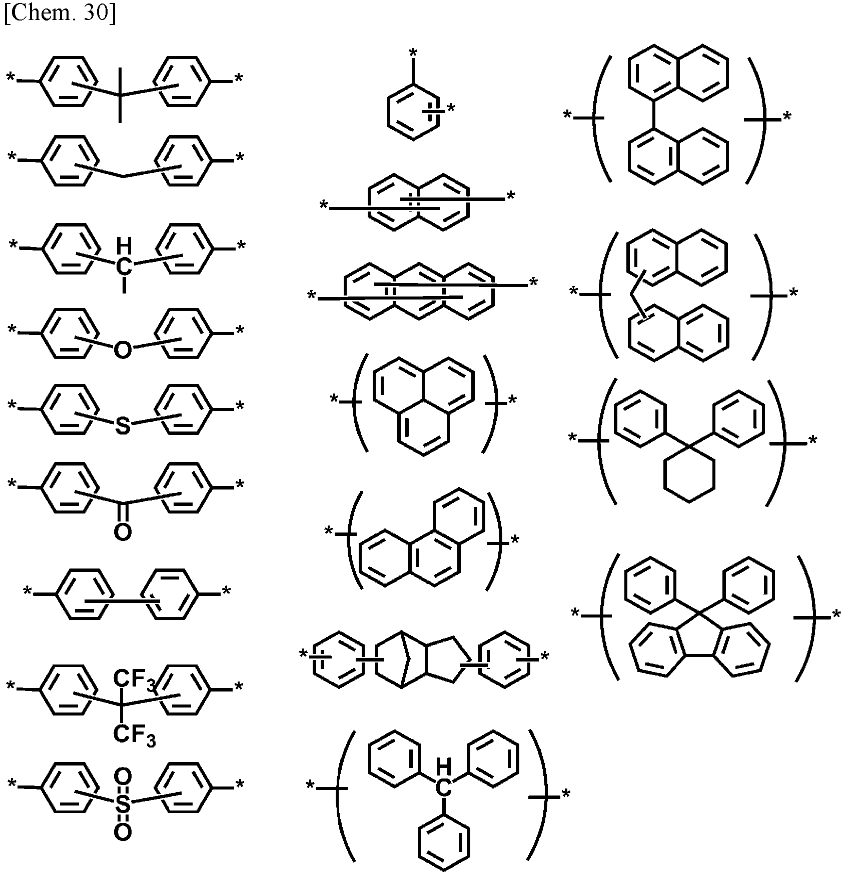 [The aromatic ring in the formula may be substituted or unsubstituted, and * denotes a binding point.]

Alternatively, the examples of Ar include structures represented by the following formulas. 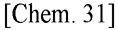 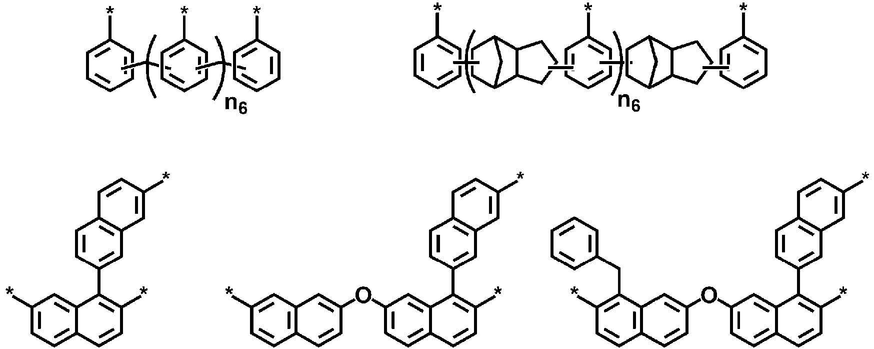 (In the formula, the aromatic ring may be substituted or unsubstituted, n₆ = 1 to 4, and * denotes a binding point.)

The following structures of Ar are particularly preferable. * denotes a binding point.

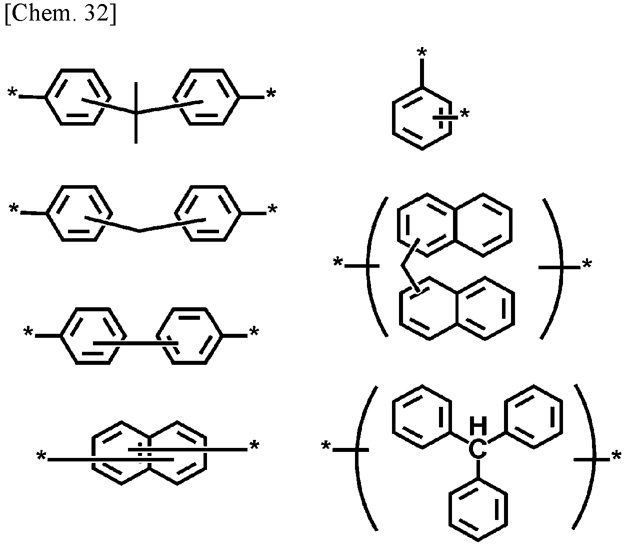

The repeating unit n₁ in the general formulas (4) and (4-1) is an integer of 2 to 16. When n₁ is 4 or more, the deformation mode when made into a cured product is likely to be elastically deformed. Alternatively, n₁ is less than or equal to 16, which prevents a decrease in crosslinking density. n₁ is preferably 4 to 15, more preferably 6 to 12.

In the general formulas (4) and (4-1), R¹ and R² are each independently a hydrogen atom, a methyl group, or an ethyl group, and R's are each independently a hydrogen atom or a methyl group. Among these, a hydrogen atom is preferable.

In the general formulas (4) and (4-2), n₂ is an average number of repeating units of 2 to 30. This range is preferable in that the balance between the viscosity of the glycidyl ether group-containing compound and the crosslinking density of the obtained cured product becomes favorable. The range is preferably 2 to 25, more preferably 4 to 20.

In the general formulas (4) and (4-2), R' is a divalent hydrocarbon group having 2 to 12 carbon atoms. In this range, adhesive strength is improved and the deformation mode of the cured product is more likely to be elastically deformed. Preferably, R' is a divalent hydrocarbon group having 2 to 6 carbon atoms.

Examples of the divalent hydrocarbon group are not particularly limited, but include a linear or branched-chain alkylene group, an alkenylene group, an alkynylene group, a cycloalkylene group, an arylene group, and an aralkylene group (a divalent group having an alkylene group and an arylene group).

Examples of the alkylene group include a methylene group, an ethylene group, a propylene group, a butylene group, a pentylene group, a hexylene group, a trimethylene group, a tetramethylene group, a pentamethylene group, and a hexamethylene group. Examples of the alkenylene group include a vinylene group, a 1-methylvinylene group, a propenylene group, a butenylene group, and a pentenylene group. Examples of the alkynylene group include an ethynylene group, a propynylene group, a butynylene group, a pentynylene group, and a hexynylene group. Examples of the cycloalkylene group include a cyclopropylene group, a cyclobutylene group, a cyclopentylene group, and a cyclohexylene group. Examples of the arylene group include a phenylene group, a tolylene group, a xylylene group, and a naphthylene group.

Among these, an ethylene group, a propylene group, and a tetramethylene group are preferable from the viewpoint of a balance between the availability of raw materials, the viscosity of the obtained glycidyl ether group-containing compound, and the flexibility of the cured product.

In the general formulas (4) and (4-2), R's are each independently a hydrogen atom or a methyl group. Among these, a hydrogen atom is preferable.

m1 and m2 in the general formula (4) are an average value of repetition number of the structural unit X and the structural unit Y described above, respectively, and are each independently 0 to 25, and m1 + m2 ≥ 1. Preferably, m1 and m2 are each in the range of 0.5 to 10.

Alternatively, k1 in the general formula (4) is an average value of repetition number and is in the range of 0.5 to 5, preferably in the range of 0.5 to 2.

In the general formula (5), n3 and n5 are an average value of repetition number and are each 0.5 to 10, n4 is an integer of 1 to 16, and R"'s are each independently a hydrogen atom, a methyl group, or an ethyl group. Among these, from the viewpoint of availability of raw materials and mechanical properties of the obtained cured product, n3 is preferably in the range of 0.5 to 10, n5 is preferably in the range of 2 to 3, n4 is preferably an integer of 1 to 8, and R" is preferably a hydrogen atom.

R¹, R², R', n1, and n2 in the general formulas (6) and (7) are the same as above, and the preferable ones are also the same as above.

Examples of the glycidyl ether group-containing compound of the present invention include, but are not limited to, those represented below.

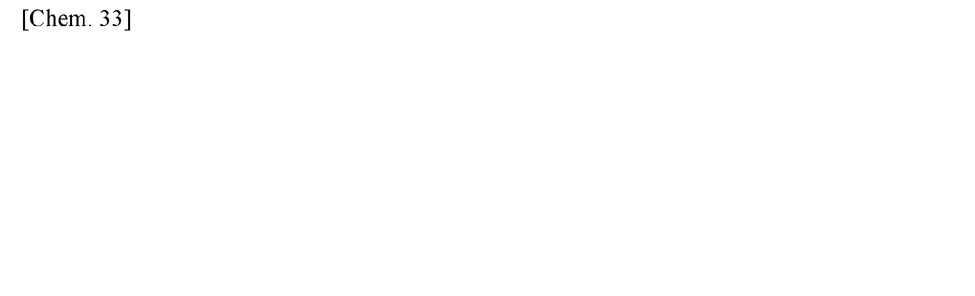

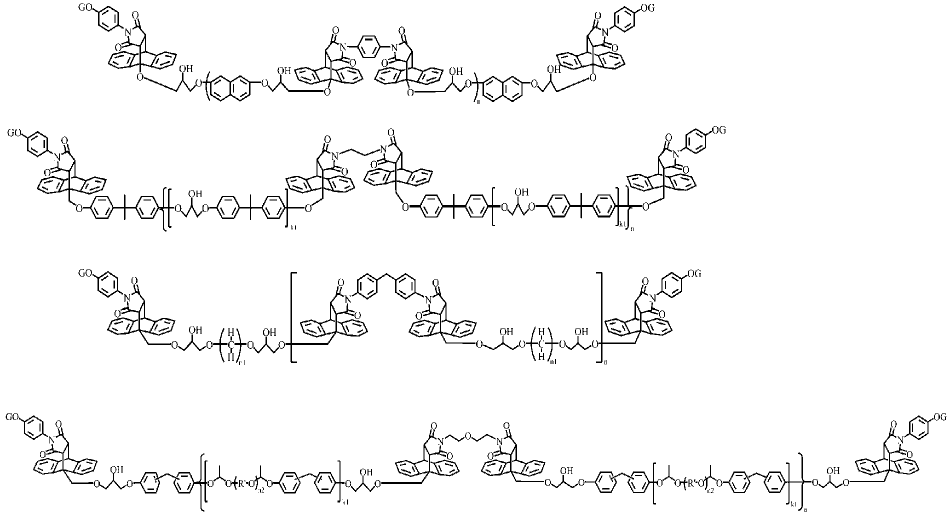

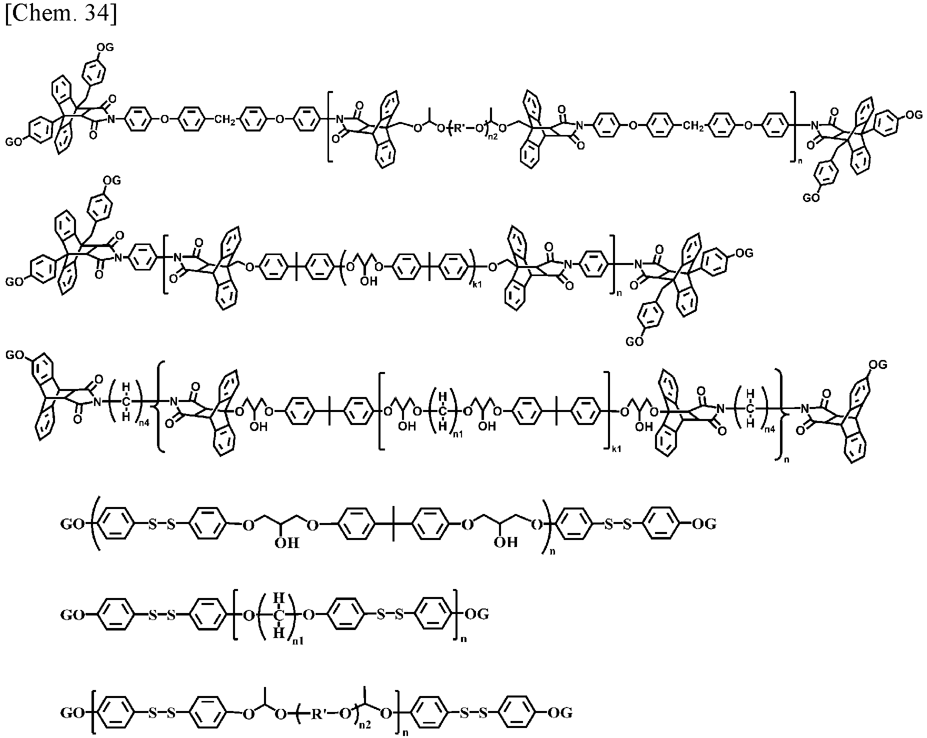

A method for producing the glycidyl ether group-containing compound according to one embodiment of the present invention is not particularly limited, and the compound may be produced stepwise by using known reactions depending on the target structure, and the compound may be obtained by appropriately combining commercially available raw materials. Hereinafter, a representative synthesis method will be described.

The general formulas (1-1) and (1-2) each have, as a reversible bond, two Diels-Alder reaction units, which are addition reaction units formed by a Diels-Alder reaction consisting of an anthracene structure and a maleimide structure, within the molecule, and can be obtained by using a maleimide compound having the structure Z1 in the general formula (1-1), and by using an anthracene compound having the structure Z1 in the general formula (1-2).

It is widely known that the so-called Diels-Alder reaction in which a conjugated diene such as an anthracene structure and a parent diene such as a maleimide structure undergo an addition reaction to form a 6-membered ring is an equilibrium reaction, and that at a temperature higher than the temperature at which the addition reaction proceeds, a retro-Diels-Alder reaction proceeds, that is a reverse reaction in which the addition reaction unit dissociates to return to the original conjugated diene and parent diene.

Examples of the maleimide compound having a hydroxyl group, which is a precursor of the maleimide compound having the structure of Z1 above, include any of the compounds listed in the following formulas. Among these, hydroxyphenylmaleimide is preferable from the viewpoint of curability, and monohydroxyphenylmaleimide is particularly preferable from the viewpoint of a balance between reactivity, physical properties of the cured product, and repairability and remoldability. Among monohydroxyphenylmaleimides, para-hydroxyphenylmaleimide is particularly preferable from the viewpoint of heat resistance. The hydroxyl group in the compound can be made into a glycidyl ether group by known methods, for example, as described in the examples.

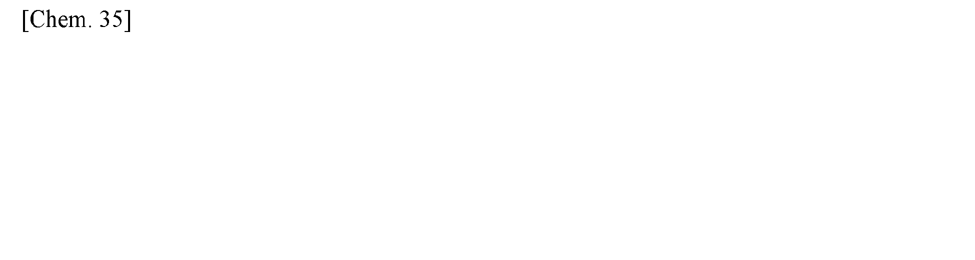

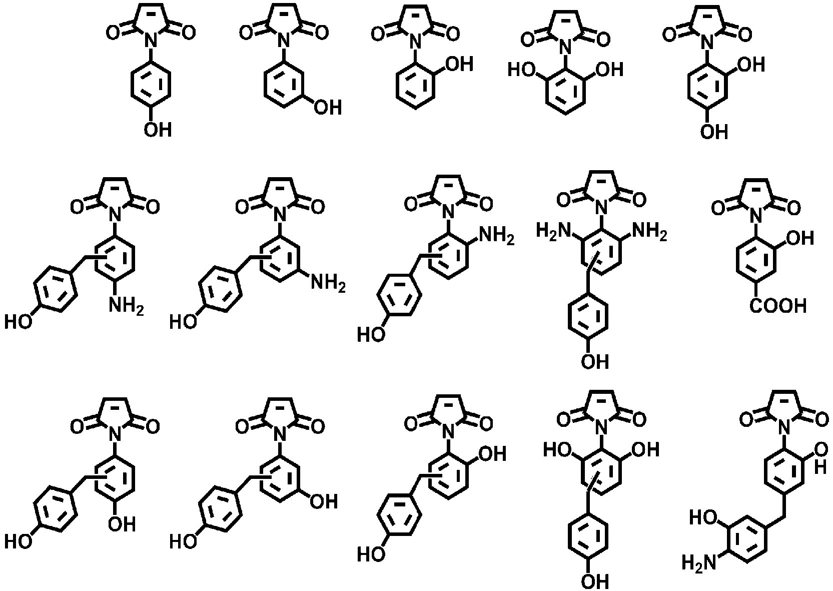

Examples of the anthracene compound having a hydroxyl group, which is a precursor of the anthracene compound having the structure of Z1 above, include any of the compounds listed in the following formulas. Among these, 9-(4-hydroxybenzyl)-10-(4-hydroxyphenyl)anthracene and hydroxyanthracene are preferable because of the good curability thereof, and 9-(4-hydroxybenzyl)-10-(4-hydroxyphenyl)anthracene and monohydroxyanthracene are particularly preferable from the viewpoint of a balance between reactivity, cured product properties, and repairability and remoldability. The hydroxyl group in the compound can be made into a glycidyl ether group by known methods, for example, as described in the examples.

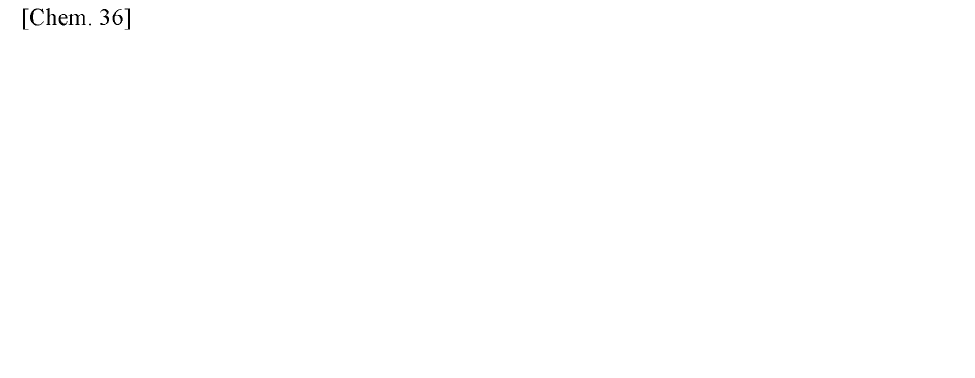

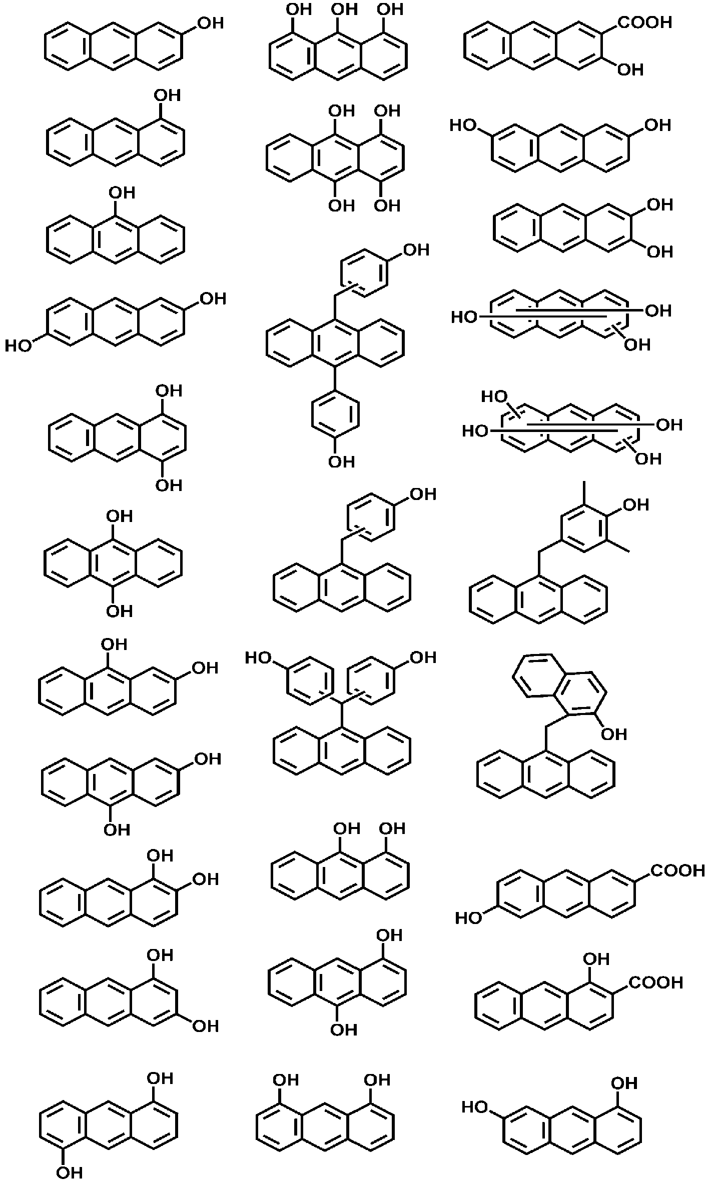

The structures of the maleimide compound and the anthracene compound each include those having, independently of each other, a hydrogen atom, a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group as a substituent. In addition, in the structures of the compounds listed in the above formula, the alkoxy group, the aralkyloxy group, the aryloxy group, the carboxy group, the alkyloxycarbonyl group, the aryloxycarbonyl group, the alkyl group, the cycloalkyl group, the aralkyl group, and the aryl group also include those having various substituents further bonded to the carbon atoms thereof.

The Diels-Alder reaction can be performed by any known method. For example, a conjugated diene compound and a parent diene compound are mixed in equimolar amounts, or in some cases, one of the components is used in excess, and the mixture is melted by heating or dissolved in a solvent and stirred at room temperature to 200°C for 1 to 24 hours. The resulting product can be obtained by filtration or solvent distillation without purification, or by a commonly used isolation and purification method such as recrystallization, reprecipitation, or chromatography.

The sites other than the reversible bond can be synthesized by known methods. For example, a diglycidyl ether of an aliphatic dihydroxy compound or an aliphatic divinyl ether is reacted with an aromatic hydroxy compound to obtain a compound having a hydroxy group at the terminal, which is then reacted with chloromethylanthracene, glycidyloxyanthracene, or the like to introduce an anthracene structure at the terminal, and further, a Diels-Alder reaction is performed with a maleimide compound having a glycidyl ether group as described above to obtain the compound represented by the general formula (1-1).

Alternatively, a compound having a hydroxy group at the terminal is obtained, and then epoxidized to convert the terminal into a glycidyl ether group. Thereafter, the compound is reacted with hydroxyanthracene or the like to introduce an anthracene structure at the terminal. Furthermore, the compound represented by the general formula (1-1) can be obtained by performing a Diels-Alder reaction with a maleimide compound having a glycidyl ether group as described above.

Alternatively, an aromatic dihydroxy compound is reacted with a dihalogenated alkyl compound or a dihalogenated aralkyl compound to obtain a compound having a halogenated alkyl group at the terminal, and then the compound is reacted with hydroxymethylanthracene or the like to introduce an anthracene structure at the terminal. Furthermore, the compound is subjected to a Diels-Alder reaction with a maleimide compound having a glycidyl ether group as described above to obtain the compound represented by the general formula (1-1).

Examples of the diglycidyl ether of the aliphatic dihydroxy compound are not particularly limited, but include 1,11-undecanediol diglycidyl ether, 1,12-dodecanediol diglycidyl ether, 1,13-tridecanediol, 1,14-tetradecanediol diglycidyl ether, 1,15-pentadecanediol diglycidyl ether, 1,16-hexadecanediol diglycidyl ether, 2-methyl-1,11-undecanediol diglycidyl ether, 3-methyl-1,11-undecanediol diglycidyl ether, and 2,6,10-trimethyl-1,11-undecanediol diglycidyl ether, which may be used alone or in combination of two or more kinds thereof.

Among these, a compound having a structure in which glycidyl groups are linked via an ether group to both terminals of an alkylene chain having 12 to 14 carbon atoms are preferable from the viewpoint of an excellent balance between flexibility and heat resistance of the obtained cured product, and it is most preferable to use 1,12-dodecanediol diglycidyl ether, 1,13-tridecanediol, or 1,14-tetradecanediol diglycidyl ether.

Examples of the aliphatic divinyl ether is not particularly limited, but include divinyl ethers of linear alkylene groups, such as polyethylene glycol divinyl ether, polypropylene glycol divinyl ether, polytetramethylene glycol divinyl ether, 1,3-butylene glycol divinyl ether, 1,4-butanediol divinyl ether, 1,6-hexanediol divinyl ether, 1,9-nonanediol divinyl ether, and 1,10-decanediol divinyl ether, divinyl ethers of branched alkylene groups, such as neopentyl glycol divinyl ether, divinyl ethers containing a cycloalkane structure, such as 1,4-cyclohexanediol divinyl ether, 1,4-cyclohexanedimethanol divinyl ether, tricyclodecanediol divinyl ether, tricyclodecane dimethanol divinyl ether, pentacyclopentadecanedimethanol divinyl ether, and pentacyclopentadecanediol divinyl ether, bisphenol A divinyl ether, bisphenol F divinyl ether, and hydroquinone divinyl ether, which may be used alone or in combination of two or more kinds thereof.

Among these, from the viewpoint of an excellent balance between flexibility and toughness of the obtained cured product, a divinyl ether having a polyether structure or a linear alkylene chain having 9 to 10 carbon atoms is preferable, and it is most preferable to use polyethylene glycol divinyl ether, polypropylene glycol divinyl ether, polytetramethylene glycol divinyl ether, 1,12-dodecanediol diglycidyl ether, 1,13-tridecanediol, and 1,14-tetradecanediol diglycidyl ether.

Examples of the aromatic hydroxy compound are not particularly limited, but include dihydroxybenzenes such as hydroquinone, resorcin, and catechol; trihydroxybenzenes such as pyrogallol, 1,2,4-trihydroxybenzene, and 1,3,5-trihydroxybenzene; triphenylmethane type phenols such as 4,4',4"-trihydroxytriphenylmethane; dihydroxynaphthalenes such as 1,6-dihydroxynaphthalene, 2,7-dihydroxynaphthalene, 1,4-dihydroxynaphthalene, 1,5-dihydroxynaphthalene, 2,3-dihydroxynaphthalene, and 2,6-dihydroxynaphthalene; tetrafunctional phenols such as 1,1'-methylenebis-(2,7-naphthalenediol), 1,1'-binaphthalene-2,2',7,7'-tetraol, and 1,1'-oxybis-(2,7-naphthalenediol) obtained by coupling reaction of dihydroxynaphthalenes; bisphenols such as bis(4-hydroxyphenyl)methane, 2,2-bis(4-hydroxyphenyl)propane, 2,2-bis(3-methyl-4-hydroxyphenyl)propane, 1,1-bis(4-hydroxyphenyl)cyclohexane, and 1,1-bis(4-hydroxyphenyl)-1-phenylethane, and bis(4-hydroxyphenyl)sulfone; biphenols such as 2,2'-biphenol, 4,4'-biphenol, (1,1'-biphenyl)-3,4-diol, 3,3'-dimethyl-(1,1'-biphenyl)-4,4'-diol, 3-methyl-(1,1'-biphenyl)-4,4'-diol, 3,3',5,5'-tetramethylbiphenyl-2,2'-diol, 3,3',5,5'-tetramethylbiphenyl-4,4'-diol, 5-methyl-(1,1'-biphenyl)-3,4'-diol, 3'-methyl-(1,1'-biphenyl)-3,4'-diol, and 4'-methyl-(1,1'-biphenyl)-3,4'-diol; alicyclic structure-containing phenols such as polyaddition products of phenol and dicyclopentadiene, and polyaddition products of phenol and terpene compounds; naphthols such as bis(2-hydroxy-1-naphthyl)methane and bis(2-hydroxy-1-naphthyl)propane; and the so-called xylox phenolic resin which is a condensation reaction product of phenol and phenylene dimethyl chloride or biphenylene dimethyl chloride, which may be used alone or in combination of two or more kinds thereof. Further, examples of the substituent in the aromatic nucleus of each of the above compounds include a bifunctional phenol compound having a structure in which a methyl group, a t-butyl group, or a halogen atom is substituted for the aromatic nucleus of each compound. The alicyclic structure-containing phenols and the xylox phenolic resin may contain not only bifunctional components but also trifunctional or higher functional components at the same time. The components may be used as they are, or may be subjected to a purification process such as a column purification process to extract only the bifunctional components.

Among these, in terms of excellent balance between flexibility and toughness when made into a cured product, bisphenols are preferable, and especially from the point of view of the remarkable performance in imparting toughness, bis(4-hydroxyphenyl)methane and 2,2-bis(4-hydroxyphenyl)propane are preferable. Alternatively, when moisture resistance of the cured product is important, phenols containing an alicyclic structure are preferable.

The reaction ratio of the diglycidyl ether of the aliphatic dihydroxy compound to the aromatic hydroxy compound is preferably reacted in the range of 1/1.01 to 1/5.0 (molar ratio) of the former/the latter, and in terms of providing a well-balanced combination of flexibility and heat resistance of the obtained cured product, it is preferable that (a1)/(a2) is 1/1.02 to 1/3.0 (molar ratio).

The reaction between the diglycidyl ether of the aliphatic dihydroxy compound and the aromatic hydroxy compound is preferably performed in the presence of a catalyst. As the catalyst, various catalysts can be used, and examples thereof include alkaline (earth) metal hydroxides such as sodium hydroxide, potassium hydroxide, lithium hydroxide, and calcium hydroxide; alkali metal carbonates such as sodium carbonate and potassium carbonate, phosphorus compounds such as triphenylphosphine, DMP-30, and DMAP, quaternary ammonium salts such as chlorides, bromides, and iodides of tetramethylammonium, tetraethylammonium, tetrabutylammonium, and benzyltributylammonium, and chlorides, bromides, and iodides of tetramethylphosphonium, tetraethylphosphonium, tetrabutylphosphonium, and benzyltributylphosphonium; tertiary amines such as triethylamine, N,N-dimethylbenzylamine, 1,8-diazabicyclo[5.4.0]undecene, and 1,4-diazabicyclo[2.2.2]octane; and imidazoles such as 2-ethyl-4-methylimidazole and 2-phenylimidazole. Two or more kinds of catalysts may be used in combination. Among these, sodium hydroxide, potassium hydroxide, triphenylphosphine, and DMP-30 are preferable because the reaction proceeds quickly and the amount of impurities is highly reduced. The amount of these catalysts used is not particularly limited, but it is preferable to use 0.0001 to 0.01 mole per mole of the phenolic hydroxyl group of the aromatic hydroxy compound. The form of these catalysts is not particularly limited, and the catalysts may be used in the form of an aqueous solution or in the form of a solid.

The reaction between the diglycidyl ether of the aliphatic dihydroxy compound and the aromatic hydroxy compound can be performed in the absence of a solvent or in the presence of an organic solvent. Examples of the organic solvent that can be used include methyl cellosolve, ethyl cellosolve, toluene, xylene, methyl isobutyl ketone, dimethyl sulfoxide, propyl alcohol, and butyl alcohol. The amount of the organic solvent used is usually 50 to 300% by mass, preferably 100 to 250% by mass, based on the total mass of the raw materials charged. These organic solvents can be used alone or in combination. In order to perform the reaction quickly, it is preferable to use no solvent, while the use of dimethyl sulfoxide is preferable in terms of reducing impurities in the final product.

The reaction temperature when performing the above reaction is usually 50 to 180°C, and the reaction time is usually 1 to 10 hours. The reaction temperature is preferably 100 to 160°C in order to reduce impurities in the final product. In addition, when the obtained compound is significantly colored, an antioxidant or a reducing agent may be added to suppress the coloring. Examples of the antioxidant are not particularly limited, but include hindered phenol compounds such as 2,6-dialkylphenol derivatives, divalent sulfur compounds, and phosphite ester compounds containing a trivalent phosphorus atom. Examples of the reducing agent are not particularly limited, but include hypophosphorous acid, phosphorous acid, thiosulfuric acid, sulfurous acid, hydro sulfite, and salts thereof.

After the reaction is completed, the reaction mixture may be neutralized or washed with water until the pH value of the reaction mixture reaches 3 to 7, preferably 5 to 7. The neutralization and aqueous washing treatments may be performed in the usual manner. For example, when a basic catalyst is used, an acidic substance such as hydrochloric acid, sodium dihydrogen phosphate monobasic, p-toluenesulfonic acid, or oxalic acid can be used as a neutralizing agent. After neutralization or washing with water, the compound can be obtained by distilling off the solvent under reduced pressure and heating, if necessary, and concentrating the product.

The reaction ratio of the aliphatic divinyl ether and the aromatic hydroxy compound is preferably reacted in the range of 1/1.01 to 1/5.0 (molar ratio) of the former/the latter, and in terms of providing a well-balanced combination of flexibility and heat resistance of the obtained cured product, it is preferable that (a1)/(a2) is in the range of 1/1.02 to 1/3.0 (molar ratio).

The reaction between the diglycidyl ether of the aliphatic dihydroxy compound and the aromatic hydroxy compound proceeds sufficiently without the use of a catalyst, but a catalyst can be used as appropriate from the viewpoint of the selection of raw materials and increasing the reaction rate. Examples of the catalyst that can be used herein include inorganic acids such as sulfuric acid, hydrochloric acid, nitric acid, and phosphoric acid; organic acids such as toluenesulfonic acid, methanesulfonic acid, xylenesulfonic acid, trifluoromethanesulfonic acid, oxalic acid, formic acid, trichloroacetic acid, and trifluoroacetic acid; and Lewis acids such as aluminum chloride, iron chloride, tin chloride, gallium chloride, titanium chloride, aluminum bromide, gallium bromide, boron trifluoride ether complex, and boron trifluoride phenol complex. The amount of the catalyst used is usually in the range of 10 ppm to 1% by weight based on the mass of the divinyl ether compound. In this case, it is preferable to select the type and amount used so as not to cause a nuclear addition reaction of the vinyl group to the aromatic ring.

In addition, the reaction between the aliphatic divinyl ether and the aromatic hydroxy compound can be performed in the absence of a solvent or in the presence of an organic solvent. Examples of the organic solvent include aromatic organic solvents such as benzene, toluene, and xylene; ketone-based organic solvents such as acetone, methyl ethyl ketone, methyl isobutyl ketone, and cyclohexanone; and alcohol-based organic solvents such as methanol, ethanol, isopropyl alcohol, and normal butanol. The amount of the organic solvent used is usually 50 to 300% by mass, preferably 100 to 250% by mass, based on the total mass of the raw materials charged. These organic solvents can be used alone or in combination.

The reaction temperature when performing the above reaction is usually 50 to 150°C, and the reaction time is usually 0.5 to 10 hours. In this case, in order to prevent self-polymerization of the vinyl ether group, the reaction is preferably performed in an oxygen atmosphere.

After completion of the reaction, when an organic solvent was used, the organic solvent is removed under reduced pressure and heating, and when a catalyst was used, the catalyst is deactivated with a deactivator or the like as necessary, and then removed by washing with water or filtration, whereby the compound can be obtained.

The compound having a hydroxyl group at the terminal thus obtained is reacted with chloromethylanthracene or the like. In this case, sodium hydroxide, potassium hydroxide, potassium carbonate, or the like can be used as a catalyst, and toluene, acetone, methyl ethyl ketone (MEK), methyl isobutyl ketone, acetonitrile, dimethylformamide, or the like can be used as a solvent. The reaction temperature is from room temperature to 200°C, and the reaction time is 1 to 24 hours. Thereafter, the catalyst is then removed by filtration or the like, and the target compound can be obtained by extraction, solvent removal, or the like. The Diels-Alder reaction for this compound is as described above.

Examples of the aliphatic hydroxy compound are not particularly limited, but include 1,4-butanediol, 1,5-pentanediol, 1,6-hexanediol, 1,7-heptanediol, 1,8-octanediol, 1,9-nonanediol, 1,10-decanediol, 1,11-undecanediol, 1,12-dodecanediol, 1,13-tridecanediol, 1,14-tetradecanediol, 1,15-pentadecanediol, 1,16-hexadecanediol, 2-methyl-1,11-undecanediol, 3-methyl-1,11-undecanediol, 2,6,10-trimethyl-1,11-undecanediol, polyethylene glycol, polypropylene glycol, polytetramethylene glycol, polypentamethylene glycol diglycidyl ether, polyhexamethylene glycol diglycidyl ether, polyheptamethylene glycol diglycidyl ether, which may be used alone or in combination of two or more kinds thereof.

Among these, from the viewpoint of an excellent balance between flexibility and heat resistance of the obtained cured product, it is preferable to use a dihydroxy compound having a polyether structure or a linear alkylene chain having 12 to 14 carbon atoms, and it is most preferable to use polyethylene glycol, polypropylene glycol, polytetramethylene glycol, 1,12-dodecanediol, 1,13-tridecanediol, or 1,14-tetradecanediol.

Examples of the dihalogenated alkyl compound are not particularly limited, but include 1,4-dichlorobutane, 1,5-dichloropentane, 1,6-dichlorohexane, 1,7-dichloroheptane, 1,8-dichlorooctane, 1,9-dichlorononane, 1,10-dichlorodecane, 1,11-dichloroundecane, 1,12-dichlorododecane, 1,4-dibromobutane, 1,5-dibromopentane, 1,6-dibromohexane, 1,7-dibromoheptane, 1,8-dibromooctane, 1,9-dibromononane, 1,10-dibromodecane, 1,11-dibromoundecane, and 1,12-dibromododecane, which may be used alone or in combination of two or more kinds thereof.

Examples of the dihalogenated aralkyl compound are not particularly limited, but include dichloroxylene, dichloromethylbiphenyl, dibromoxylene, and dibromomethylbiphenyl, which may be used alone or in combination of two or more kinds thereof.

The reaction ratio of the aromatic dihydroxy compound to the dihalogenated alkyl compound or dihalogenated aralkyl compound is preferably reacted in the range of 1/1.01 to 1/5.0 (molar ratio) of the former/the latter, and from the viewpoint of providing a well-balanced combination of flexibility and heat resistance of the obtained cured product, it is preferable that (a1)/(a2) is in the range of 1/1.02 to 1/3.0 (molar ratio).

The reaction between the aromatic dihydroxy compound and the dihalogenated alkyl compound or dihalogenated aralkyl compound is preferably performed in the presence of a catalyst. As the catalyst, various catalysts can be used, such as alkali (earth) metal hydroxides sodium hydroxide, potassium hydroxide, lithium hydroxide, and calcium hydroxide, and alkali metal carbonates such as sodium carbonate and potassium carbonate. Two or more kinds of catalysts may be used in combination. Among these, sodium hydroxide, potassium hydroxide and potassium carbonate are preferable because the reaction proceeds quickly and the amount of impurities is highly reduced. The amount of these catalysts used is not particularly limited, but it is preferable to use 0.0001 to 10 moles per mole of the phenolic hydroxyl group of the aromatic hydroxy compound. The form of these catalysts is not particularly limited, and the catalysts may be used in the form of an aqueous solution or in the form of a solid.

Further, the reaction between the aromatic dihydroxy compound and the dihalogenated alkyl compound or the dihalogenated aralkyl compound can be performed in the presence of an organic solvent or in the presence of an organic solvent. Examples of the organic solvent that can be used include toluene, acetone, methyl ethyl ketone (MEK), methyl isobutyl ketone, acetonitrile, and dimethylformamide. The amount of organic solvents used is usually 50 to 300% by mass compared to the total mass of the charged raw material, and preferably 100 to 1000 % by mass. These organic solvents can be used alone or in combination.

The reaction temperature when performing the reaction is usually from the normal room temperature to 150°C, and the reaction time is usually 1 to 24 hours. The reaction temperature is preferably at room temperature to 100°C because the impurities of the final product can be reduced.

Hydroxymethylanthracene or the like is reacted to a compound having a halogenated alkyl group at the terminal obtained in this way. In this case, sodium hydroxide, potassium hydroxide, potassium carbonate, or the like can be used as a catalyst, and toluene, acetone, methyl ethyl ketone (MEK), methyl isobutyl ketone, acetonitrile, dimethylformamide, or the like can be used as a solvent. The reaction temperature is from room temperature to 200°C, and the reaction time is 1 to 24 hours. Thereafter, the catalyst is then removed by filtration or the like, and the target compound can be obtained by extraction, solvent removal, or the like. The Diels-Alder reaction for this compound is as described above.

The intermediate of the conjugated diene or parent diene intermediate before the Diels-Alder reaction can be represented by the following general formula (1-1)', (1-2)'. 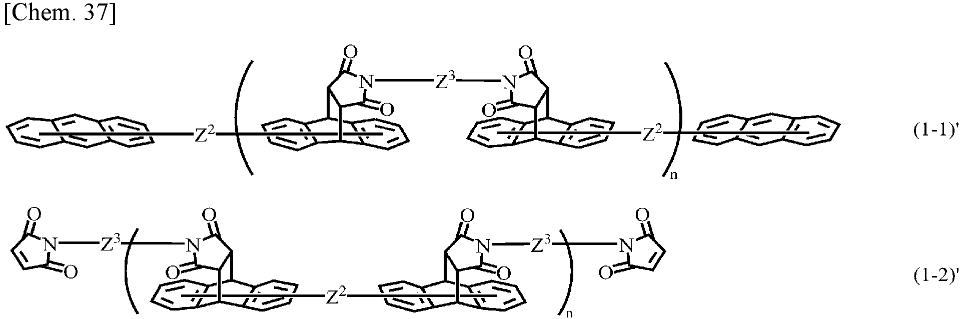 [in the formula, n, Z², and Z³ are the same as above].

The method for producing a compound having a disulfide bond as the reversible bond represented by the general formula (3) is not particularly limited.

The following compounds can be listed as compounds that can be used as a raw material of the compound having a disulfide bond.

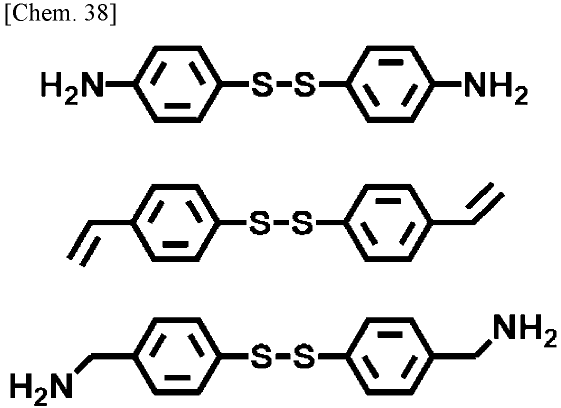

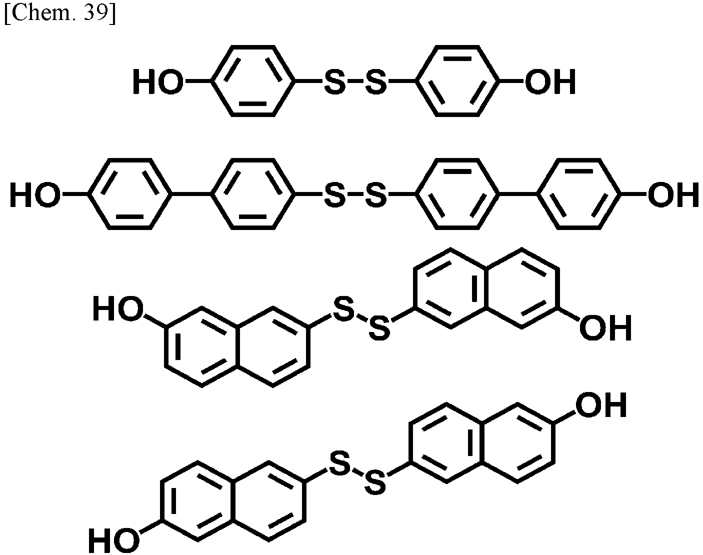

A known method may be used for the compound having a disulfide bond. For example, a compound having a thiol group is oxidatively bonded. As an oxidizing agent, iodine or hydrogen peroxide is generally used. A compound having a thiol group is melted by heating or dissolved in a solvent and stirred at room temperature to 200°C for 1 to 24 hours. The resulting product can be obtained by filtration or solvent distillation without purification, or by a commonly used isolation and purification method such as recrystallization, reprecipitation, or chromatography. After obtaining the precursor hydroxyl group-containing compound, known methods can be used to convert the hydroxy group to a glycidyl ether group.

The glycidyl ether group-containing compound of the present invention can be made into a curable resin composition by combining the glycidyl ether group-containing compound with the reactive compound (I). The curable resin composition can be suitably used for various electrical and electronic components such as an adhesive, a coating material, a photo resist, a printed wiring board, a semiconductor encapsulation material, and the like.

Examples of the compound (I) having reactivity with the glycidyl ether group-containing compound include various known curing agents for epoxy resins, such as an amine compound, an acid anhydride, an amide compound, a phenolic hydroxyl group-containing compound, a carboxylic acid compound, and a thiol compound. The curing agent can be appropriately selected depending on the physical properties of the desired cured product, but in particular, a hydroxyl group-containing compound is preferable from the viewpoint of mechanical strength and adhesion to the base material.

Examples of the amine compound include aliphatic amine compounds such as trimethylenediamine, ethylenediamine, N,N,N',N'-tetramethylethylenediamine, pentamethyldiethylenediamine, triethylenediamine, dipropylenediamine, N,N,N',N'-tetramethylpropylenediamine, tetramethylenediamine, pentanediamine, hexamethylenediamine, trimethylhexamethylenediamine, N,N,N',N'-tetramethylhexamethylenediamine, N,N-dimethylcyclohexylamine, diethylenetriamine, triethylenetetetramine, tetraethylenepentamine, dimethylaminopropylamine, diethylaminopropylamine, dibutylaminopropylamine, 1,4-diazabicyclo(2,2,2)octane (triethylenediamine), polyoxyethylenediamine, polyoxypropylenediamine, bis(2-dimethylaminoethyl) ether, dimethylaminoethoxyethanol, triethanolamine, dimethylaminohexanol, benzylmethylamine, dimethylbenzylamine, m-xylenediamine, and alpha-methylbenzylmethylamine;
alicyclic and heterocyclic amine compounds such as piperidine, piperazine, mentandiamine, isophoronediamine, methylmorpholine, ethylmorpholine, N,N',N"-tris(dimethylaminopropyl)hexahydro-s-triazine, 3,9-bis(3-aminopropyl)-2,4,8,10-tetraoxyspiro(5,5)undecane adduct, N-aminoethylpiperazine, trimethylaminoethylpiperazine, bis(4-aminocyclohexyl)methane, N,N'-dimethylpiperazine, and 1,8-diazabicyclo-[5.4.0]-undecene (DBU);
aromatic amine compounds such as o-phenylenediamine, m-phenylenediamine, p-phenylenediamine, diaminodiphenylmethane, diaminodiphenylsulfone, pyridine, and picoline; and
modified amine compounds such as an epoxy compound addition polyol, a Michael addition polyol, a Mannich addition polyamine, a thiourea addition polyamine, a ketone blocked polyamine, dicyandiamide, guanidine, an organic acid hydrazide, diaminomaleonitrile, an amine imide, a boron trifluoride/piperidine complex, and a boron trifluoride-monoethylamine complex.

Specific examples of the acid anhydride include phthalic anhydride, trimellitic anhydride, pyromellitic anhydride, maleic anhydride, maleic anhydride polypropylene glycol, tetrahydrophthalic anhydride, methyltetrahydrophthalic anhydride, methylnadic anhydride, hexahydrophthalic anhydride, and methylhexahydrophthalic anhydride.

Examples of the phenolic hydroxyl group-containing compound include bisphenols such as bis(4-hydroxyphenyl)methane, 2,2-bis(4-hydroxyphenyl)propane, 2,2-bis(3-methyl-4-hydroxyphenyl)propane, 1,1-bis(4-hydroxyphenyl)cyclohexane, and 1,1-bis(4-hydroxyphenyl)-1-phenylethane, and bis(4-hydroxyphenyl) sulfone, a phenolic novolac resin, a cresol novolac resin, an aromatic hydrocarbon formaldehyde resin-modified phenolic resin, a dicyclopentadiene phenol addition resin, a phenol aralkyl resin (xylox resin), a naphthol aralkyl resin, a trimethylol methane resin, a tetraphenylol ethane resin, a naphthol novolac resin, a naphthol-phenol co-fused novolac resin, a naphthol-cresol co-fused novolac resin, a biphenyl-modified phenol resin (polyvalent phenol compound with phenol nuclei linked by bis-methylene groups), a biphenyl-modified phenol resin (polyvalent phenol compound with phenol nuclei linked by bismethylene groups), a biphenyl-modified naphthol resin (polyvalent naphthol compound with phenol nuclei linked by bismethylene groups), an aminotriazine-modified phenolic resin (polyvalent phenol compound with phenol nuclei linked by melamine, benzoguanamine, and the like), and an alkoxy group-containing aromatic ring-modified novolac resin (polyvalent phenolic compound in which the phenol nuclei and alkoxy group-containing aromatic rings are linked by formaldehyde).

Examples of the amide compound include dicyandiamide and polyamide amine. Examples of the polyamide amine include the product obtained by reacting an aliphatic dicarboxylic acid such as succinic acid, glutaric acid, adipic acid, pimelic acid, suberic acid, or azelaic acid, or a carboxylic acid compound such as a fatty acid or a dimer acid with an aliphatic polyamine or a polyamine with polyoxyalkylene chains.

Examples of the carboxylic acid compound include a carboxylic acid polymer such as a carboxylic acid terminal polyester, a polyacrylic acid, and a maleic acid-modified polypropylene glycol.

The thiol compound is preferably one that contains two or more thiol groups in two molecules. Examples of the thiol compound include 3,3'-dithiodipropionic acid, trimethylolpropane tris(thioglycolate), pentaerythritol tetrakis(thioglycolate), ethylene glycol dithioglycolate, 1,4-bis(3-mercaptobutyryloxy)butane, tris[(3-mercaptopropionyloxy)-ethyl]-isocyanurate, trimethylolpropane tris(3-mercaptopropionate), pentaerythritol tetrakis(3-mercaptopropionate), pentaerythritol tetrakis(3-mercaptobutyrate), dipentaerythritol hexakis(3-mercaptopropionate), 1,3,4,6-tetrakis(2-mercaptoethyl)glycoluryl, 4-butanedithiol, 1,6-hexanedithiol, and 1,10-decanedithiol.

When using these curing agents, only one type of curing agent may be used, or two or more may be mixed. For applications such as underfill materials and general coating applications, it is preferable to use the amine, carboxylic acid, and/or acid anhydride compounds. Alternatively, for adhesive and flexible wiring board applications, amine compounds, especially dicyandiamide, are preferable from the viewpoint of workability, curability, and long-term stability. Alternatively, for semiconductor encapsulation material applications, solid-type phenolic compounds are preferable from the viewpoint of heat resistance of the cured product. In addition, for battery applications, aliphatic amines and thiol compounds are preferable from the viewpoint of low-temperature curing.

In addition, from the viewpoint that the effects of the present invention can be further exhibited, it is preferable that the compound (I) having reactivity with the glycidyl ether group-containing compound is a hydroxyl group-containing compound having a reversible bond.

Examples of the hydroxyl group-containing compound having a reversible bond include a hydroxyl group-containing compound in which a structural unit A' having one or more hydroxyl groups and a structural unit B' different from the A' are linked in A'-B'-A', and the structural unit A' and the structural unit B' are bonded by a reversible bond. **In** this case, the reversible bond is preferably a dissociation temperature of 120°C or higher from the viewpoint that the effect of the present invention is further exhibited.

The reversible bond is the same as the reversible bond in the glycidyl ether group-containing compound in the present invention.

The hydroxyl group-containing compound with a reversible bond can be, for example, those represented by the following formulas. Among these, from the viewpoint of the dissociation temperature of the reversible bond, it is preferable to use compounds represented by the following formulas (8-1), (8-2) and (9).

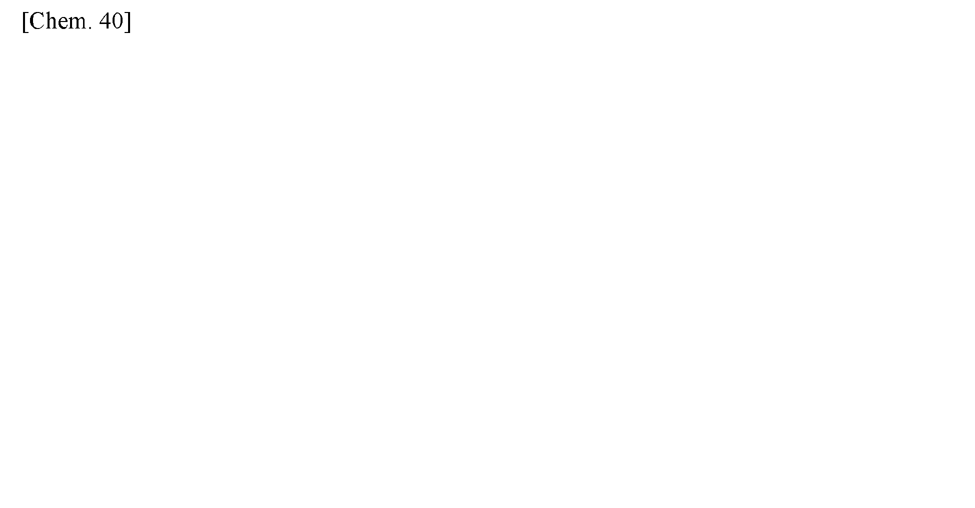

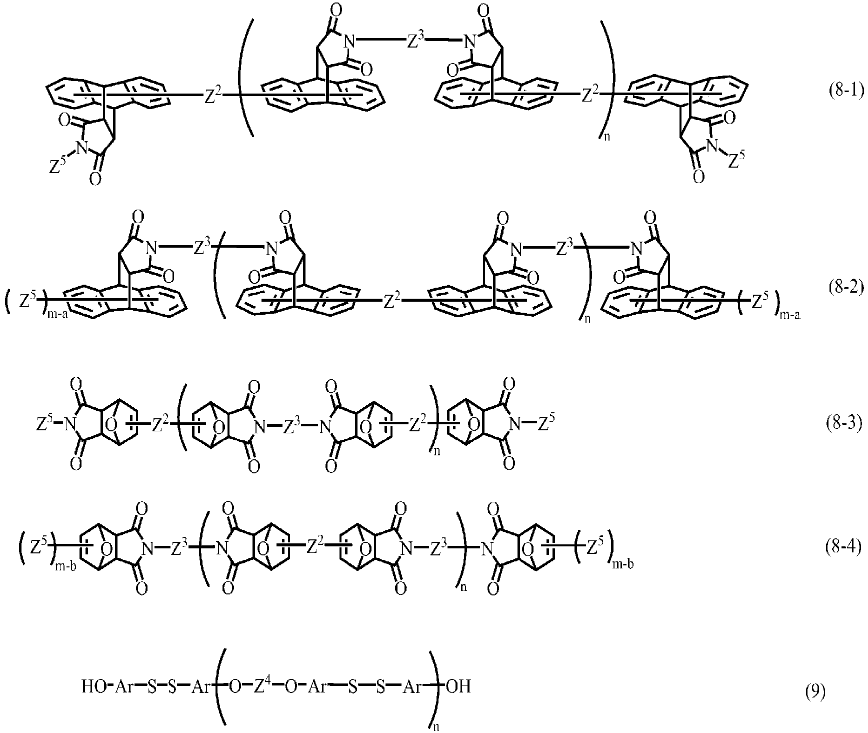

[Ar's in formula (9) are each independently a structure containing an unsubstituted aromatic ring or an aromatic ring having a substituent, and the anthracene-derived structure in formulas (8-1) and (8-2) and the furan-derived structure in formulas (8-3) and (8-4) may have a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group as a substituent.

In the formula, m-a is an integer of 1 to 10, m-b is an integer of 1 to 4, and n is an average value of repetition number of 0 to 10. Z⁵ is any of the structures represented by the following formula (10), Z² is any of the structures represented by the following formula (11), Z³ is any of the structures represented by the following formula (12), Z⁴ is any of the structures represented by the following formula (13) or (14), and the multiple structures present in one molecule may be identical or different from each other. 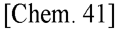 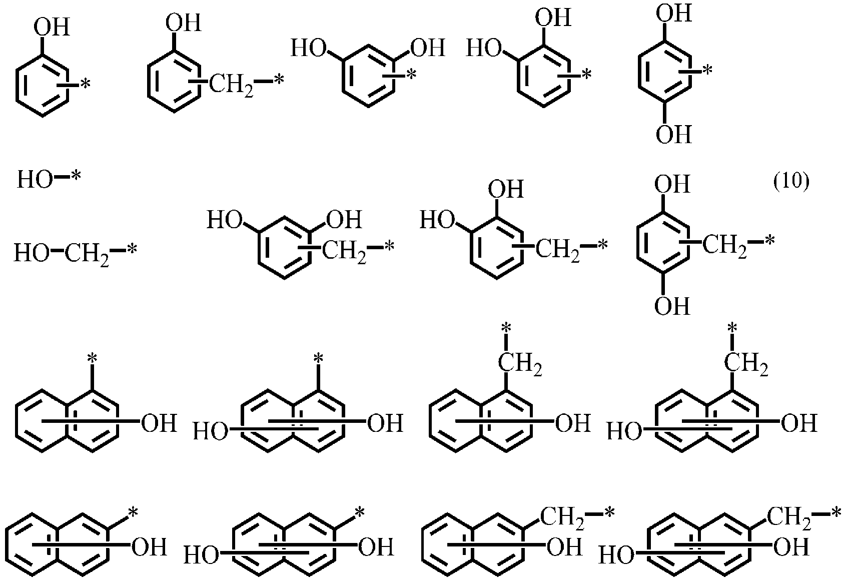 [The aromatic ring in the formula (10) may be substituted or unsubstituted, and * denotes a binding point. The hydroxyl group on the naphthalene ring in the formula indicates that the hydroxyl group may be bonded at any point.] 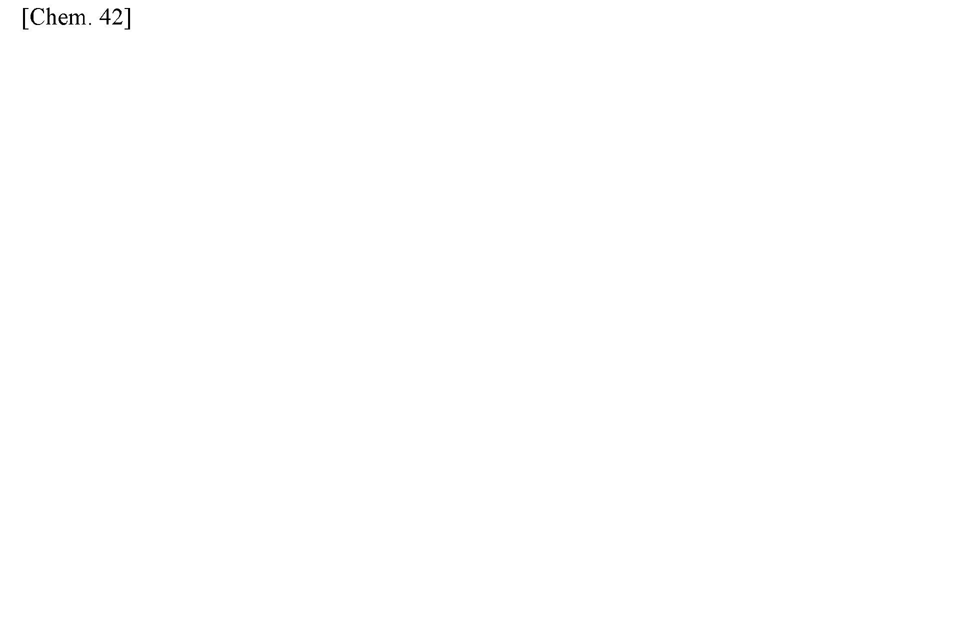 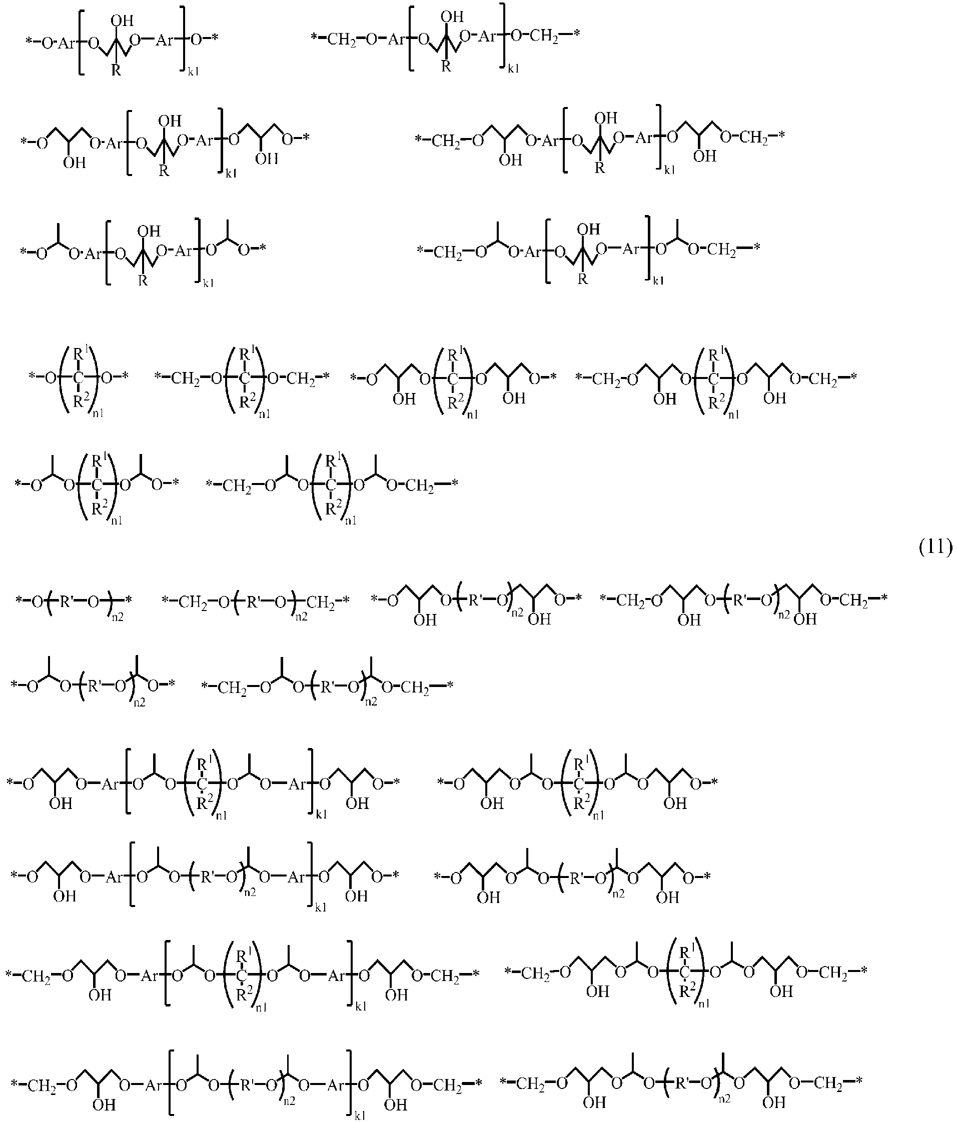 [In the formula (11),
Ar's are each independently a structure containing an unsubstituted aromatic ring or an aromatic ring having a substituent,
R¹ and R² are each independently a hydrogen atom, a methyl group, or an ethyl group,
R is a hydrogen atom or a methyl group,
R' is a divalent hydrocarbon group having 2 to 12 carbon atoms,
n1 is an integer of 2 to 16, and n2 is an average number of repeating units of 2 to 30,
k1 is an average value of repetition number, from 0.5 to 10,
p1 and p2 are each independently 0 to 5,
X is a structural unit represented by the following formula (11-1), and Y is a structural unit represented by the following formula (11-2), 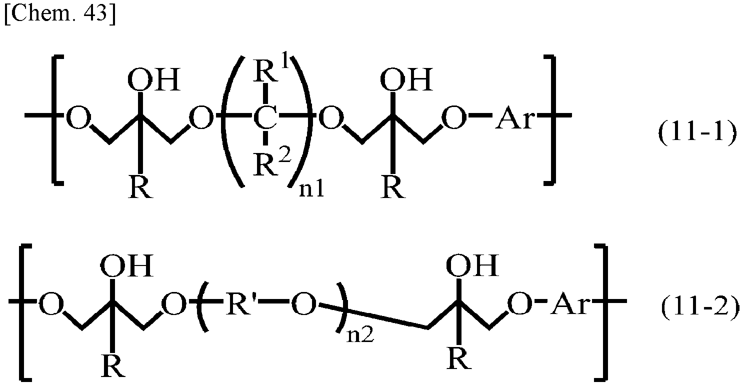
[In the formulas (11-1) and (11-2), Ar, R, R¹, R², R', n1, and n2 are the same as above.]
m1 and m2 are an average value of repetition number, each independently 0 to 25, and m1 + m2 ≥ 1.

Provided that a bond between the structural unit X represented by the formula (11-1) and the structural unit Y represented by the formula (11-2) may be random or blocked, and the respective total numbers of structural units X and Y present in one molecule are m1 and m2, respectively.] 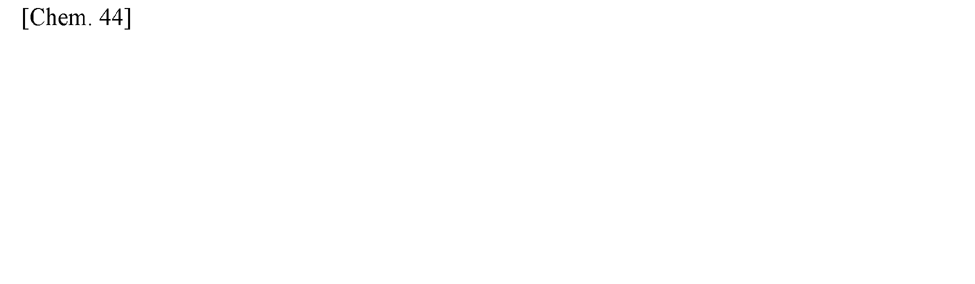 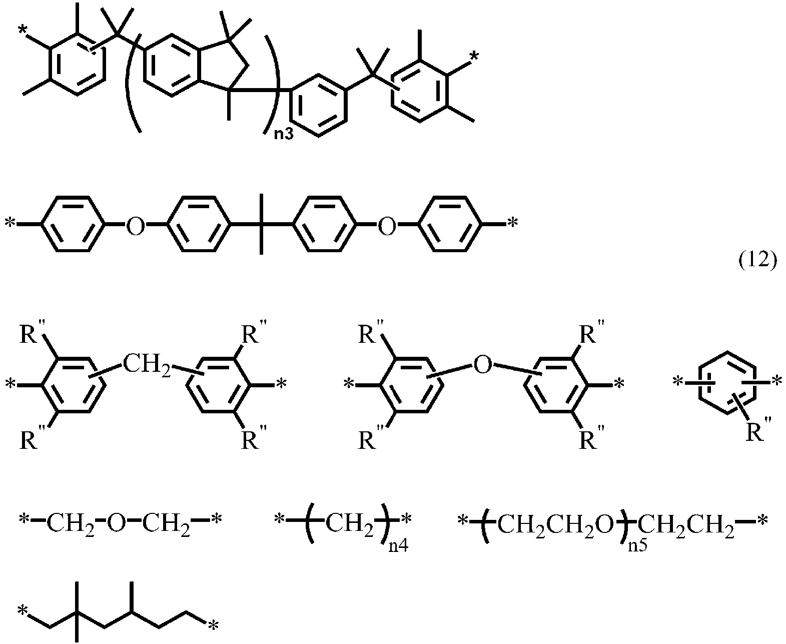 [In the formula (12), n3 and n5 are an average value of repetition number and are each 0.5 to 10, n4 is an integer of 1 to 16, and R"'s are each independently a hydrogen atom, a methyl group, or an ethyl group.] 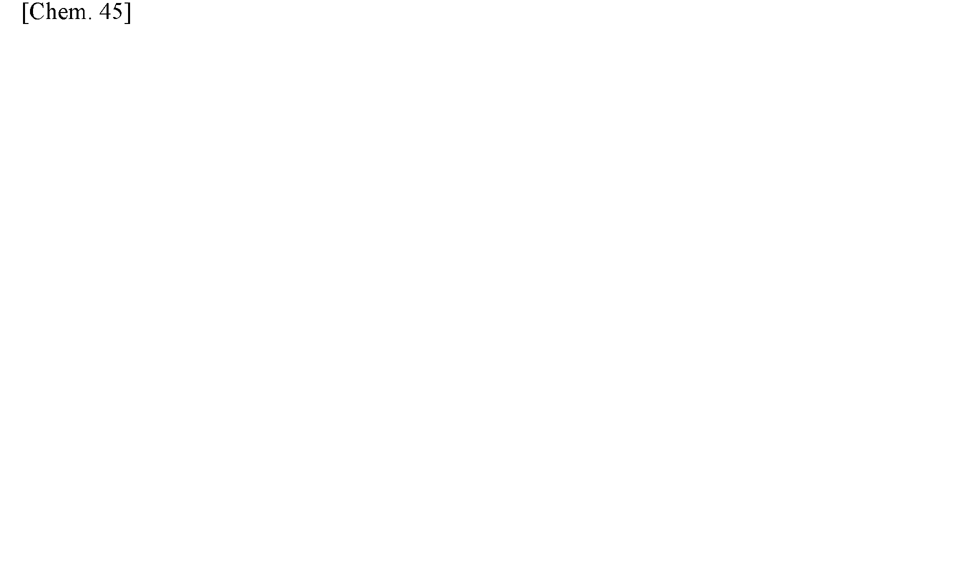 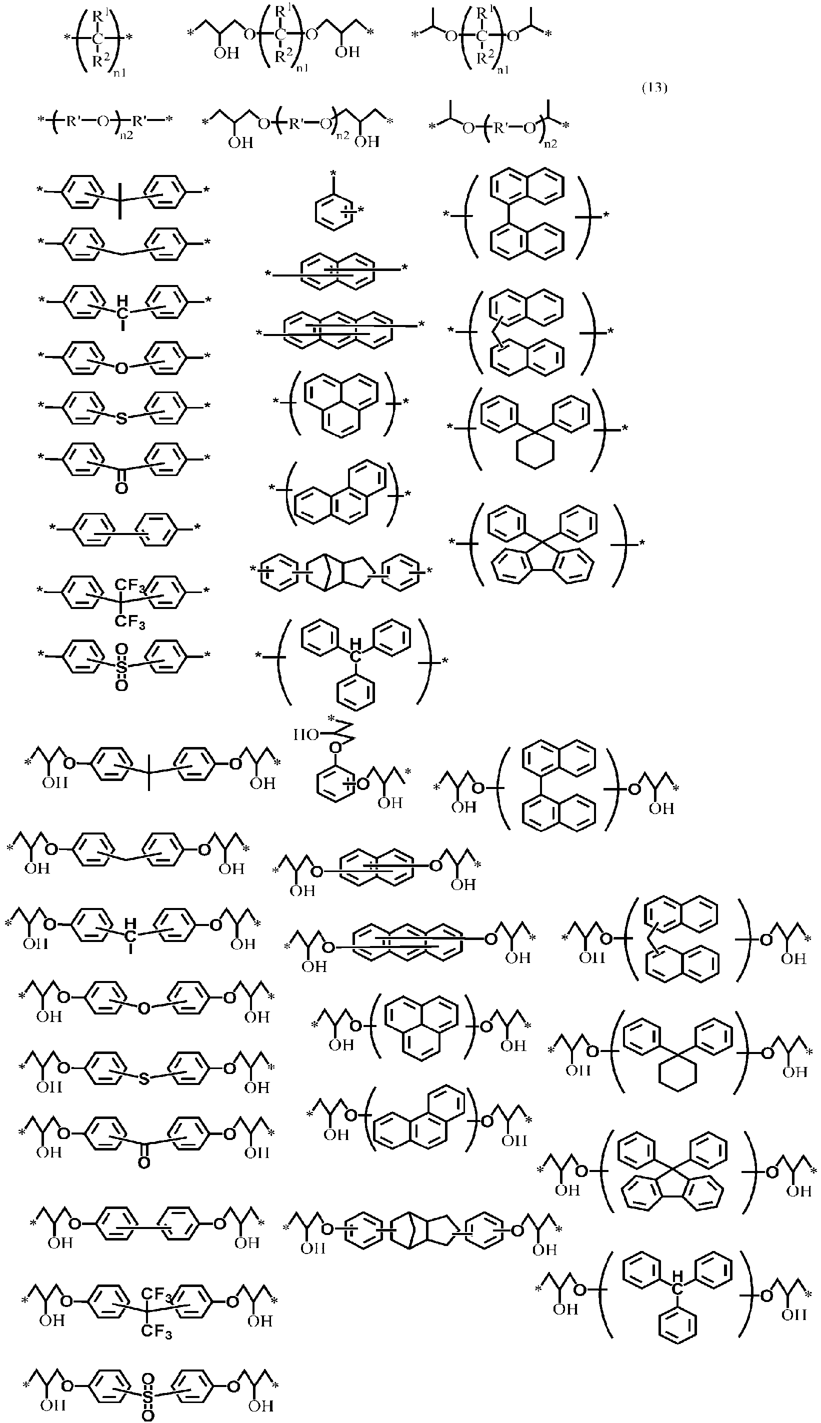 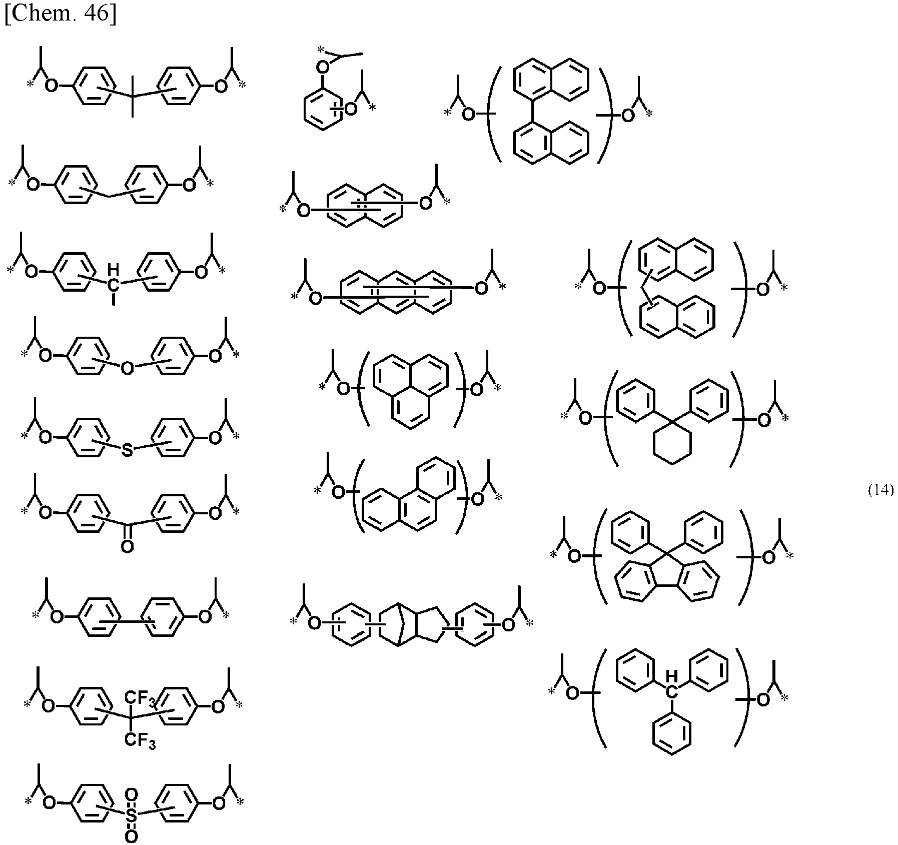 [In the formulas (13) and (14), R¹, R², R', n1, and n2 are the same as above].

The hydroxyl group-containing compounds with the reversible bond described above, for example, can be specifically represented by the following structural formulas.

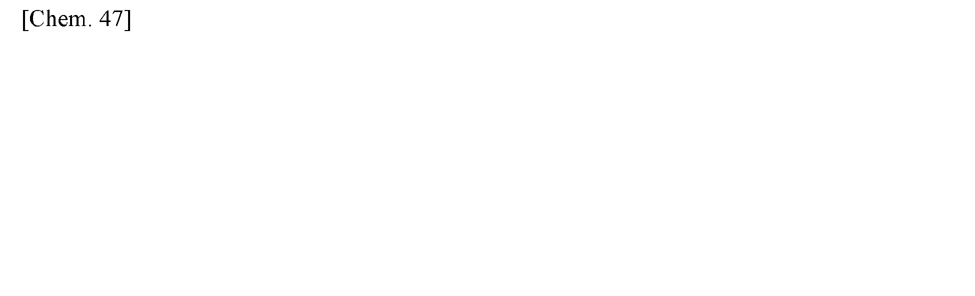

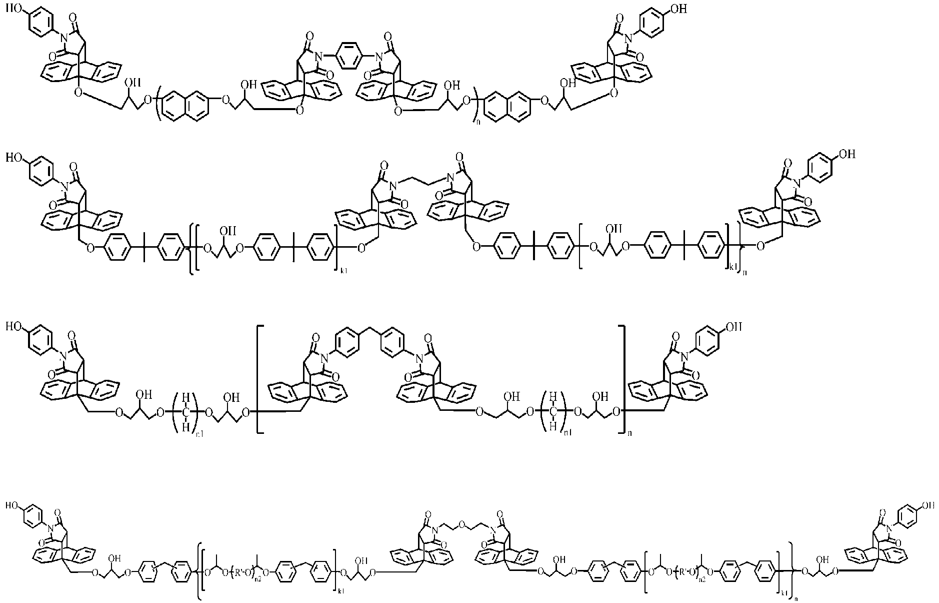

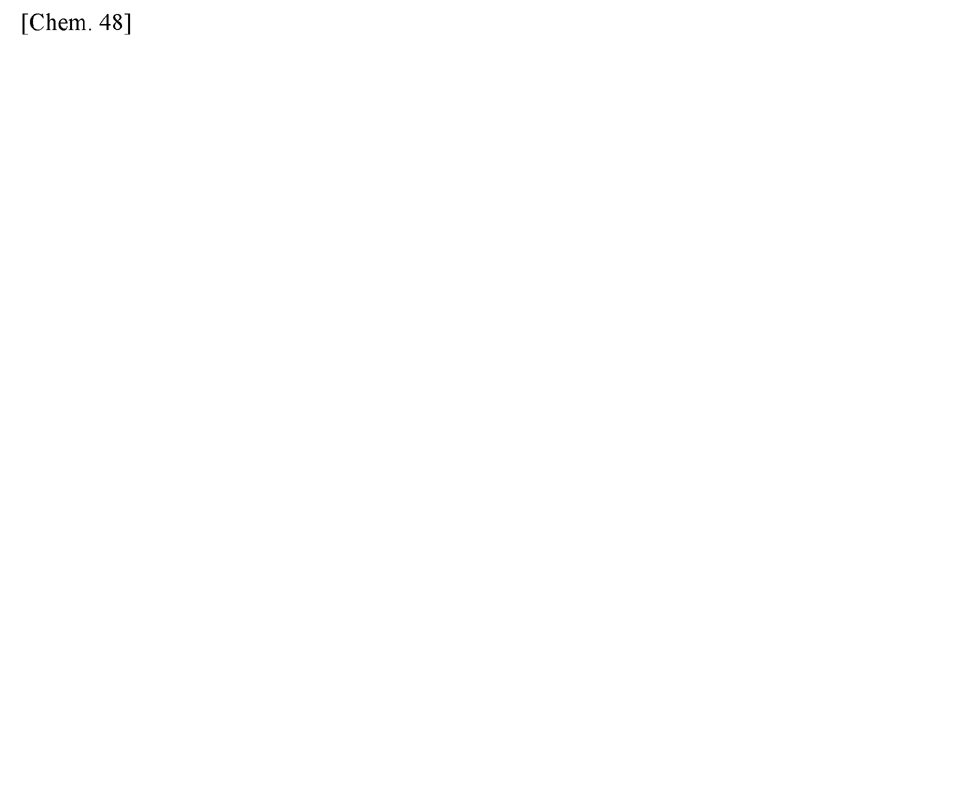

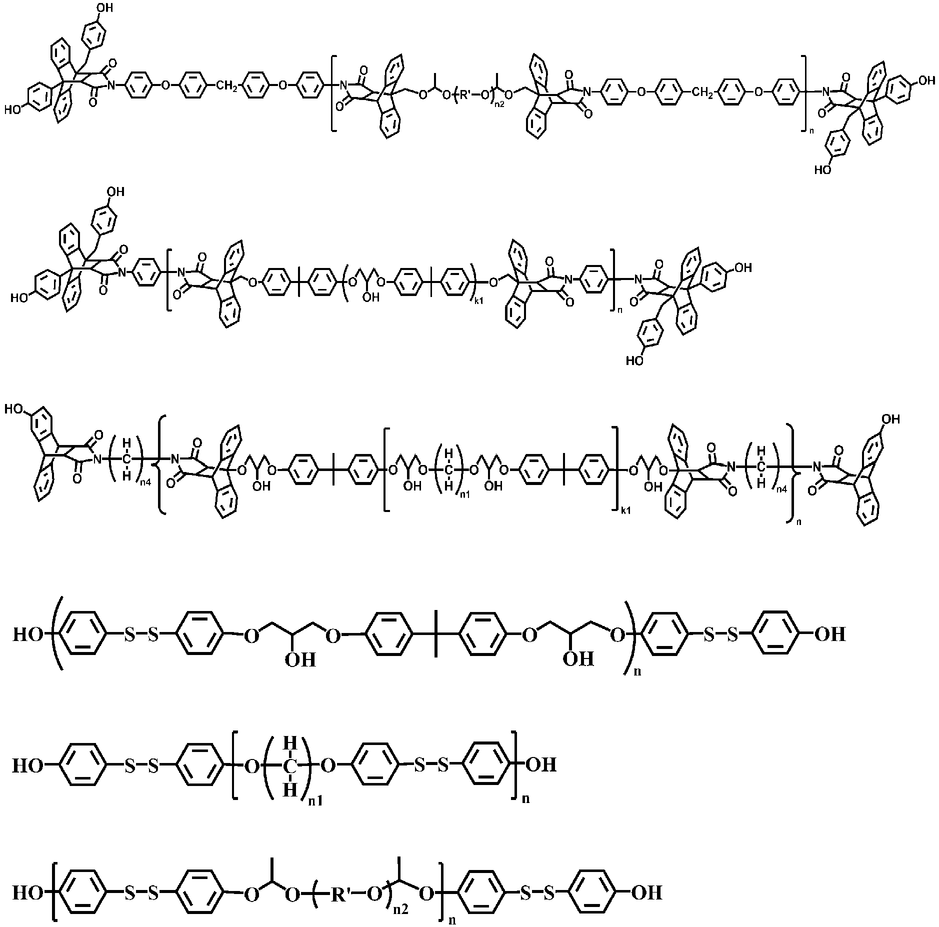

These hydroxyl group-containing compounds correspond to intermediates (precursors) of the above-mentioned glycidyl ether group-containing compound of the present invention, and therefore can be obtained by using commercially available raw materials such as maleimide, furan, and anthracene having a hydroxyl group according to the above-mentioned production method.

Furthermore, in the curable resin composition of the present invention, epoxy resins other than the glycidyl ether group-containing compound of the present invention can be used in combination as long as the effects of the present invention are not impaired.

Examples of the other epoxy resins include liquid epoxy resins such as a bisphenol A epoxy resin, a bisphenol F epoxy resin, a bisphenol S epoxy resin, a bisphenol AD epoxy resin, a polyhydroxybenzene epoxy resin, a polyhydroxynaphthalene epoxy resin, a biphenyl epoxy resin, and a tetramethyl biphenyl epoxy resin, a brominated epoxy resin such as a brominated phenolic novolac epoxy resin, a solid bisphenol A epoxy resin, a phenolic novolac epoxy resin, a cresol novolac epoxy resin, a triphenylmethane epoxy resin, a tetraphenylethane epoxy resin, a dicyclopentadiene-phenol addition reaction epoxy resin, a phenolic aralkyl epoxy resins, a phenylene ether epoxy resin, a naphthylene ether epoxy resin, a naphthol novolac epoxy resin, a naphthol aralkyl epoxy resin, a naphthol-phenol co-fused novolac epoxy resin, a naphthol-cresol co-fused novolac epoxy resin, an aromatic hydrocarbon formaldehyde resin-modified phenolic resin epoxy resin, and a biphenyl-modified novolac epoxy resin, which may be used alone or in combination of two or more kinds thereof, and it is preferable to select various kinds depending on the intended application and physical properties of the cured product.

Among these, it is preferable to use an epoxy resin having an epoxy equivalent weight of 100 to 10,000 g/eq in combination from the viewpoint of an an excellent balance between the curability and the crosslinking density of the obtained cured product, and it is particularly preferable to use an epoxy resin represented by the following formula (15) and having an epoxy equivalent weight of 500 to 10,000 g/eq. 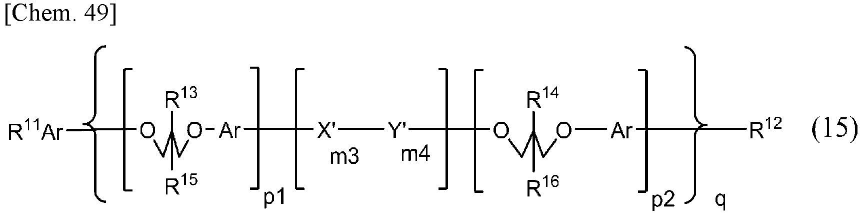
[in the formula (15), Ar's are each independently a structure containing an unsubstituted aromatic ring or an aromatic ring having a substituent,
X' is a structural unit represented by the following formula (15-1), and Y' is a structural unit represented by the following formula (15-2),

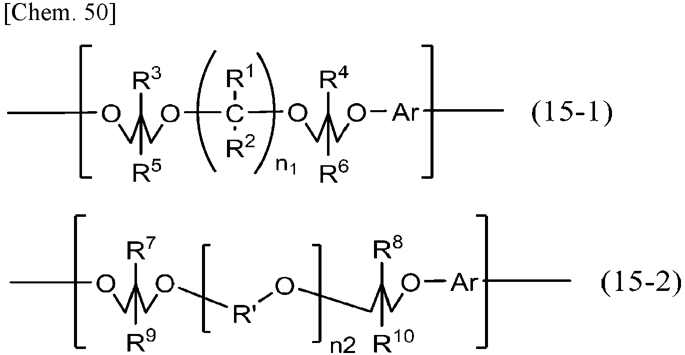

[In the formulas (15-1) and (15-2), Ar is the same as above,
R¹ and R² are each independently a hydrogen atom, a methyl group, or an ethyl group,
R' is a divalent hydrocarbon group having 2 to 12 carbon atoms,
R³, R⁴, R⁷, and R⁸ are each independently a hydroxyl group, a glycidyl ether group, or a 2-methylglycidyl ether group,
R⁵, R⁶, R⁹, and R¹⁰ are each independently a hydrogen atom or a methyl group,
n₁ is an integer of 4 to 16, and
n₂ is an average number of repeating units of 2 to 30]
R¹¹ and R¹² are each independently a glycidyl ether group or a 2-methyl glycidyl ether group,
R¹³ and R¹⁴ are each independently a hydroxyl group, a glycidyl ether group, or a 2-methyl glycidyl ether group,
R¹⁵ and R¹⁶ are hydrogen atoms or methyl groups,
m3, m4, p1, p2, and q are an average value of repetition number,
m3 and m4 are each independently 0 to 25 and m3 + m4 ≥ 1,
p1 and p2 are each independently 0 to 5, and
q is 0.5 to 5,
provided that a bond between the structural unit X' represented by the formula (15-1) and the structural unit Y' represented by the formula (15-2) may be random or blocked, and the respective total numbers of structural units X and Y present in one molecule are m3 and m4, respectively).

The epoxy resin represented by the general formula (15) may be combined alone as a curable resin composition, but from the viewpoint of adding more flexibility to the cured product and making it easier to disassemble, it is also preferable to use an epoxy resin with an epoxy equivalent weight of 100 to 300 g/eq in combination.

The epoxy resin that can be used in combination may have an epoxy equivalent weight in the range of 100 to 300 g/eq, but is not limited in the structure thereof. Examples of the epoxy resin include liquid epoxy resins such as a bisphenol A epoxy resin, a bisphenol F epoxy resin, a bisphenol S epoxy resin, a bisphenol AD epoxy resin, a polyhydroxybenzene epoxy resin, a polyhydroxynaphthalene epoxy resin, a biphenyl epoxy resin, and a tetramethyl biphenyl epoxy resin, a brominated epoxy resin such as a brominated phenolic novolac epoxy resin, a solid bisphenol A epoxy resin, a phenolic novolac epoxy resin, a cresol novolac epoxy resin, a triphenylmethane epoxy resin, a tetraphenylethane epoxy resin, a dicyclopentadiene-phenol addition reaction epoxy resin, a phenolic aralkyl epoxy resins, a phenylene ether epoxy resin, a naphthylene ether epoxy resin, a naphthol novolac epoxy resin, a naphthol aralkyl epoxy resin, a naphthol-phenol co-fused novolac epoxy resin, a naphthol-cresol co-fused novolac epoxy resin, an aromatic hydrocarbon formaldehyde resin-modified phenolic resin epoxy resin, and a biphenyl-modified novolac epoxy resin, which may be used alone or in combination of two or more kinds thereof, and it is preferable to select various kinds depending on the intended application and physical properties of the cured product.

Among these, it is preferable to use an epoxy resin having an epoxy equivalent weight of 100 to 300 g/eq among liquid epoxy resins such as a bisphenol A epoxy resin, a bisphenol F epoxy resin, a bisphenol S epoxy resin, a bisphenol AD epoxy resin, a polyhydroxybenzene epoxy resin, a polyhydroxynaphthalene epoxy resin, a biphenyl epoxy resin, and a tetramethylbiphenyl epoxy resin, and it is particularly preferable to use an epoxy resin having an epoxy equivalent weight of 100 to 300 g/eq among a bisphenol A epoxy resin, a bisphenol F epoxy resin, a bisphenol S epoxy resin, and a bisphenol AD epoxy resin.

The use ratio of the epoxy resin represented by the general formula (15) and the epoxy resin having the epoxy equivalent weight of 100 to 300 g/eq is not particularly limited, but from the viewpoint of easy phase separation in the cured product, the mass ratio of the former to the latter is 97:3 to 3:97, preferably 10:90 to 90: 10, and particularly preferably 80:20 to 20:80. Phase separation in the cured product results in a sea-island structure, which has both adhesion and stress relaxation properties in the cured product, especially high adhesion over a wide temperature range, and has the effect of reducing mold shrinkage before and after heat curing of the resin composition.

The concentration of the reversible bond in the curable resin composition of the present invention is preferably 0.10 mmol/g or higher based on the total mass of the curable components in the curable resin composition. According to such a configuration, both repairability and remoldability of the cured product obtained from the curable resin composition are further improved. The concentration of the reversible bond described above is more preferably between 0.10 and 3.00 mmol/g, and even more preferably between 0.15 and 2.00 mmol/g. Alternatively, when the glycidyl ether group-containing compound of the present invention has multiple reversible bonds, or when the hydroxyl group-containing compound having the above-mentioned reversible bonds is used as a curing agent alone or in combination with another curing agent, the concentration as the total of the reversible bonds is preferably 0.10 mmol/g or more, more preferably 0.10 to 3.00 mmol/g, and even more preferably 0.15 to 2.00 mmol/g, based on the total mass of the curable components in the curable resin composition. The concentration of reversible bond can be appropriately selected according to the glass transition temperature or the like as defined by the tan δ peak top of the dynamic viscoelasticity analyzer (DMA) of the target cured product. For example, if the glass transition temperature of the cured product is used as a guide, sufficient repairability and remoldability functions are likely to be exhibited even at the low end of the preferred range if the glass transition temperature of the cured product is near room temperature. On the other hand, if the glass transition temperature of the target cured product is above 100°C as a guide, the function is more likely to be exhibited at the high end of the preferred range. However, in the temperature range above the glass transition temperature measured by DMA, molecular mobility is generally high and sufficient repairability and remoldability functions are easily exhibited even when the concentration of the glycidyl ether group-containing compound is low. Accordingly, the effect of the repairability and remoldability functions can also be adjusted by, for example, adjusting the aging temperature for repair and the heating temperature for remolding in a timely manner. Thus, the relationship between the glass transition temperature of the cured product and the concentration of reversible bond is not limited to these factors.

The ratio of the total number of glycidyl ether groups to the total number of active groups having reactivity with the glycidyl ether groups in the curable resin composition of the present invention is not particularly limited, but from the viewpoint of good mechanical properties and the like of the obtained cured product, it is preferable that the amount of active groups having reactivity with the glycidyl ether groups be 0.4 to 1.5 equivalents per equivalent of the total number of glycidyl ether groups in the resin composition.

Alternatively, the curable resin composition may also contain a curing accelerator. Various types of curing accelerators can be used, such as urea compounds, phosphorus compounds, tertiary amines, imidazoles, imidazolines, organic acid metal salts, Lewis acids, and amine complex salts. When used for adhesive applications, from the viewpoint of excellent workability and low-temperature curing properties, a urea compound, particularly 3-(3,4-dichlorophenyl)-1,1-dimethylurea (DCMU), is preferable. For use in semiconductor encapsulation material applications, from the viewpoint of excellent curability, heat resistance, electrical properties, moisture resistance reliability, and the like, triphenylphosphine is preferable for phosphorus compounds, and 1,8-diazabicyclo-[5.4.0]-undecene is preferable for tertiary amines.

Examples of the phosphorus compound include alkyl phosphines such as ethyl phosphine and butyl phosphine; a primary phosphine such as phenyl phosphine; dialkyl phosphines such as dimethyl phosphine and dipropyl phosphine; secondary phosphines such as diphenyl phosphine and methyl ethyl phosphine; and tertiary phosphines such as trimethyl phosphine, triethyl phosphine, and triphenyl phosphine.

Examples of the imidazole include imidazole, 1-methylimidazole, 2-methylimidazole, 3-methylimidazole, 4-methylimidazole, 5-methylimidazole, 1-ethylimidazole, 2-ethylimidazole, 3-ethylimidazole, 4-ethylimidazole, 5-ethylimidazole, 1-n-propylimidazole, 2-n-propylimidazole, 1-isopropylimidazole, 2-isopropylimidazole, 1-n-butylimidazole, 2-n-butylimidazole, 1-isobutyl imidazole, 2-isobutyl-imidazole, 2-undecyl-1H-imidazole, 2-heptadecyl-1H-imidazole, 1,2-dimethylimidazole, 1,3-dimethylimidazole, 2,4-dimethylimidazole, 2-ethyl-4-methylimidazole, 1-phenyl imidazole, 2-phenyl-1H-imidazole, 4-methyl-2-phenyl-1H-imidazole, 2-phenyl-4-methylimidazole, 1-benzyl-2-methylimidazole, 1-benzyl-2-phenylimidazole, 1-cyanoethyl-2-methylimidazole, 1-cyanoethyl-2-ethyl-4-methylimidazole, 1-cyanoethyl-2-undecylimidazole, 1-cyanoethyl-2-phenyl-imidazole, 2-phenyl-imidazole isocyanuric acid adduct, 2-methylimidazole isocyanuric acid adduct, 2-phenyl-4,5-dihydroxymethylimidazole, 2-phenyl-4-methyl-5-hydroxymethylimidazole, 1-cyanoethyl-2-phenyl-4,5-di(2-cyanoethoxy)methylimidazole, 1-dodecyl-2-methyl-3-benzylimidazolium chloride, and 1-benzyl-2-phenyl imidazole hydrochloride.

Examples of the imidazoline compound include 2-methyl-imidazoline and 2-phenylimidazoline.

Examples of the urea compound include p-chlorophenyl-N,N-dimethyl urea, 3-phenyl-1,1-dimethyl urea, 3-(3,4-dichlorophenyl)-N,N-dimethyl urea, and N-(3-chloro-4-methylphenyl)-N',N'-dimethyl urea.

Alternatively, in the curable resin composition of the present invention, other thermosetting resins and thermoplastic resins may be used in combination as long as the effects of the present invention are not impaired.

Examples of other thermosetting resins include cyanate ester resins, resins having a benzoxazine structure, active ester resins, vinyl benzyl compounds, acrylic compounds, and copolymers of styrene and maleic anhydride. When the other thermosetting resins are used in combination, the amount thereof is not particularly limited as long as the effects of the present invention are not impaired, but is preferably in the range of 1 to 50 parts by mass in 100 parts by mass of the curable resin composition.

Examples of the cyanate ester resin include a bisphenol A-type cyanate ester resin, a bisphenol F-type cyanate ester resin, a bisphenol E-type cyanate ester resin, a bisphenol S-type cyanate ester resin, a bisphenol sulfide-type cyanate ester resin, a phenylene ether-type cyanate ester resin, a naphthylene ether-type cyanate ester resin, a biphenyl-type cyanate ester resin, a tetramethylbiphenyl-type cyanate ester resin, a polyhydroxynaphthalene-type cyanate ester resin, a phenolic novolac-type cyanate ester resin, a cresol novolac-type cyanate ester resin, a triphenylmethane-type cyanate ester resin, a tetraphenylethane-type cyanate ester resin, a dicyclopentadiene-phenol addition-reaction-type cyanate ester resin, a phenol-aralkyl-type cyanate ester resin, a naphthol novolac-type cyanate ester resin, a naphthol aralkyl-type cyanate ester resin, a naphthol-phenol co-fused novolac-type cyanate ester resin, a naphthol-cresol co-fused novolac-type cyanate ester resin, an aromatic hydrocarbon formaldehyde resin-modified phenolic resin-type cyanate ester resin, a biphenyl-modified novolac-type cyanate ester resin, and an anthracene-type cyanate ester resin. Each of these products may be used alone, or two or more may be used in combination of two or more kinds thereof.

Among these cyanate ester resins, particularly from the viewpoint of obtaining a cured product with excellent heat resistance, it is preferable to use a bisphenol A-type cyanate ester resin, a bisphenol F-type cyanate ester resin, a bisphenol E-type cyanate ester resin, a polyhydroxynaphthalene-type cyanate ester resin, a naphthylene ether-type cyanate ester resin, and a novolac-type cyanate ester resin, and from the viewpoint of obtaining a cured product with excellent dielectric properties, a dicyclopentadiene-phenol addition-reaction-type cyanate ester resin is preferable.

Examples of the resin having a benzoxazine structure is not particularly limited, but include reaction products of bisphenol F, formalin, and aniline (F-a type benzoxazine resin), reaction products of diaminodiphenylmethane, formalin, and phenol (P-d type benzoxazine resin), reaction products of bisphenol A, formalin, and aniline, reaction products of dihydroxydiphenyl ether, formalin, and aniline, reaction products of diaminodiphenyl ether, formalin, and phenol, reaction products of a dicyclopentadiene-phenol addition-type resin, formalin, and aniline, reaction products of phenolphthalein, formalin, and aniline, reaction products of diphenyl sulfide, formalin, and aniline. Each of these products may be used alone, or two or more may be used in combination of two or more kinds thereof.

The active ester resin is not particularly limited, but in general, compounds having two or more ester groups with high reactivity in one molecule, such as phenol esters, thiophenol esters, N-hydroxyamine esters, and esters of heterocyclic hydroxy compounds are preferably used. The active ester resin is preferably obtained by condensation reaction of a carboxylic acid compound and/or thiocarboxylic acid compound with a hydroxy compound and/or thiol compound. In particular, from the viewpoint of improving heat resistance, active ester resins obtained from carboxylic acid compounds or their halides and hydroxy compounds are preferable, and active ester resins obtained from carboxylic acid compounds or their halides and phenolic and/or naphthol compounds are more preferable. Examples of the carboxylic acid compound include benzoic acid, acetic acid, succinic acid, maleic acid, itaconic acid, phthalic acid, isophthalic acid, terephthalic acid, pyromellitic acid, or halides thereof. Examples of the phenol compound or the naphthol compound include hydroquinone, resorcin, bisphenol A, bisphenol F, bisphenol S, dihydroxydiphenyl ether, phenolphthalein, methylated bisphenol A, methylated bisphenol F, methylated bisphenol S, phenol, o-cresol, m-cresol, p-cresol, catechol, α-naphthol, β-naphthol, 1,5-dihydroxynaphthalene, 1,6-dihydroxynaphthalene, 2,6-dihydroxynaphthalene, dihydroxybenzophenone, trihydroxybenzophenone, tetrahydroxybenzophenone, phloroglucin, benzene triol, and a dicyclopentadiene-phenol addition resin.

As the active ester resin, specifically, an active ester resin containing a dicyclopentadiene-phenol addition structure, an active ester resin containing a naphthalene structure, an active ester resin that is an acetylated phenolic novolac, an active ester resin that is a benzoylated phenolic novolac, and the like are preferable, and among these, an active ester resin containing a dicyclopentadiene-phenol adduct structure and an active ester resin containing a naphthalene structure are more preferable from the viewpoint of superior peel strength improvement.

Furthermore, various novolac resins, addition polymerization resins of alicyclic diene compounds such as dicyclopentadiene and phenolic compounds, modified novolac resins of phenolic hydroxyl group-containing compounds and aromatic compounds containing alkoxy groups, phenolic aralkyl resins (xylox resin), naphthol aralkyl resins, trimethylolmethane resins, tetraphenylol ethane resins, biphenyl-modified phenolic resins, biphenyl-modified naphthol resins, aminotriazine-modified phenolic resins, and various vinyl polymers may be used in combination.

More specifically, examples of the various novolac resins include polymers obtained by reacting a phenol, a phenyl phenol, a resorcinol, a biphenyl, a bisphenol such as bisphenol A or bisphenol F, a phenolic hydroxyl group-containing compound such as naphthol or dihydroxynaphthalene, and an aldehyde compound under an acid catalyst condition.

Examples of the various types of vinyl polymers include homopolymers or copolymers of vinyl compounds such as poly hydroxy styrene, polystyrene, polyvinyl naphthalene, polyvinyl anthracene, polyvinyl carbazole, polyindene, polyacenaphthylene, polynorbornene, polycyclohexane, polytetracyclododecene, polynortricyclene, and poly(meth) acrylate.

The thermoplastic resin refers to a resin that can be melt molded by heating. Specific examples of the thermoplastic resin include a polyethylene resin, a polypropylene resin, a polystyrene resin, a rubber-modified polystyrene resin, an acrylonitrile-butadienestyrene (ABS) resin, an acrylonitrile-styrene (AS) resin, a polymethyl methacrylate resin, an acrylic resin, a polyvinyl chloride resin, a polyvinylidene chloride resin, a polyethylene terephthalate resin, an ethylene vinyl alcohol resin, an acetic acid cellulose resin, an ionomer resin, a polyacrylonitrile resin, a polyamide resin, a polyacetal resin, a polybutylene terephthalate resin, a polylactic acid resin, a polyphenylene ether resin, a modified polyphenylene ether resin, a polycarbonate resin, a polysulfone resin, a polyphenylene sulfide resin, a polyether imide resin, a polyether sulfone resin, a polyarylate resin, a thermoplastic polyimide resin, a polyamide imide resin, a polyether ether ketone resin, a polyketone resin, a liquid crystal polyester resin, a fluororesin, a syndiotactic polystyrene resin, and a cyclic polyolefin resin. These thermoplastic resins can be used alone or in combination of two or more kinds thereof

When these other resins are used, the mixing ratio of glycidyl ether group-containing compounds of the present invention and other resins can be set as desired depending on the application, but from the viewpoint of not interfering with the repairability and remoldability that the present invention achieves, the ratio of other resins is preferably 0.5 to 100 parts by mass of other resins to 100 parts by mass of the glycidyl ether group-containing compound of the present invention.

Alternatively, when the curable resin composition of the present invention is used for applications that require high flame retardancy, non-halogen flame retardants that contain virtually no halogen atoms may be blended.

Examples of the non-halogen flame retardant include phosphorus-based flame retardants, nitrogen-based flame retardants, silicone-based flame retardants, inorganic flame retardants, and organic metal salt flame retardants. The use of these flame retardants is not limited in any way, and the flame retardant may be used alone or in combination with other flame retardants of the same system, or a combination of flame retardants of different systems can be used.

The phosphorus-based flame retardant can be either an inorganic or organic. Examples of the inorganic compound include inorganic nitrogen-containing phosphorus compounds such as ammonium phosphate such as red phosphorus, ammonium phosphate, diammonium phosphate, triammonium phosphate, ammonium polyphosphate, and phosphoric acid amide.

Alternatively, the surface treatment is preferably performed for the red phosphorus for the purpose of preventing hydrolysis and the like, and examples of the surface treatment method include (i) a method for coating with inorganic compounds such as magnesium hydroxide, aluminum hydroxide, zinc hydroxide, titanium hydroxide, bismuth oxide, bismuth hydroxide, bismuth nitrate or mixtures thereof, (ii) a method for coating with a mixture of inorganic compounds such as magnesium hydroxide, aluminum hydroxide, zinc hydroxide, titanium hydroxide, and the like, and thermosetting resins such as phenol resin, or (iii) a method for double coating with thermosetting resin such as phenol resin on top of a film of inorganic compound such as magnesium hydroxide, aluminum hydroxide, zinc hydroxide, titanium hydroxide, and the like.

Examples of the organophosphorus compounds include, in addition to general-purpose organic phosphorus compounds such as phosphate compounds, phosphonic acid compounds, phosphinic acid compounds, phosphine oxide compounds, phosphorane compounds, and organic nitrogen-containing phosphorus compounds, cyclic organophosphorus compounds such as 9,10-dihydro-9-oxa-10-phosphaphenanthrene-10-oxide, 10-(2,5-dihydroxyphenyl)-10H-9-oxa-10-phosphaphenanthrene-10-oxide, and 10-(2,7-dihydroxynaphthyl)-10H-9-oxa-10-phosphaphenanthrene-10-oxide and derivatives made by reacting them with compounds such as epoxy and phenolic resins.

Although the blending amount of the phosphorus-based flame retardant is appropriately selected depending on the type of the phosphorus-based flame retardant, the other components of the resin composition, and the desired degree of incombustibility, for example, in 100 parts by mass of the resin composition in which all of the non-halogen flame retardant and the other filler and additives are blended, when red phosphorus is used as the non-halogen flame retardant, the amount is preferably in the range of 0.1 part by mass to 2.0 parts by mass, and when an organophosphorus compound is used, the amount is preferably in the range of 0.1 to 10.0 parts by mass and is more preferably in the range of 0.5 to 6.0 parts by mass.

Alternatively, when using the phosphorus-based flame retardant, hydrotalcite, magnesium hydroxide, a boron compound, zirconium oxide, black dye, calcium carbonate, zeolite, zinc molybdate, activated carbon, or the like may be used in combination with the phosphorus-based flame retardant.

Examples of the nitrogen-based polymer include a triazine compound, a cyanuric acid compound, an isocyanuric acid compound, and phenothiazine, and a triazine compound, a cyanuric acid compound, and an isocyanuric acid compound are preferable.

Examples of the triazine compound include, in addition to melamine, acetoguanamine, benzoguanamine, melon, melram, succinoguanamine, ethylene dimelamine, melamine polyphosphate, and triguanamine, for example, (1) aminotriazine sulfate compounds such as guanylmelamine sulfate, melem sulfate, and melam sulfate, (2) co-condensates of phenols such as phenol, cresol, xylenol, butylphenol, nonylphenol, etc., with melamines such as melamine, benzoguanamine, acetoguanamine, formoguanamine, and formaldehyde, (3) mixtures of the co-condensates of (2) above with phenol resins such as phenol-formaldehyde condensates, (4) a product obtained by further modifying (2) and (3) with paulownia oil, isomerized amani-oil, and the like.

Examples of the cyanuric acid compound include cyanuric acid and cyanuric acid melamine.

The amount of nitrogen-based flame retardant to be blended is appropriately selected according to the type of nitrogen-based flame retardant, other components of the resin composition, and the desired degree of flame retardancy, but for example, it is preferable to blend in the range of 0.05 to 10 parts by mass in 100 parts by mass of the resin composition containing non-halogen flame retardants and all other fillers, additives, and the like, and it is more preferable to blend in the range of 0.1 to 5 parts by mass.

Alternatively, when using the nitrogen-based flame retardant, metal hydroxides, molybdenum compounds, and the like may be used in combination.

The silicone-based flame retardant can be used with no particular restrictions as long as the silicone flame retardant is an organic compound containing silicon atoms. Examples of the silicone flame retardant include silicone oil, silicone rubber, and silicone resin. The amount of the silicone-based flame retardant to be blended is appropriately selected according to the type of silicone flame retardant, other components of the resin composition, and the desired degree of flame retardancy, but for example, it is preferable to blend in the range of 0.05 to 20 parts by mass in 100 parts by mass of the resin composition containing non-halogen flame retardants and all other fillers, additives, and the like. Alternatively, when using the silicone-based flame retardant, a molybdenum compound, alumina, or the like may be used in combination.

Examples of the inorganic flame retardant include a metal hydroxide, a metal oxide, a metal carbonate compound, a metal powder, a boron compound, and a low melting point glass.

Examples of the metal hydroxide include aluminum hydroxide, magnesium hydroxide, dromite, hydrotalcite, calcium hydroxide, barium hydroxide, and zirconium hydroxide.

Examples of the metal oxide include zinc molybdate, molybdenum trioxide, zinc stannate, tin oxide, aluminum oxide, iron oxide, titanium oxide, manganese oxide, zirconium oxide, zinc oxide, molybdenum oxide, cobalt oxide, bismuth oxide, chromium oxide, nickel oxide, copper oxide, and tungsten oxide.

Examples of the metal carbonate compound include zinc carbonate, magnesium carbonate, calcium carbonate, barium carbonate, basic magnesium carbonate, aluminum carbonate, iron carbonate, cobalt carbonate, and titanium carbonate.

Examples of the metal powder include aluminum, iron, titanium, manganese, zinc, molybdenum, cobalt, bismuth, chromium, nickel, copper, tungsten, and tin.

Examples of the boron compound include zinc borate, zinc metaborate, barium metaborate, boric acid, and borax.

Examples of the low melting point glass include glass-like compounds such as Sheeply (Boxui Brown Co.), hydrated glass SiO₂-MgO-H₂O, PbO-B₂O₃ based, ZnO-P₂O₅-MgO based, P₂O₅-B₂O₃-PbO-MgO based, P-Sn-O-F based, PbO-V₂O₅-TeO₂ based, Al₂O₃-H₂O based, and lead borosilicate.

The amount of the inorganic flame retardant to be blended is appropriately selected according to the type of inorganic flame retardant, other components of the resin composition, and the desired degree of flame retardancy, but for example, it is preferable to blend in the range of 0.05 to 20 parts by mass in 100 parts by mass of the resin composition containing non-halogen flame retardants and all other fillers, additives, and the like, and it is more preferable to blend in the range of 0.5 to 15 parts by mass.

Examples of the organic metal salt flame retardant include ferrocene, acetylacetonate metal complexes, organometallic carbonyl compounds, organocobalt salt compounds, organosulfonic acid metal salts, compounds in which metal atoms and aromatic or heterocyclic compounds are ionically or coordinately bonded.

The amount of the organic metal salt flame retardant to be blended is appropriately selected according to the type of organometallic salt flame retardant, other components of the resin composition, and the desired degree of flame retardancy, but for example, it is preferable to blend in the range of 0.005 to 10 parts by mass in 100 parts by mass of the resin composition containing non-halogen flame retardants and all other fillers, additives, and the like.

The curable resin composition of the present invention may contain a filler. Examples of the filler include an inorganic filler and an organic filler. Examples of the inorganic filler include inorganic fine particles.

Inorganic fine particles with excellent heat resistance include, for example, alumina, magnesia, titania, zirconia, silica (quartz, fumed silica, precipitated silica, silica anhydride, fused silica, crystalline silica, ultrafine amorphous silica, and the like), and the like; those with excellent heat conduction include boron nitride, aluminum nitride, alumina oxide, titanium oxide, magnesium oxide, zinc oxide, silicon oxide, diamond, and the like; those with excellent conductivity includes a metal filler and/or a metal-coated filler made of single metals or alloys (for example, iron, copper, magnesium, aluminum, gold, silver, platinum, zinc, manganese, stainless steel, and the like); those with excellent barrier properties include minerals such as mica, clay, kaolin, talc, zeolite, wollastonite, smectite, and the like, potassium titanate, magnesium sulfate, sepiolite, zonolite, aluminum borate, calcium carbonate, titanium dioxide, barium sulfate, zinc oxide, magnesium hydroxide; those with high refractive index include barium titanate, zirconium oxide, titanium oxide, and the like; those with photocatalytic properties includes photocatalytic metals such as titanium, cerium, zinc, copper, aluminum, tin, indium, phosphorus, carbon, sulfur, ruthenium, nickel, iron, cobalt, silver, molybdenum, strontium, chromium, barium, and lead, a composite of the above metals, and the oxides thereof; those with excellent wear resistance include metals such as silica, alumina, zirconia, and magnesium oxide, and the composites and oxides thereof; those with excellent conductivity include metals such as silver and copper, tin oxide, indium oxide, and the like; those with excellent insulating properties include silica and the like; those with excellent UV shielding include titanium dioxide, zinc oxide, and the like. These inorganic fine particles may be selected depending on the application in a timely manner, and can be used alone or in combination of two or more kinds thereof. Alternatively, in addition to the properties listed in the examples, the above inorganic fine particles have various other properties and thus, may be selected to suit the application in a timely manner.

For example, when silica is used as inorganic fine particles, there are no particular limitations, and known silica fine particles such as powdered silica and colloidal silica can be used. Examples of commercially available powdered silica fine particles include Aerosil 50 and 200 (trade names, manufactured by Nippon Aerosil Co., Ltd.), Sildex H31, H32, H51, H52, H121, and H122 (trade names, manufactured by Asahi Glass Co., Ltd.), E220A and E220 (trade names, manufactured by Nippon Silica Industrial Co., Ltd.), SYLYSIA 470 (trade name, manufactured by Fuji Silysia Co., Ltd.), and SG Flake (trade name, manufactured by Nippon Sheet Glass Co., Ltd.).

Alternatively, examples of commercially available colloidal silica include methanol-silica sol, IPA-ST, MEK-ST, NBA-ST, XBA-ST, DMAC-ST, ST-UP, ST-OUP, ST-20, ST-40, ST-C, ST-N, ST-O, ST-50, and ST-OL manufactured by Nissan Chemical Industries, Ltd.

Surface-modified silica fine particles may be used, for example, the silica fine particles are surface treated with a reactive silane coupling agent having hydrophobic groups or modified with a compound having (meth)acryloyl groups. Examples of commercially available powdered silica modified with a compound having (meth)acryloyl groups include Aerosil RM50 and R711 manufactured by Nippon Aerosil Co., Ltd., and examples of commercially available colloidal silica modified with compounds having (meth)acryloyl groups include MIBK-SD manufactured by Sangyo Chemical Industry Co.

The shape of the silica fine particles is not limited and can be spherical, hollow, porous, rod-shaped, plate-shaped, fibrous, or irregularly shaped. Alternatively, the primary particle diameter is preferably in the range of 5 to 200 nm.

As titanium oxide fine particles, not only body pigments but also ultraviolet light responsive photocatalysts can be used, such as anatase titanium dioxide, rutile titanium dioxide, brookite titanium dioxide. Furthermore, particles designed to respond to visible light by doping different elements into the crystal structure of titanium oxide can also be used. Anionic elements such as nitrogen, sulfur, carbon, fluorine, and phosphorus, and cationic elements such as chromium, iron, cobalt, and manganese are suitable as elements to be doped into titanium oxide. Alternatively, the form can be a powder, a sol or slurry dispersed in an organic solvent or in water. Examples of commercially available powdered titanium oxide fine particles include Aerosil P-25 manufactured by Nippon Aerosil Co., Ltd., and ATM-100 manufactured by Tayca Corporation. Alternatively, examples of commercially available slurry-like titanium oxide fine particles include TKD-701 manufactured by Tayca Corporation.

The curable resin composition of the present invention may further contain a fibrous substrate. The fibrous substrate is not particularly limited, but those used for a fiber-reinforced resin are preferable, and examples thereof include inorganic fibers and organic fibers.

Examples of the inorganic fiber include inorganic fibers such as carbon fibers, glass fibers, boron fibers, alumina fibers, and silicon carbide fibers, mineral fibers such as carbon fibers, activated carbon fibers, graphite fibers, tungsten carbide fibers, silicon carbide fibers (silicon carbide fibers), ceramic fibers, natural fibers, and brown balls, boron nitride fibers, boron carbide fibers, and metal fibers. Examples of the metal fibers include aluminum fibers, copper fibers, brass fibers, stainless steel fibers, and steel fibers.

Examples of the organic fiber include synthetic fibers made of a resin material such as polybenzazole, aramid, PBO (polyparaphenylene benzoxazole), polyphenylene sulfide, polyester, acrylic, polyamide, polyolefin, polyvinyl alcohol, and polyarylate, and natural fibers such as cellulose, pulp, cotton, wool, and silk, and regenerated fibers such as proteins, polypeptides, and arginic acid.

Among these, carbon and glass fibers are particularly preferred because of the wide range of industrial applications. Among these, only one type may be used, or multiple types may be used at the same time.

The fibrous substrate may be an aggregate of fibers, and the fibers may be continuous or discontinuous, woven or non-woven. Alternatively, the fibrous substrate can also be a fiber bundle with fibers aligned in one direction or a sheet with fiber bundles arranged. Alternatively, the fibrous substrate can also be a three-dimensional shape with a thicker aggregate of fibers.

The curable resin composition of the present invention may use a dispersion medium for the purpose of adjusting the solid content and viscosity of the resin composition. The dispersion medium can be any liquid medium that does not impair the effects of the present invention, and can include various organic solvents, liquid organic polymers, and the like.

Examples of the organic solvent include ketones such as acetone, methyl ethyl ketone (MEK), and methyl isobutyl ketone (MIBK), annular ethers such as tetrahydrofuran (THF) and dioxolane, esters such as methyl acetate, ethyl acetate, and butyl acetate, and aromatics such as toluene and xylene, and alcohols such as carbitol, cellosolve, methanol, isopropanol, butanol, propylene glycol monomethyl ether, which may be used alone or in combination, and methyl ethyl ketone is preferable from the viewpoint of volatility and solvent recovery during coating.

Examples of the liquid organic polymer include a liquid organic polymer which does not directly contribute to a curing reaction, and examples thereof include an acrylic polymer (FLOWLEN WK-20: manufactured by Kyowa Chemical Industry Co., Ltd.), an amine salt of a special modified phosphate ester (HIPLAADED-251: Kusumoto Chemicals, Ltd.), and a modified acrylic block copolymer (DISPERBYK 2000; manufactured by BYK Chemie).

The resin composition of the present invention may have other components. Examples thereof include a catalyst, a polymerization initiator, an inorganic pigment, an organic pigment, an extender pigment, a clay mineral, a wax, a surfactant, a stabilizer, a flow regulating agent, a coupling agent, a dye, a leveling agent, a rheology control agent, an ultraviolet light absorbent, an antioxidant, a flame retardant, a plasticizer, and a reactive diluent.

By curing the resin composition of the present invention, a cured product can be obtained. Curing can be performed at room temperature or by heating. When performing thermal curing, curing may be done in a single heating step or through a multi-step heating process.

Alternatively, the curable resin compositions of the present invention can also be cured with active energy rays. In this case, a photocationic polymerization initiator can be used as a polymerization initiator. Visible light, ultraviolet light, X-rays, electron beams, or the like can be used as active energy rays.

As the photocationic polymerization initiator, aryl-sulfonium salts, aryl-iodonium salts, and specifically, arylsulfonium hexafluorophosphate, arylsulfonium hexafluoroantimonate, arylsulfonium tetrakis(pentafluoro)borate, tri(alkylphenyl)sulfonium hexafluorophosphate, and the like can be used. The photocationic polymerization initiator may be used alone or in combination with two or more kinds thereof.

The curable resin composition of the present invention may be prepared by uniformly mixing the above-described components, and the method for mixing is not particularly limited. For example, the curable resin composition can be prepared by uniformly mixing using a pot mill, ball mill, bead mill, roll mill, homogenizer, super mill, homo-disper, universal mixer, Banbury mixer, kneader, and the like.

The curable resin composition of the present invention may be prepared by dissolving the glycidyl ether group-containing compound of the present invention and a compound (I) having reactivity with the glycidyl ether group-containing compound, as well as the curing agents, fillers, fibrous substrates, dispersion medium, and resins other than the various compounds mentioned above that can be used in combination, as needed, in the dispersion medium such as organic solvents mentioned above. After dissolution, the solvent is removed, followed by decompression drying in a vacuum oven and the like, whereby the curable resin composition can be obtained. Alternatively, the curable resin composition of the present invention may be in a state in which the above-described constituent materials are uniformly mixed. At this time, the mixture is preferably uniformly mixed in a mixer or the like. The mixing ratio of each of the constituent materials can be appropriately adjusted depending on the desired properties of the cured product, such as mechanical strength, heat resistance, repairability, and remoldability. Alternatively, in the production of the curable resin composition, the order in which the constituent materials are mixed is not particularly limited.

The cured product of the present invention is obtained by curing the compound (I) having reactivity with the glycidyl ether group-containing compound by the glycidyl ether group-containing compound of the present invention. The curing method can be appropriately selected from known methods depending on the properties of the compound (I) having reactivity with the glycidyl ether group-containing compound to be used.

Since the cured product of the present invention is cured by the glycidyl ether group-containing compound of the present invention as described above, it is possible to develop an appropriate crosslinking density and thereby maintain good mechanical strength. Alternatively, when the cured product of the present invention is scratched or subjected to mechanical energy such as external force, the reversible bond is broken, but since the equilibrium shifts in the binding direction, an adduct is formed again, which is thought to enable the damage to be repaired and the product to be remolded.

The structure of the obtained cured product can be confirmed by infrared absorption (IR) spectroscopy using Fourier transform infrared spectroscopy (FT-IR) or the like, elemental analysis, X-ray scattering, or the like.

As described above, the cured product according to an embodiment of the present invention can be obtained by using the glycidyl ether group-containing compound of the present invention as one component of the curable resin composition, but the intermediate of a conjugated diene or parent diene intermediate described above, which is an intermediate of glycidyl ether group-containing compounds, can be used in combination with an addition-reactive compound by the Diels-Alder reaction to from a cured product while forming the glycidyl ether group-containing compound during the curing process (while synthesizing in situ).

For example, when the curing reaction is performed by using the compound represented by the above formula (1-1)', a maleimide having a glycidyl ether group, and the compound (I) having reactivity with the the glycidyl ether group-containing compound as essential raw materials, in the process of the curing reaction, a glycidyl ether group-containing compound represented by the formula (1-1) can be obtained, and as the curing reaction progresses, a cured product can be obtained. The maleimide having a glycidyl ether group that can be used in this process is the same as described above.

Alternatively, when the curing reaction is performed by using the compound represented by the above formula (1-2)', an anthracene having a glycidyl ether group, and the compound (I) having reactivity with the the glycidyl ether group-containing compound as essential raw materials, in the process of the curing reaction, a glycidyl ether group-containing compound represented by the formula (1-2) can be obtained, and as the curing reaction progresses, a cured product can be obtained. The anthracene having a glycidyl ether group that can be used in this process is the same as described above.

The curable resin composition of the present invention and a cured product produced from the curable resin composition are excellent in both heat resistance and repairability, and are also remoldable, and are useful for the following applications.

The curable resin cured product of the present invention can be made into a laminate by being laminated with a base material. Inorganic materials such as metal and glass, as well as organic materials such as plastic and wood, can be used as the base material for the laminate, depending on the application in a timely manner, and the materials may be in the form of a laminated product, such as a flat plate, a sheet-shaped product, or may have a three-dimensional structure, or may be three-dimensional. The base material may be of any shape to suit the purpose, such as having curvature on all or part of the surface. Alternatively, the hardness and thickness of the base material are not limited. Alternatively, a multi-laminate in which a first base material, a layer formed of a cured product of the curable resin composition of the present invention, and a second base material are laminated in this order may be used. Since the curable resin composition of the present embodiment has excellent adhesive properties, the curable resin composition can be suitably used as an adhesive to bond the first base material to the second base material. Alternatively, the curable resin cured product of the present invention may be used as the base material, and the cured product of the present invention may be further laminated.

Alternatively, the curable resin cured product of the present invention is particularly suitable for bonding dissimilar materials because of the ability to relieve stress. For example, even in a laminate of the base material of a metal and/or metal oxide and the second base material of a dissimilar material, such as a plastic layer, the adhesive strength is maintained due to the stress relaxation ability of the cured product of the present invention.

In the laminate obtained by laminating the cured product of the present invention and a base material, the layer containing the cured product may be formed by coating or molding directly onto the base material, or an already molded composition may be laminated thereon. In the case of direct coating, examples of the coating method include a spray method, a spin coating method, a dipping method, a roll coating method, a blade coating method, a doctor roll method, a doctor blade method, a curtain coating method, a slit coating method, a screen printing method, and an inkjet method. Examples of the direct molding include in-mold molding, insert molding, vacuum molding, extrusion lamination molding, and press molding. When the molded composition is laminated, the uncured or semi-cured composition layer may be laminated and then cured, or a layer containing a cured product obtained by completely curing the composition may be laminated on the base material. Alternatively, lamination may be achieved by applying onto the cured product of the present invention a precursor that can become a base material and curing it, or may be achieved by bonding the precursor that can become a base material or the composition of the present invention in an uncured or semi-cured state and then curing it. Examples of the precursor that can become a base material is not particularly limited, but include various curable resin compositions.

The cured product obtained by using the curable resin composition of the present invention has particularly high adhesion to metals and/or metal oxides and thus, can be used particularly well as primers for metals. Examples of metals include copper, aluminum, gold, silver, iron, platinum, chromium, nickel, tin, titanium, zinc, various alloys, and composite materials thereof, and examples of metal oxides include single oxides and/or composite oxides of these metals. In particular, the cured product of the present invention has excellent adhesive strength to iron, copper, and aluminum, and thus, can be used well as an adhesive for iron, copper, and aluminum.

The curable resin composition of the present invention can be suitably used as an adhesive for structural members in the fields of automobiles, trains, civil engineering and construction, electronics, aircraft, and the space industry. The adhesive can maintain high adhesiveness without being affected by changes in the temperature environment, even when used to bond dissimilar materials, such as between a metal and a non-metal, and is unlikely to peel off. Alternatively, in addition to structural member applications, the adhesive can also be used as an adhesive for general office use, medical use, carbon fiber, storage battery cells, modules and cases, and the like, and can be used as optical component bonding adhesives, optical disc bonding adhesives, printed wiring board mounting adhesives, die bonding adhesives, semiconductor adhesives such as underfill, BGA reinforcement underfill, anisotropic conductive film, anisotropic conductive paste, and other mounting adhesives.

When the curable resin composition of the present invention has a fibrous substrate, and the fibrous substrate is a reinforcing fiber, the curable resin composition containing the fibrous substrate can be used as a fiber-reinforced resin. The method of incorporating the fibrous substrate into the composition is not particularly limited as long as the effects of the present invention are not impaired, and examples thereof include a method in which the fibrous substrate and the composition are combined by a method such as kneading, coating, impregnation, injection, and pressure bonding, which can be selected in a timely manner depending on the form of the fibers and the application of the fiber-reinforced resin.

The method for molding the fiber-reinforced resin is not particularly limited. When producing a plate-shaped product, an extrusion molding method is generally used, but flat press can also be used. In addition, an extrusion molding method, a blow molding method, a compression molding method, a vacuum molding method, an injection molding method, or the like can be used. Alternatively, if a film-shaped products is to be produced, the solution casting method can be used in addition to the melt extrusion method, and when a melt molding method is used, the examples thereof include inflation film molding, cast molding, extrusion lamination molding, calendar molding, sheet molding, fiber molding, blow molding, injection molding, rotational molding, and coated molding. Alternatively, in the case of a resin that is cured by active energy rays, a cured product can be produced by using various curing methods using active energy rays. In particular, when thermosetting resin is the main component of a matrix resin, an example of the molding method is to prepreg the molding material and pressurize and heat the material by pressing or autoclaving, and other examples thereof include resin transfer molding (RTM), vacuum assist resin transfer molding (VaRTM), a laminated molding, and hand layup molding.

The curable resin composition of the present invention can be used as molding materials for large cases, motor housings, pouring materials inside cases, gears and pulleys, and the like because the cured product made from the curable resin composition has both good heat resistance and repairability, and are remoldable. The cured product may be a cured product of a resin alone or a cured product of fiber reinforced, such as a glass chip.

The fiber-reinforced resin can form a state called uncured or semi-cured prepreg. After distributing the product in a prepreg state, final curing may be performed to form a cured product. When forming a laminate, it is preferable to form the prepreg and then layer the other layers before final curing to form a laminate in which the layers adhere closely to each other. Although there is no particular limit to the mass ratio of the composition and fibrous substrate used in this process, it is usually preferable to prepare the resin content in the prepreg to be 20 to 60% by mass.

The cured product of the present invention has good heat resistance and repairability, as well as remoldability, and can be used as a heat-resistant material and electronic material. In particular, the cured product is suitable for semiconductor encapsulation materials, circuit boards, build-up films, build-up substrates, or the like, as well as adhesives and resist materials. Alternatively, the cured product is also suitable for use as a matrix resin for fiber-reinforced resins, and is particularly suitable as a prepreg with high heat resistance. The heat resistant member and the electronic component obtained in this manner can be suitably used for various applications, and the examples thereof include, but are not limited to, industrial machinery parts, general machinery parts, parts for automobiles, railroads, vehicles, or the like, aerospace and aviation-related parts, electronic and electrical parts, construction materials, container and packaging parts, household goods, sports and leisure goods, and housing parts for wind power generation.

Among these, the heat resistant member can be suitably used as an adhesive for structural members in the automobile, train, civil engineering and construction, electronics, aircraft, and space industries, taking advantage of the excellent flexibility in the cured product. The adhesive of the present invention can maintain high adhesiveness without being affected by changes in the temperature environment, even when used to bond dissimilar materials, such as between a metal and a non-metal, and is unlikely to peel off. Alternatively, in addition to structural member applications, the adhesive of the present invention can also be used as an adhesive for general office use, medical use, carbon fiber, storage battery cells, modules and cases, and the like, and the examples thereof include optical component bonding adhesives, optical disc bonding adhesives, printed wiring board mounting adhesives, die bonding adhesives, semiconductor adhesives such as underfill, BGA reinforcement underfill, anisotropic conductive film, anisotropic conductive paste, and other mounting adhesives.

Hereinafter, a representative product will be described as an example.

### 1. Semiconductor Encapsulation Material

Examples of the method for obtaining a semiconductor encapsulation material from the resin composition of the present invention include a method for thoroughly melting and mixing the resin composition, and compounding agents such as a curing accelerator and an inorganic filler by using an extruder, a kneader, a roll, or the like, as necessary, until the material becomes uniform. In this case, fused silica is usually used as the inorganic filler, but when used as a high thermal conductivity semiconductor encapsulant for power transistors and power ICs, it is recommended to use crystalline silica, alumina, or silicon nitride, which have higher thermal conductivity than fused silica. The filling rate of the inorganic filler per 100 parts by mass of the curable resin composition is preferably in the range of 30 to 95% by mass, and among these, 70 or more parts by mass is more preferable, and 80 or more parts by mass is even more preferable, in order to improve flame retardancy, moisture resistance and solder cracking resistance, and to lower the coefficient of linear expansion.

### 2. Semiconductor Device

Semiconductor package molding to obtain a semiconductor device from the curable resin composition of the present invention includes a method for molding the semiconductor encapsulation materials by using a pouring mold, or transfer molding machine, injection molding machine, or the like, and further heating at 50 to 250°C for 2 to 10 hours.

### 3. Printed Circuit Board

An example of the method for obtaining a printed circuit board from the composition of the present invention is to laminate the above prepregs in the usual manner, overlap copper foil as appropriate, and heat and press bond the prepregs at 170 to 300°C for 10 minutes to 3 hours under pressure of 1 to 10 MPa.

### 4. Flexible Substrate

The method for producing a flexible substrate from the crosslinkable resin composition of the present invention includes a method including the following three processes. A first process is to apply the crosslinkable resin composition, which contains resin components and organic solvents, to electrically insulating film using a coating machine such as a reverse roll coater or comma coater, a second process is to heat the electrically insulating film coated with the crosslinkable resin composition for 1 to 15 minutes at 60 to 170°C by using a heater to volatilize the solvent from the electrically insulating film, thereby B-staging the crosslinkable resin composition, and a third process is to thermo-compress metal foil onto the adhesive (crimping pressure of 2 to 200 N/cm and crimping temperature of 40 to 200°C are preferable) by using a heated roll or the like on the electrically insulating film in which the crosslinkable resin composition has been B-staged. If sufficient adhesive performance is obtained by going through the above three processes, the process may be terminated here, but if complete adhesive performance is required, it is preferable to further post-cure the composition under the condition of 100 to 200°C for 1 to 24 hours. The thickness of the resin composition layer after being finally cured is preferably in the range of 5 to 100 µm.

### 5. Build-up Substrate

The method for obtaining a build-up substrate from the composition of the present invention includes, for example, the following processes. First, the composition appropriately containing a rubber, filler, and the like is applied to a circuit board having a circuit formed thereon by spray coating, curtain coating, or the like, and then cured (process 1). Thereafter, if necessary, holes such as predetermined through-hole portion are drilled, the surface is then treated with a roughening agent, and the surface is washed with hot water to form irregularities, and then plated with a metal such as copper (process 2). This procedure is repeated as desired to alternately build up resin insulating layers and conductor layers of a predetermined circuit pattern (process 3). Drilling of the through-hole portion is performed after the formation of the outermost resin insulating layer. Alternatively, the build-up substrate of the present invention can be produced by forming a roughened surface by semi-curing the resin composition on the copper foil, and then heat-pressing the obtained resin-coated copper foil onto a wiring board having a circuit formed thereon at 170 to 300°C, thereby eliminating the need for a plating process.

### 6. Build-up Film

A build-up film can be obtained from the composition of the present invention by applying the composition to the surface of a support film (Y) as a base material, and then drying the organic solvent by heating or blowing hot air or the like to form a layer (X) of the composition.

As the organic solvent used here, it is preferable to use, for example, ketones such as acetone, methyl ethyl ketone, and cyclohexanone, acetic acid esters such as ethyl acetate, butyl acetate, cellosolve acetate, propylene glycol monomethyl ether acetate, and carbitol acetate, carbitols such as cellosolve and butylcarbitol, aromatic hydrocarbons such as toluene and xylene, dimethylformamide, dimethylacetamide, and N-methylpyrrolidone, and it is preferable to use in a ratio of a non-volatile content of 30 to 60% by mass.

The thickness of the layer (X) to be formed is generally equal to or larger than the thickness of the conductor layer. The thickness of the conductor layer of the circuit board is usually in the range of 5 to 70 µm, and thus, the thickness of the resin composition layer is preferably 10 to 100 µm. The layer (X) of the composition of the present invention may be protected by a protective film to be described later. Protection with a protective film prevents dust and other debris from adhering to or scratching the surface of the resin composition layer.

Examples of the support film and the protective film described above include polyolefins such as polyethylene, polypropylene, and polyvinyl chloride, polyesters such as polyethylene terephthalate (hereinafter, abbreviated as "PET") and polyethylene naphthalate, polycarbonate, polyimide, release paper, and metal foils such as copper foil and aluminum foil. The support film and the protective film may be subjected to a releasing treatment in addition to a mat treatment and a corona treatment. The thickness of the support film is not particularly limited, but is usually from 10 to 150 µm, preferably from 25 to 50 µm. Alternatively, the thickness of the protective film is preferably 1 to 40 µm.

The above-described support film (Y) is peeled after lamination onto a circuit board or after forming an insulating layer by heating and curing. When the support film (Y) is peeled after the curable resin composition layer constituting the build-up film is cured by heating, adhesion of dust or the like in the curing process can be prevented. In the case of peeling after curing, the support film is usually subjected to a releasing treatment in advance.

A multilayer printed circuit board can be produced by using the build-up film obtained as described above. For example, when the layer (X) is protected with a protective film, the film is peeled off and then the layer (X) is laminated onto one or both sides of the circuit board so as to be in direct contact with the circuit board, for example, by a vacuum lamination method. The lamination method may be batch or continuous on rolls. Alternatively, if necessary, the build-up film and the circuit board may be heated (preheated), as necessary, before lamination. The conditions for lamination are preferably as follows: the crimping temperature (lamination temperature) is preferably 70 to 140°C, and the crimping pressure is preferably 1 to 11 kgf/cm2 (9.8 × 10⁴ to 107.9 × 10⁴ N/m²), and it is preferable to laminate under reduced air pressure of 20 mmHg (26.7 hPa) or less.

### 7. Conductive Paste

Examples of the method for obtaining the conductive paste from the composition of the present invention include a method for dispersing conductive particles in the composition. The conductive paste can be a circuit connection paste resin composition or an anisotropic conductive adhesive depending on the type of conductive particles used.

### Examples

Hereinafter, the present invention will be described in detail by means of examples and comparative examples, and in the following, "part" and "%" are on a mass basis unless otherwise specified. The present invention is not limited thereto.

¹H and ¹³C-NMR, FD-MS spectra and GPC were measured under the following conditions.
¹H-NMR: "JNM-ECA600" manufactured by JEOL RESONANCE
Magnetic field intensity: 600 MHz
Number of times of integration: 32
Solvent : CDCl₃, DMSO-d₆
Sample concentration: 30% by mass
¹³C-NMR: "JNM-ECA600" manufactured by JEOL RESONANCE
Magnetic field intensity: 150 MHz
Number of times of integration: 320
Solvent: DMSO-d₆
Sample concentration: 30% by mass
FD-MS: "JMS-T100GC AccuTOF" manufactured by JOEL, Ltd.
Measurement range: m/z = 50.00 to 2000.00
Rate of change: 25.6 mA/min
Final current value: 40 mA
Cathode voltage: -10 kV
GPC: "HLC-8320GPC" manufactured by Tosoh Corporation
Column: "TSK-GEL G2000HXL" + "TSK-GEL G3000HXL" + "TSK-GEL G4000HXL" manufactured by Tosoh Corporation
Detector: RI (differential refractometer)
Measuring conditions: 40°C
Mobile phase: tetrahydrofuran
Flow rate: 1 ml/min
Standard: "PStquick A", "PStquick B", "PStQuick E", and "PStquick F" manufactured by Tosoh Corporation

The epoxy equivalent weight of the synthesized epoxy resin was measured in accordance with JIS K7236, and the epoxy equivalent weight (g/eq) was calculated.

The number of repeating units can be calculated, for example, from the results of GPC molecular weight measurement, or various appropriate instrumental analyses such as FD-MS and NMR.

### Synthesis Example 1

A flask equipped with a thermometer and a stirrer was charged with 151 g (1.0 mol) of 4-hydroxyacetanilide, 300 g of acetone, and 166 g (1.2 mol) of potassium carbonate, and the temperature was raised to the reflux temperature. Thereafter, 144 g (1.2 mol) of allyl bromide was added dropwise and, followed by reaction for another 7 hours. After cooling to room temperature, the potassium carbonate was removed by filtration, and acetone and excess allyl bromide in the filtrate were distilled off under reduced pressure by using an evaporator. The obtained solid was dissolved in 222 g of ethanol, and 417 g (4.0 mol) of 35% aqueous hydrochloric acid solution was added dropwise, followed by reaction at 60 to 65°C for 12 hours. After cooling to room temperature, the mixture was neutralized with 880 g (4.4 mol) of 20% aqueous sodium hydroxide solution, and then separated three times with 373 g of ethyl acetate. After the organic layer was dehydrated with sodium sulfate, ethyl acetate was distilled off under reduced pressure by using an evaporator to obtain 113 g (0.76 mol, yield: 76%) of 4-allyloxy aniline.

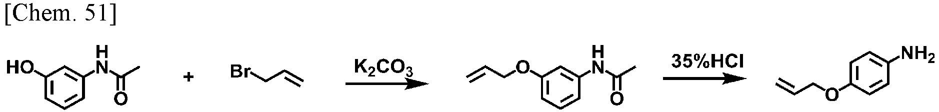

Into a flask equipped with a thermometer and a stirrer, 82 g (0.84 moles) of maleic anhydride was added and dissolved in 1590 g toluene and 9.0 g DMF. While keeping the temperature below 10°C in an ice bath, 113 g (0.76 mol) of 4-allyloxyaniline dissolved in 150 g of DMF was added dropwise, followed by reaction at 25°C for 2 hours. Subsequently, 10 g of 95% sulfuric acid was added and reacted at 110°C for 5 hours, after which toluene was distilled off under reduced pressure. 1200 g of ethyl acetate was added and separation was carried out three times, and then separation was carried out twice with a 2% aqueous sodium hydrogen carbonate solution. After the organic layer was dehydrated with sodium sulfate, ethyl acetate was distilled off under reduced pressure by using an evaporator to obtain 150 g (0.66 mol, yield 87%) of N-allyloxyphenylmaleimide.

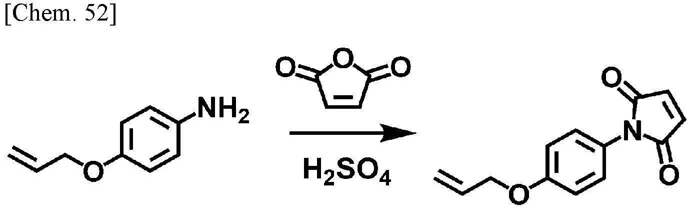

Into a flask equipped with a thermometer and a stirrer, 150 g (0.66 mol) of N-allyloxyphenylmaleimide and 3390 g of dichloromethane were charged and dissolved. While keeping the temperature below 10°C in an ice bath, 1200 g of m-chlorobenzoic acid was added dropwise, followed by reaction at room temperature for 72 hours. After neutralization with 402 g of saturated aqueous sodium bicarbonate solution, 403 g of saturated aqueous sodium sulfite solution was added dropwise. After removing the aqueous layer, the solution was separated three times with 2% aqueous sodium bicarbonate solution and four times with water. After the organic layer was dehydrated with sodium sulfate, dichloromethane was distilled off under reduced pressure by using an evaporator to obtain 60 g (0.24 mol, yield 37%) of a brown solid. The epoxy equivalent weight was 299. ¹H-NMR analysis confirmed that 4-glycidyloxyphenylmaleimide (M-1) was obtained. ¹H-NMR (DMSO-d₆, 600 MHz): 2.72-2.87 (m, 2H), 3.84-3.90 (m, 1H), 4.36 (dd, 1H), 4.39 (dd, 1H), 7.1 6 (s, 2H), 7.03-7.30 (m, 4H).

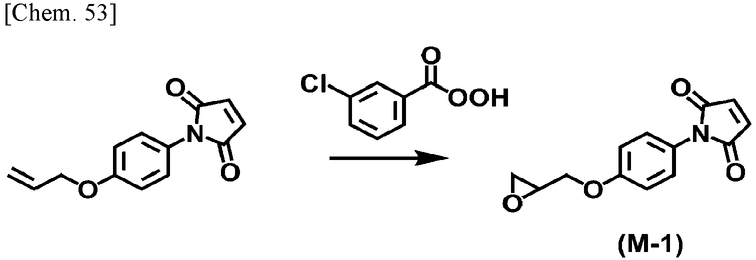

### Synthesis Example 2

A flask equipped with a thermometer and a stirrer was charged with 445 g (0.5 mol) of diglycidyl ether of polytetramethylene glycol ("Denacol EX-991L" manufactured by Nagase ChemteX: epoxy equivalent weight of 445 g/eq) and 76 g (0.33 mol) of bisphenol A (hydroxyl group equivalent 114 g/eq), and the temperature was raised to 140°C over 30 minutes, after which 2.6 g of 4% aqueous sodium hydroxide solution was added. Thereafter, the temperature was then increased to 150°C over 30 minutes, followed by reaction at 150°C for another 7 hours. Subsequently, 61 g (0.32 mol) of 2-hydroxyanthracene was added and reacted at 150°C for 12 hours to obtain anthracene compound (A-1). The compound was confirmed to be the target compound because the mass spectrum showed a peak of M+ = 1454, corresponding to the theoretical structure of m₁ = 1 and n₁ = 4 in the following formula.

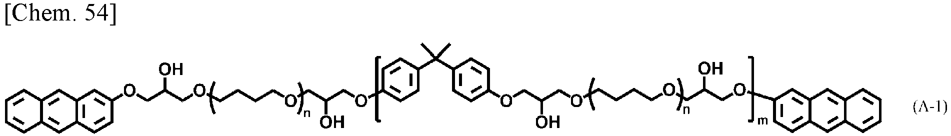

### Example 1

110 g (0.37 mol) of the glycidyloxyphenylmaleimide obtained in Synthesis Example 1, 2620 g of methyl isobutyl ketone, and 2620 g of toluene were charged into 582 g of the anthracene compound of Synthesis Example 2 in a flask equipped with a thermometer and a stirrer, and the mixture was reacted at 80°C for 17 hours to obtain a Diels-Alder reaction product (D-1). The epoxy equivalent weight was 1843. The compound was confirmed to be the target compound because the mass spectrum showed a peak of M+ = 1944, corresponding to the theoretical structure of m₁ = 1 and n₁ = 4 in the following formula.

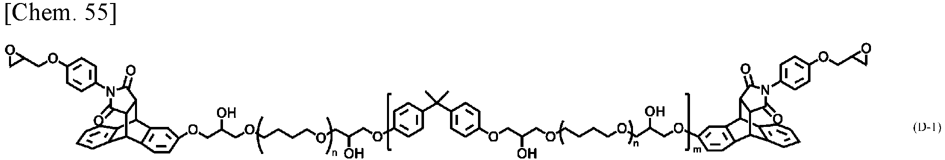

### Synthesis Example 3

Into a flask equipped with a thermometer, a dropping funnel, a condenser, and a stirrer, 180 ml of dimethylacetamide was added to 38.8 g (0.20 mol) of 9-anthrone under a nitrogen atmosphere while being immersed in an ice bath to prepare a slurry, and then 110 g (0.22 mol) of 8.09% aqueous sodium hydroxide solution was added thereto. Next, 27.7 g (0.20 mol) of epibromohydrin dissolved in 40 ml of dimethylacetamide was added dropwise. After stirring for 1 hour, the ice bath was removed, 70 g of water was added, and the precipitated solid was separated by filtration and dried under reduced pressure to obtain 23.6 g (47% yield) of solid. ¹H-NMR analysis confirmed that 9-(2-glycidyloxy)anthracene (A-2) was obtained. ¹H-NMR (CDCl₃, 600 MHz): δ = 2.79-2.96 (m, 2H), 3.54-3.62 (m, 1H), 4.17 (dd, 1H), 4.50 (dd, 1H), 7.40 -7.56 (m, 4H), 7.94-8.05 (m, 2H), 8.24 (s, 1H), 8.30-8.42 (m, 2H).

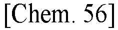

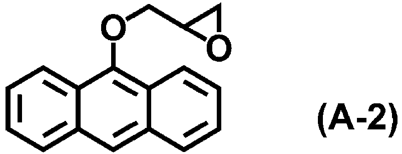

### Example 2

Into a flask equipped with a thermometer, a stirrer, and a condenser, 12.5 g (0.05 mol) of 9-(2-glycidyloxy)anthracene obtained in Synthesis Example 3, 6.6 g (0.02 mol) of 1,6'-bismaleimide-(2,2,4-trimethyl)hexane (BMI-THM manufactured by Daiwa Kasei Kogyo Co., Ltd.), 62.5 g of methyl isobutyl ketone, and 62.5 g of toluene were charged, and after replacing with nitrogen, reacted at 100°C for 7 hours. Thereafter, the precipitate was removed by filtration, and the organic solvent in the filtrate was distilled off under reduced pressure at 50°C. Subsequently, drying was performed at 80°C under reduced pressure to obtain 34.1 g of a Diels-Alder reaction product (D-2). The obtained Diels-Alder reaction product (D-2) had an epoxy equivalent weight of 438 g/eq. In the Diels-Alder reaction product (D-2), a peak of M + = 819 was obtained in the mass spectrum, confirming the production of the target compound.

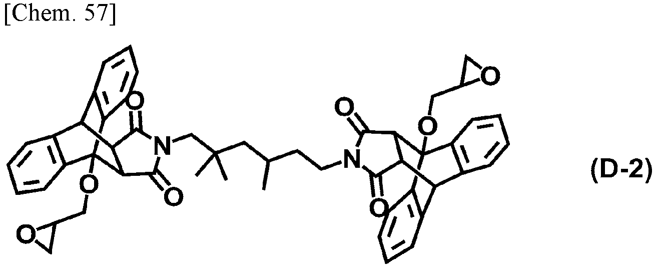

### Example 3

The reaction was carried out in the same manner as in Example 2, except that 6.6 g (0.02 mol) of 1,6'-bismaleimide-(2,2,4-trimethyl)hexane (BMI-THM, manufactured by Daiwa Chemical Industry Co., Ltd.) was changed to 7.4 g (0.02 mol) of 4,4'-diphenylmethane bismaleimide (BMI-1000, manufactured by Daiwa Chemical Industry Co., Ltd.), and 34.3 g of a Diels-Alder reaction product (D-3) was obtained. The resulting Diels-Alder reaction product (D-3) had an epoxy equivalent weight of 495 g/eq. In the Diels-Alder reaction product (D-3), a peak of M + = 859 was obtained in the mass spectrum, confirming the production of the target compound.

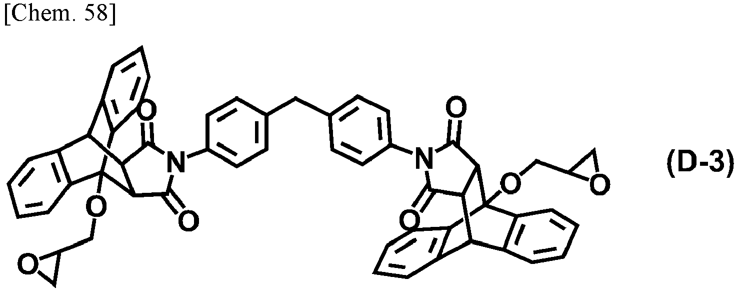

### Synthesis Example 4

An anthracene modified product (A-3) with the following structure was synthesized according to a previous report (Scientific Reports, 10(1), 20214 (2020)).

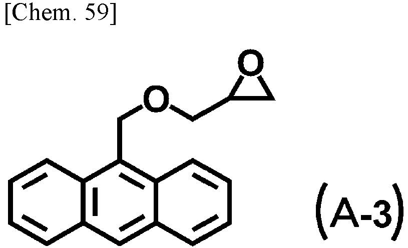

### Example 4

Into a flask equipped with a thermometer, a stirrer, and a condenser, 26.4 g (0.1 mol) of the anthracene modified product (A-3) obtained in Synthesis Example 4, 15.9 g (0.05 mol) of 1,6'-bismaleimide-(2,2,4-trimethyl)hexane (BMI-THM manufactured by Daiwa Chemical Industry Co., Ltd.), and 169.4 g of methyl isobutyl ketone were charged, and after replacing with nitrogen, reacted at 110°C for 7 hours. Thereafter, the temperature was raised to 150°C, and methyl isobutyl ketone was distilled off under reduced pressure, and the mixture was cooled to room temperature to obtain 41.8 g of a Diels-Alder reaction product (D-4). The obtained Diels-Alder reaction product (D-4) had an epoxy equivalent weight of 435 g/eq. In the Diels-Alder reaction product (D-4), a peak of M + = 847 was obtained in the mass spectrum, confirming the production of the target compound.

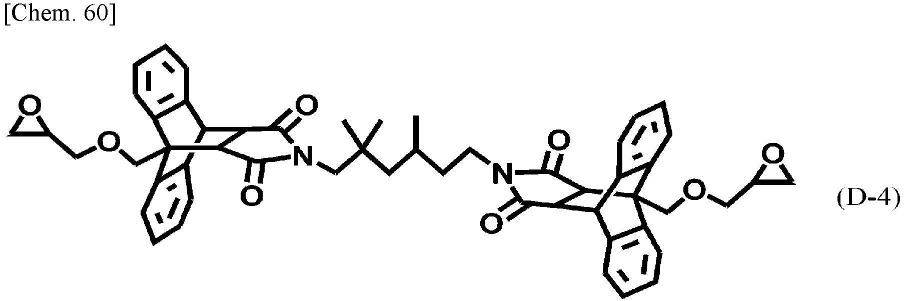

### Synthesis Example 5

A disulfide bond-containing epoxy resin (S-1) with the following structure was synthesized according to a previous report (Polymer, 82, 319 (2016)).

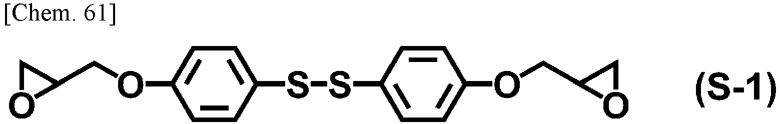

### Synthesis Example 6

Into a flask equipped with a thermometer and a stirrer, 445 g (0.5 mol) of diglycidyl ether of polytetramethylene glycol ("Denacol EX-991L" manufactured by Nagase ChemteX: epoxy equivalent weight of 445 g/eq) and 190 g (0.76 mol) of 4,4'-dihydroxydiphenyl disulfide (hydroxyl group equivalent 125 g/eq) were added, and the temperature was raised to 140°C over 30 minutes, after which 3.2 g of a 4% aqueous sodium hydroxide solution was added. Thereafter, the temperature was then increased to 150°C over 30 minutes, followed by reaction at 150°C for another 5 hours. Thereafter, a neutralizing amount of sodium phosphate was added to obtain 635 g of a hydroxy compound (S-2) represented by the following formula. The hydroxy compound was confirmed to be the target compound because the mass spectrum showed a peak of M+ = 1424, corresponding to the theoretical structure of m₁ = 1 and n₁ = 11 in the following formula. The hydroxyl group equivalent of this hydroxy compound (S-2) calculated by GPC was 750 g/eq, the average value of n₁ was 10.6, and the average value of m₁ was 1.09.

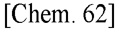

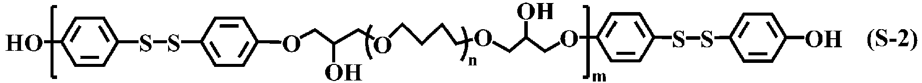

### Example 5

Into a flask equipped with a thermometer, a dropping funnel, a condenser, and a stirrer, 37.5 g (0.025 mol) of the hydroxy compound (S-2) obtained in Synthesis Example 6, 750 mL of DMF, 69 g (0.5 mol) of potassium carbonate, and 69 g (0.5 mol) of epibromohydrin were added and dissolved while purging with nitrogen gas. After the temperature was raised to 60°C, stirring was continued for 20 hours. The mixture was cooled to room temperature, the potassium carbonate was removed by filtration, and 1,000 g each of water and chloroform were added, and separation was carried out three times. The organic layer was dehydrated with sodium sulfate, and the solvent was then distilled off under reduced pressure by using an evaporator to obtain 37.5 g of an epoxy compound (S-3) represented by the following structural formula. The obtained epoxy compound (S-3) had an epoxy equivalent weight of 806 g/eq. The epoxy resin was confirmed to be the target compound because the mass spectrum showed a peak of M+ = 1536, corresponding to the theoretical structure of m₁ = 1 and n₁ = 11 in the following formula.

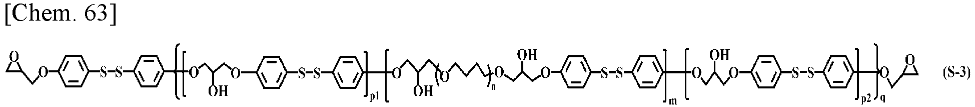

### Synthesis Example 7

Into a flask equipped with a thermometer and a stirrer, 210 g (0.5 mol) of diglycidyl ether of 1,12-dodecanediol (manufactured by Yokkaichi Synthetic Co., Ltd.: epoxy equivalent weight of 210 g/eq) and 145 g (0.58 mol) of 4,4'-dihydroxydiphenyl disulfide (hydroxyl group equivalent 125 g/eq) were added, and the temperature was raised to 140°C over 30 minutes, after which 3.6 g of 4% aqueous sodium hydroxide solution was added. Thereafter, the temperature was then increased to 150°C over 30 minutes, followed by reaction at 150°C for another 5 hours. Thereafter, a neutralizing amount of sodium phosphate was added to obtain 355 g of a hydroxy compound (S-4) represented by the following formula. The hydroxy compound was confirmed to be the target compound because the mass spectrum showed a peak of M+ = 814, corresponding to the theoretical structure in the following formula. The hydroxyl group equivalent of this hydroxy compound (S-4) calculated from GPC was 1076 g/eq.

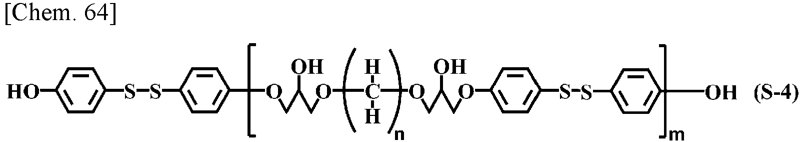

### Example 6

Into a flask equipped with a thermometer, a dropping funnel, a condenser, and a stirrer, 53.8 g (0.025 mol) of the hydroxy compound (S-4) obtained in Synthesis Example 7, 750 mL of DMF, 69 g (0.5 mol) of potassium carbonate, and 69 g (0.5 mol) of epibromohydrin were added and dissolved while purging with nitrogen gas. After the temperature was raised to 60°C, stirring was continued for 20 hours. The mixture was cooled to room temperature, the potassium carbonate was removed by filtration, and 1,000 g each of water and chloroform were added, and separation was carried out three times. The organic layer was dehydrated with sodium sulfate, and the solvent was then distilled off under reduced pressure by using an evaporator to obtain 53.8 g of an epoxy compound (S-5) represented by the following structural formula. The obtained epoxy compound (S-5) had an epoxy equivalent weight of 1020 g/eq. The epoxy resin was confirmed to be the target compound because the mass spectrum showed a peak of M+ = 926, corresponding to the theoretical structure in the mass spectrum.

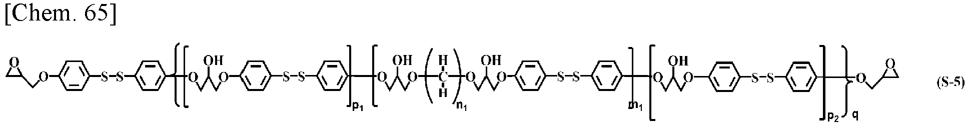

### Synthesis Example 8

A flask equipped with a thermometer and a stirrer was charged with 445 g (0.5 mol) of diglycidyl ether of polytetramethylene glycol ("Denacol EX-991L" manufactured by Nagase ChemteX: epoxy equivalent weight of 445 g/eq) and 171 g (0.75 mol) of bisphenol A (hydroxyl group equivalent 114 g/eq), and the temperature was raised to 140°C over 30 minutes, after which 3.1 g of 4% aqueous sodium hydroxide solution was added. Thereafter, the temperature was then increased to 150°C over 30 minutes, followed by reaction at 150°C for another 16 hours. Thereafter, a neutralizing amount of sodium phosphate was added to obtain 616 g of a hydroxy compound represented by the following formula (Ph-1). The hydroxy compound was confirmed to contain a PTMG (polytetramethylene ether glycol) type (BPA: bisphenol A) hydroxy compound because the mass spectrum showed a peak of M+=1380, corresponding to the theoretical structure of m₁ = 1 and n₁ = 11 in the following formula. The hydroxyl group equivalent of this hydroxy compound (Ph-1) calculated by GPC was 1080 g/eq, the average value of n₁ was 10.6, and the average value of m₁ was 0.76.

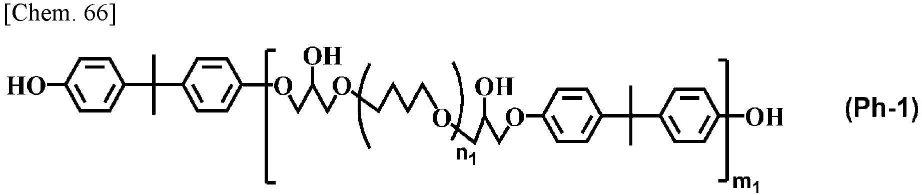

### Synthesis Example 9

Into a flask equipped with a thermometer, a dropping funnel, a condenser, and a stirrer, 200 g of the hydroxy compound Ph-1 obtained in Synthesis Example 5, 437 g (4.72 mol) of epichlorohydrin, and 118 g of n-butanol were added and dissolved while purging with nitrogen gas. After the temperature was raised to 65°C, the pressure was reduced to an azeotropic pressure, and 6.66 g (0.08 mol) of 49% aqueous sodium hydroxide solution was added dropwise over 5 hours.

Next, stirring was continued for 0.5 hours under the same conditions. During the reaction, the distillates from the azeotropic distillation were separated with a Dean Stark trap to remove the aqueous phase, while the oil phase was returned to the reaction system. Thereafter, the unreacted epichlorohydrin was distilled off by vacuum distillation. To the obtained crude epoxy resin, 150 g of methyl isobutyl ketone and 150 g of n-butanol were added and dissolved.

Furthermore, 10 g of a 10% aqueous sodium hydroxide solution was added to this solution, and the mixture was reacted at 80°C for 2 hours, after which washing with 50 g of water was repeated three times until the pH of the cleaning liquid became neutral.

Next, the system was dehydrated by azeotropy, and after undergoing microfiltration, the solvent was distilled off under reduced pressure to obtain 190 g of an epoxy resin (Ep-1). The obtained epoxy resin (Ep-1) had an epoxy equivalent weight of 1192 g/eq. The epoxy resin Ep-1 was confirmed to contain a PTMG-type (BPA) epoxy resin because the mass spectrum showed a peak of M+ = 1492, corresponding to the theoretical structure of m₁ = 1, n₁ = 11, q = 1, p₁ = 0, p₂ = 0 in the following formula.

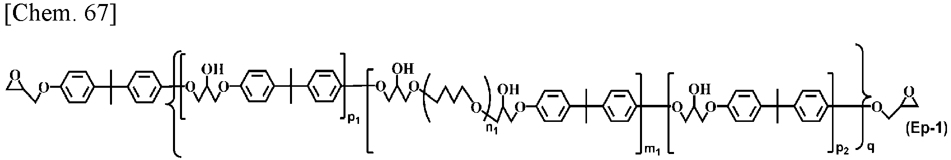

### Production of Composition and Cured Product

The compounds were used according to the formulation (the numbers in the table are based on mass) shown in Tables 1 and 2 and mixed uniformly in a mixer ("Thinner Mixer ARV-200" manufactured by Thinky Corporation) to obtain a curable resin composition. This curable resin composition was sandwiched between mirror-finished aluminum plates ("JIS H 4000 A1050P" manufactured by Engineering Test Services Co., Ltd.) using a silicone tube as a spacer, and cured by heating under predetermined conditions to obtain a cured product having a thickness of 0.7 mm.

### <Remolding Test>

The produced cured product was frozen and pulverized. 0.07 g of the pulverized cured product was placed in a mold measuring 10 mm square and 0.5 mm thick, and vacuum pressed under predetermined conditions. The appearance of the obtained cured product was visually observed. The criteria for determination are as follows.
A: Seams disappeared and cured product was integrated.
B: Seams were partially visible, but the cured product was integrated.
C: The shape was solid and fell apart when light force was applied.

### <Repair Test>

The produced cured product was broken with a razor blade and the resulting fracture surfaces were brought into contact with each other, and then aged for 24 hours at 150°C in a dryer. After the cured product was taken out of the dryer, the presence or absence of bonding between the cross sections of the cured product was visually determined. The criteria for determination are as follows.
A: The surfaces are bonded and the bonded portions are not dissociated when the cured product is bent by 90°.
B: The surfaces are bonded but the bonded portions are dissociated when the cured product is bent.
C: The surfaces were not bonded.

**[Table 1]**

| | | Example 7 | Example 8 | Example 9 | Example 10 | Comparative Example 1 |
|---|---|---|---|---|---|---|
| Epoxy Resin | EPICL0N850S | 50.0 | 50.0 | 50.0 | 50.0 | |
| | Compound (D-1) synthesized in Example 1 | 50.0 | | | | |
| | Compound (D-2) synthesized in Example 2 | | 50.0 | | | |
| | Compound (D-3) synthesized in Example 3 | | | 50.0 | | |
| | Compound (D-4) synthesized in Example 4 | | | | 50.0 | |
| | Compound (Ep-1) synthesized in Synthesis Example 9 | | | | | 100.0 |
| Curing Agent | DICY | 6.2 | 8.0 | 7.7 | 8/0 | 1.8 |
| Curing Accelerator | DCMU | 0.85 | 0.85 | 0.85 | 0.85 | 0.85 |
| Curing Conditions | Temperature/Time | 170°C/1 hour | | | | |
| Remolding Test | Vacuum Pressing Conditions Temperature/Time/Pressure | 150°C/4 hours/10 MPa | | | | |
| | Determination | A | A | B | A | C |
| Repair Test | Aging Conditions Temperature/Time | 150°C/24 hours | | | | |
| | Determination | A | A | B | A | C |

**[Table 2]**

| | | Example 11 | Example 12 | Example 13 | Example 14 | Comparative Example 2 | Comparative Example 3 |
|---|---|---|---|---|---|---|---|
| Epoxy Resin | EPICLON850S | 32.9 | 32.9 | 32.9 | 32.9 | | 100.0 |
| | Compound (S-3) synthesized in Example 5 | 67.1 | 67.1 | | | | |
| | Compound (S-5) synthesized in Example 6 | | | 67.1 | 67.1 | | |
| | Compound (Ep-1) synthesized in Synthesis Example 9 | | | | | 100.0 | |
| Curing Agent | DICY | 5.4 | | 5.1 | | | |
| | 4,4'-diaminodiphenylsulfone | | 16.0 | | 14.9 | 5.2 | 33.0 |
| Curing Accelerator | DCMU | 0.85 | | 0.85 | | | |
| Curing Conditions | Temperature/Time | 170°C/ 1 hour | 150°C/1 hour + 180°C/3 hours | 170°C/ 1 hour | 150°C/1 hour + 180°C/3 hours | 150°C/1 hour + 180°C/3 hours | 150°C/1 hour + 180°C/3 hours |
| Remolding Test | Vacuum Pressing Conditions Temperature/Time/ Pressure | 180°C/20 minutes/20 MPa | | | | | 150°C/4 hours/ 10 MPa |
| | Determination | A | A | A | A | C | C |
| Repair Test | Aging Conditions Temperature/Time | 150°C/24 hours | | | | | |
| | Determination | A | A | B | A | C | C |

EPICLON 850-S: BPA-type liquid epoxy resin, epoxy equivalent weight of 188 g/eq
DICY: dicyandiamide
DCMU: 3-(3,4-dichlorophenyl)-1,1-dimethylurea

## Claims

1. A glycidyl ether group-containing compound in which a structural unit A having one or more glycidyl ether groups and a structural unit B different from the structural unit A are linked in A-B-A, wherein
the structural unit A and the structural unit B are bonded by a reversible bond having a dissociation temperature of 120°C or higher.

2. The glycidyl ether group-containing compound according to Claim 1, wherein
the reversible bond is a covalent reversible bond.

3. The glycidyl ether group-containing compound according to Claim 2, wherein
the reversible bond is any one of an addition-type structure by a Diels-Alder reaction, a disulfide bond, an ester bond, a boronic acid ester bond, a hemiaminal bond, an imine bond, an acylhydrazone bond, an olefin metathesis reaction, an alkoxyamine skeleton, and an amide bond, all of which have a dissociation temperature of 120°C or higher.

4. The glycidyl ether group-containing compound according to Claim 3, wherein
the reversible bond is an anthracene-type addition-type structure by a Diels-Alder reaction or a disulfide bond sandwiched between aromatic rings.

5. The glycidyl ether group-containing compound according to Claim 1, wherein
the structural unit B has an alkylene chain or an alkylene ether chain.

6. The glycidyl ether group-containing compound according to Claim 5, wherein
the alkylene chain has 4 to 16 carbon atoms.

7. The glycidyl ether group-containing compound according to Claim 1, wherein
the structural unit B further has a reversible bond that is the same as the reversible bond having a dissociation temperature of 120°C or higher, which is a linkage site between the structural unit A and the structural unit B.

8. A glycidyl ether group-containing compound represented by the following general formula: 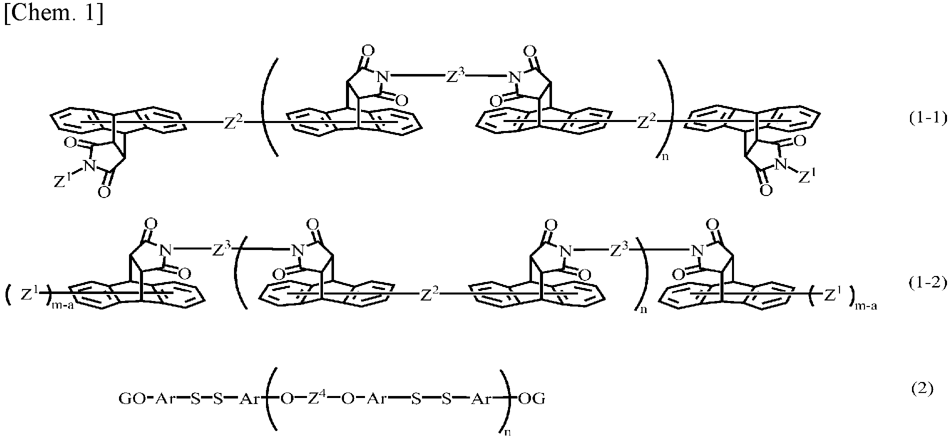 [in the formula (2),
Ar's are each independently a structure containing an unsubstituted aromatic ring or an aromatic ring having a substituent,
an anthracene-derived structure in the formulas (1-1) and (1-2) may have a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group as a substituent,
G is a glycidyl group or a 2-methylglycidyl group,
in the formulas,
m-a is an integer of 1 to 10,
n is an average value of repetition number of 0 to 10, and
Z¹ is any of structures represented by the following formula (3), Z² is any of structures represented by the following formula (4), Z³ is any of structures represented by the following formula (5), and Z⁴ is any of structures represented by the following formula (6) or (7), and the multiple structures present in one molecule may be identical or different from each other, 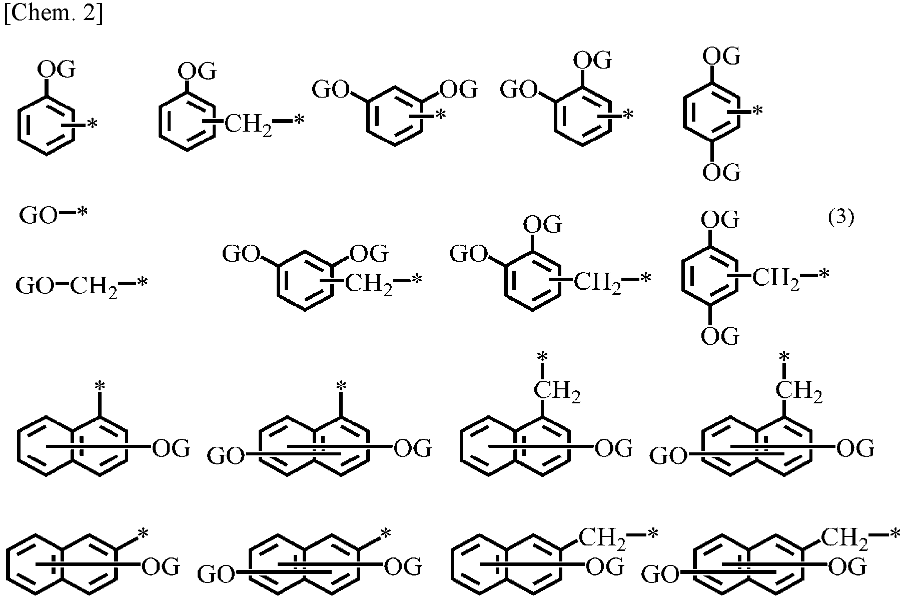
[the aromatic ring in the formula (3) may be substituted or unsubstituted, and * denotes a binding point,
G is the same as above, and
the -OG on the naphthalene ring in the formula indicates that the glycidyl ether group may be bonded at any point], 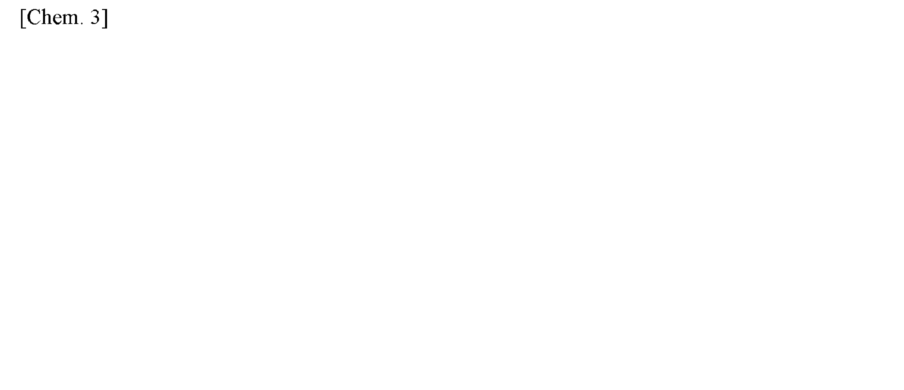 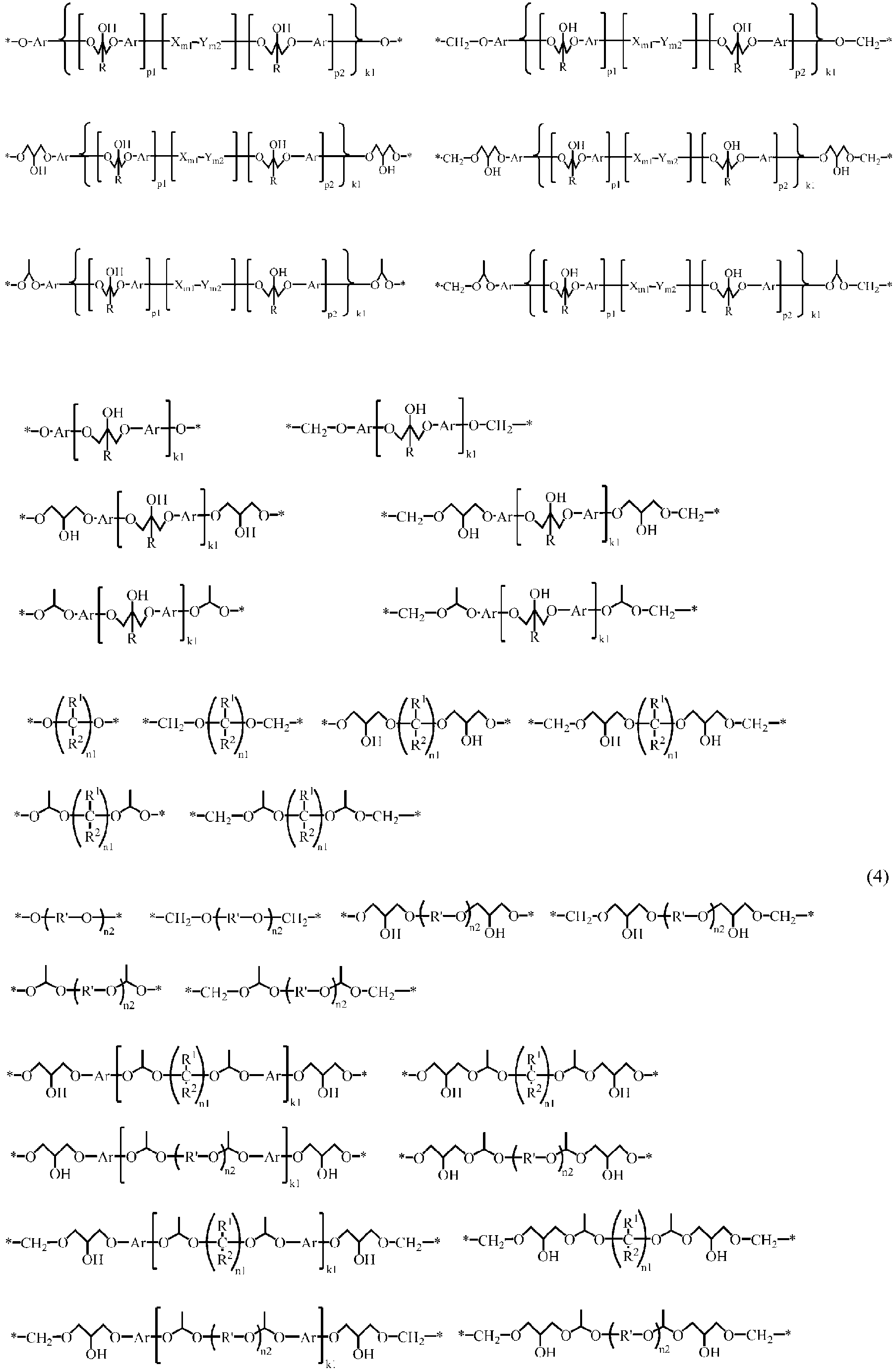
[in the formula (4),
Ar's are each independently a structure containing an unsubstituted aromatic ring or an aromatic ring having a substituent,
R¹ and R² are each independently a hydrogen atom, a methyl group, or an ethyl group,
R is a hydrogen atom or a methyl group,
R' is a divalent hydrocarbon group having 2 to 12 carbon atoms,
n1 is an integer of 2 to 16, and n2 is an average number of repeating units of 2 to 30,
k1 is an average value of repetition number, from 0.5 to 10,
p1 and p2 are each independently 0 to 5, and
X is a structural unit represented by the following formula (4-1) and Y is a structural unit represented by the following formula (4-2), 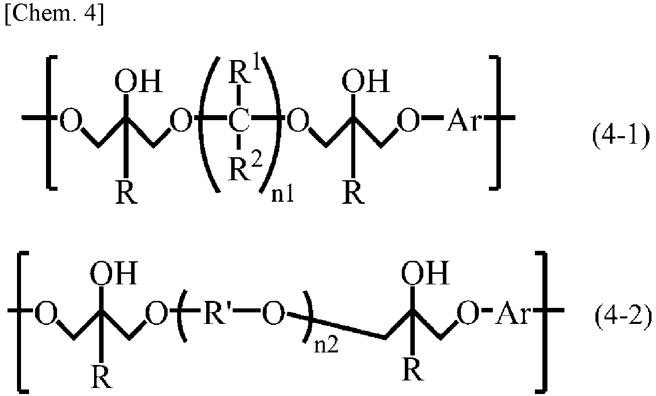
[in the formulas (4-1) and (4-2), Ar, R, R¹, R², R', n1, and n2 are the same as above],
m1 and m2 are an average value of repetition number, each independently 0 to 25, and m1 + m2 ≥ 1,
provided that a bond between the structural unit X represented by the formula (4-1) and the structural unit Y represented by the formula (4-2) may be random or blocked, and the respective total numbers of structural units X and Y present in one molecule are m1 and m2, respectively], 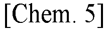 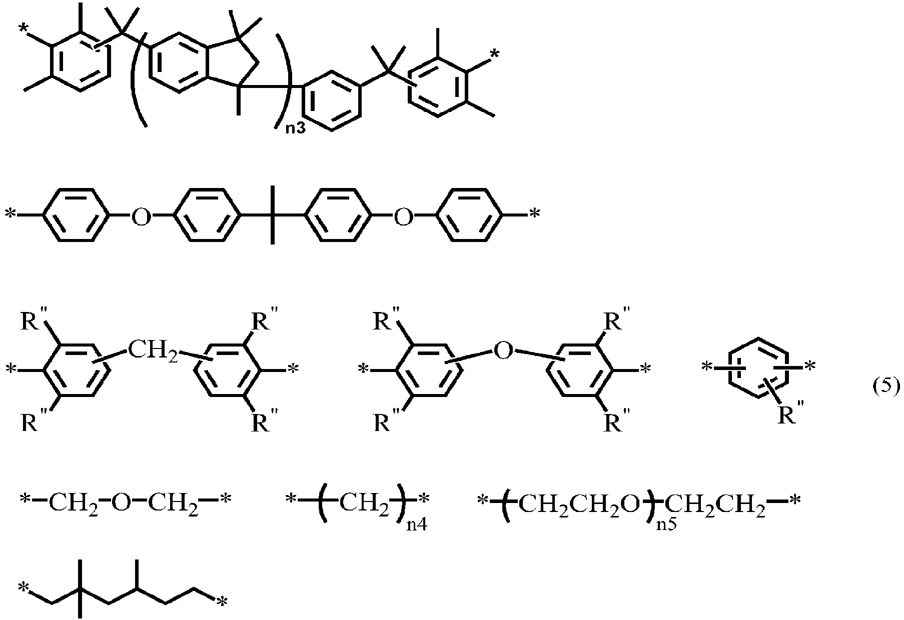
[in the formula (5), n3 and n5 are an average value of repetition number and are each 0.5 to 10, n4 is an integer of 1 to 16, and R"'s are each independently a hydrogen atom, a methyl group, or an ethyl group], 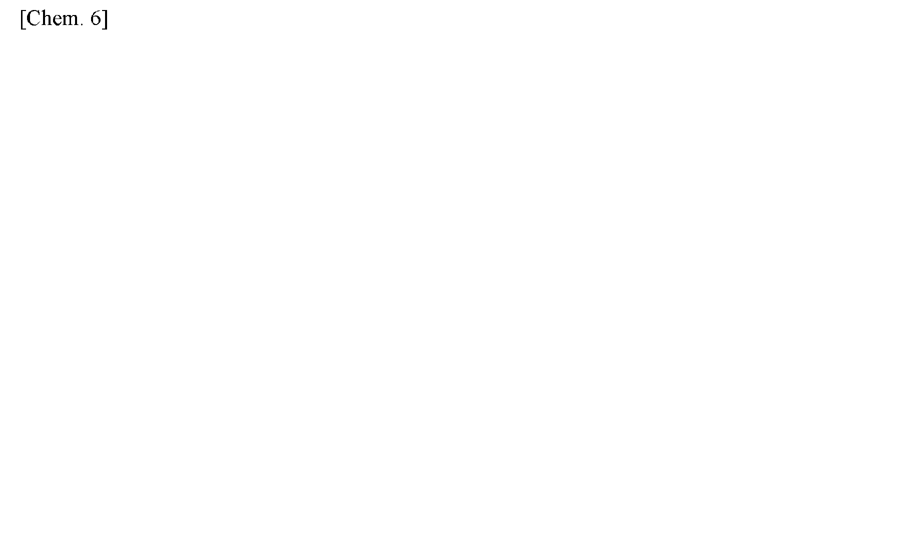 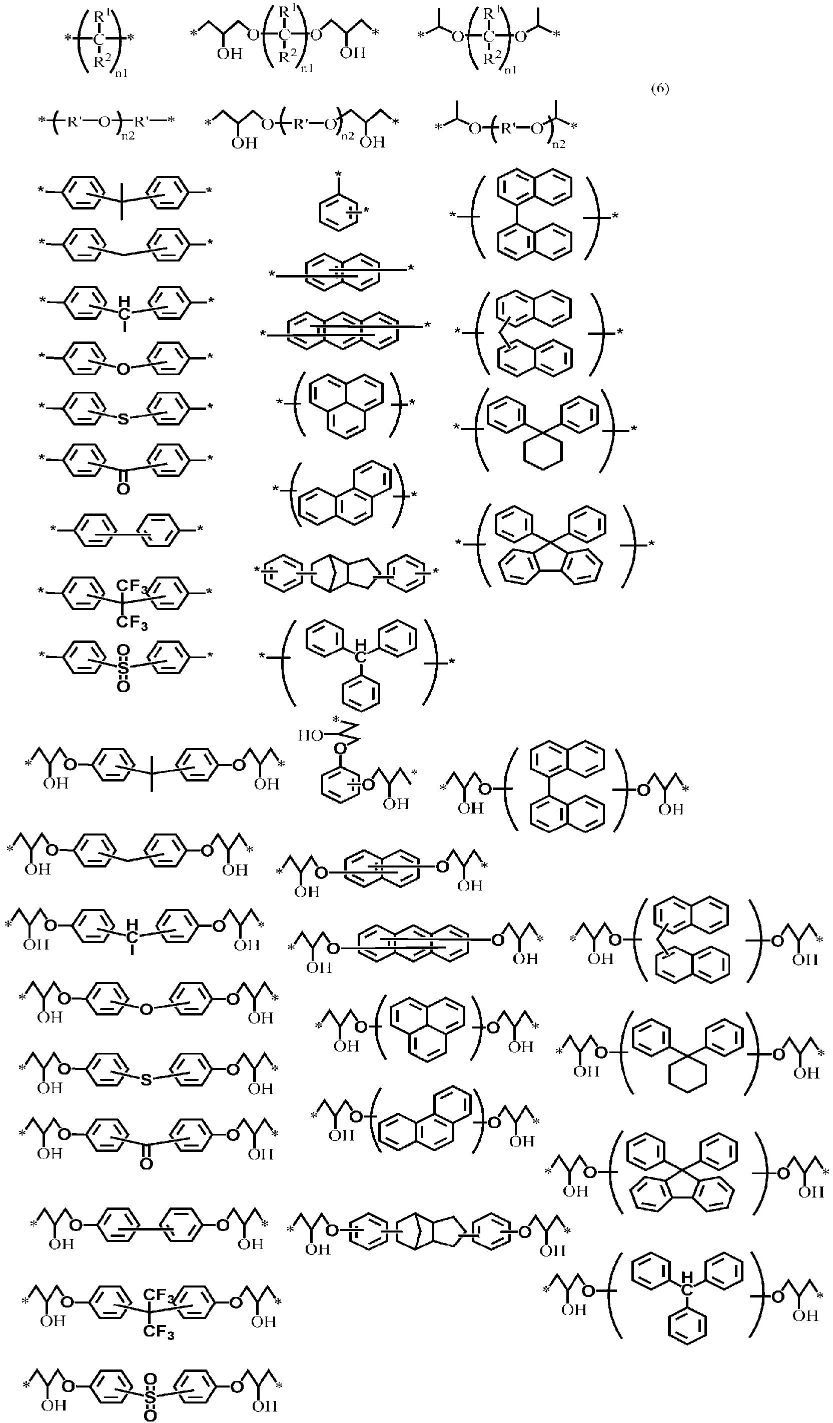 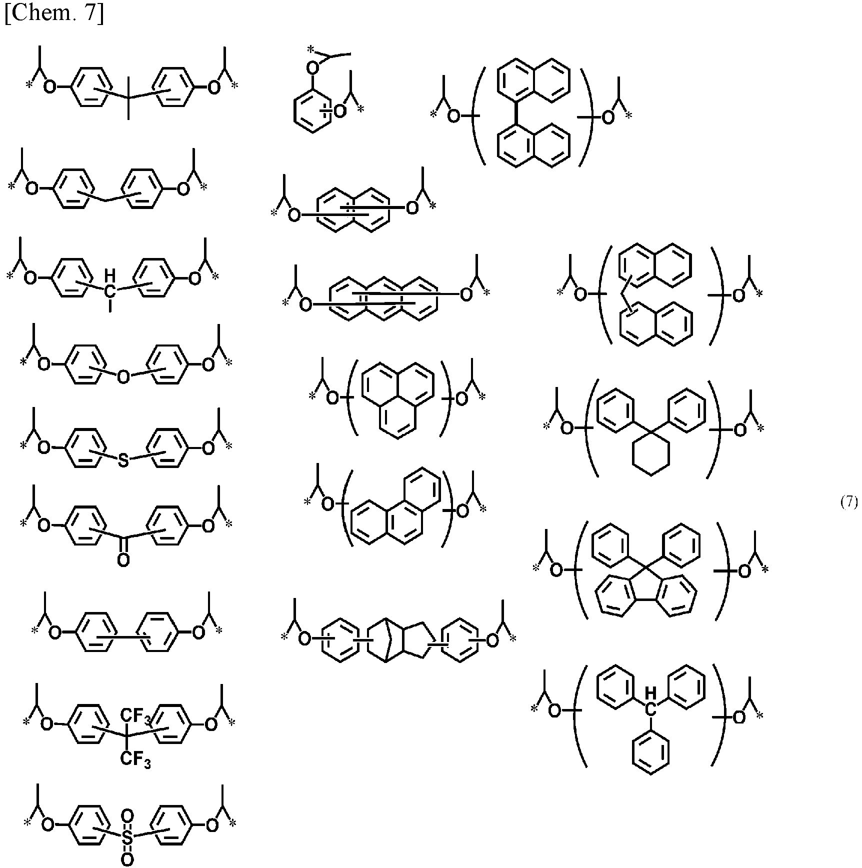
[in the formulas (6) and (7), R¹, R², R', n1, and n2 are the same as above].

9. A curable resin composition comprising, as essential components:
the glycidyl ether group-containing compound according to any one of Claims 1 to 8; and
a compound (I) having reactivity with the glycidyl ether group-containing compound.

10. The curable resin composition according to Claim 9, wherein
the compound (I) having reactivity with the glycidyl ether group-containing compound is a hydroxyl group-containing compound.

11. The curable resin composition according to Claim 10, wherein
the compound (I) having reactivity with the glycidyl ether group-containing compound is a hydroxyl group-containing compound having a reversible bond.

12. The curable resin composition according to Claim 11, wherein
the hydroxyl group-containing compound having the reversible bond is a hydroxyl group-containing compound represented by the following general formula: 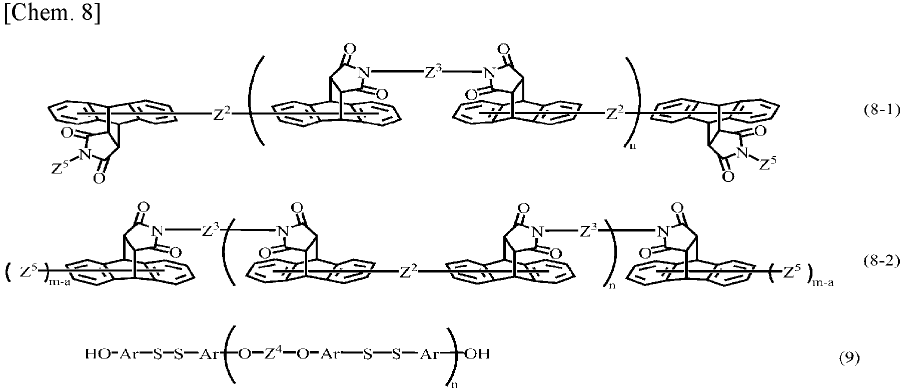
[in the formula (9),
Ar's are each independently a structure containing an unsubstituted aromatic ring or an aromatic ring having a substituent,
an anthracene-derived structure in the formulas (8-1) and (8-2) may have a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group as a substituent,
in the formula,
m-a is an integer of 1 to 10,
n is an average value of repetition number of 0 to 10, and
Z⁵ is any of structures represented by the following formula (10), Z² is any of structures represented by the following formula (11), Z³ is any of structures represented by the following formula (12), and Z⁴ is any of structures represented by the following formula (13) or (14), and the multiple structures present in one molecule may be identical or different from each other, 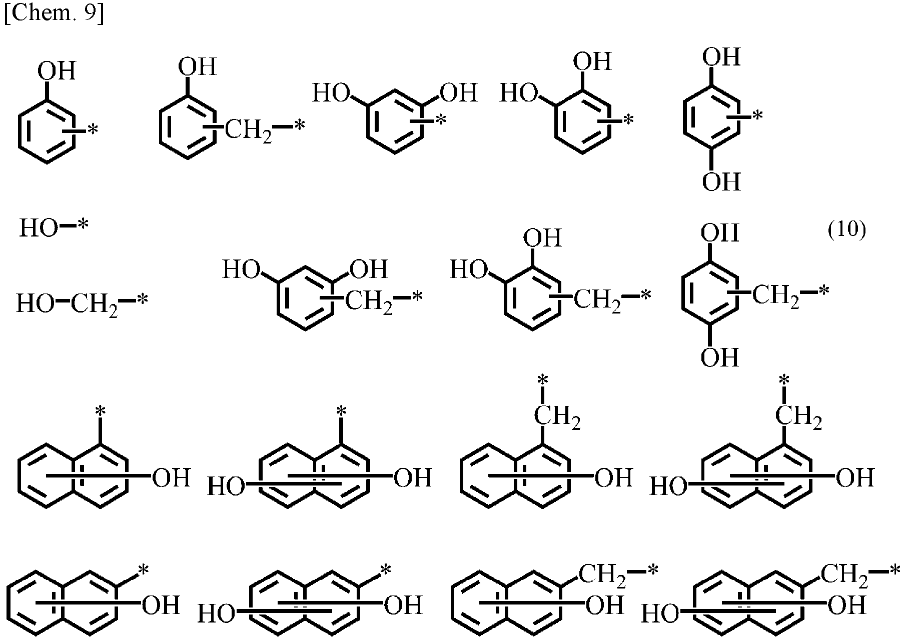
[the aromatic ring in the formula (10) may be substituted or unsubstituted, and * denotes a binding point, the hydroxyl group on the naphthalene ring in the formula indicates that the hydroxyl group may be bonded at any point], 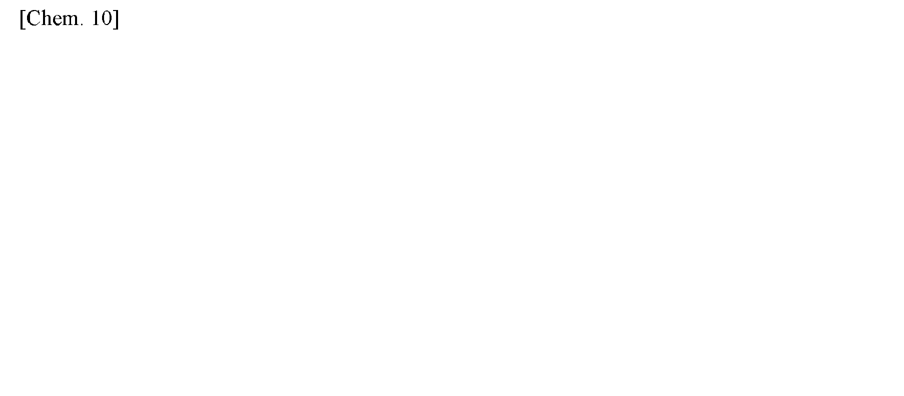 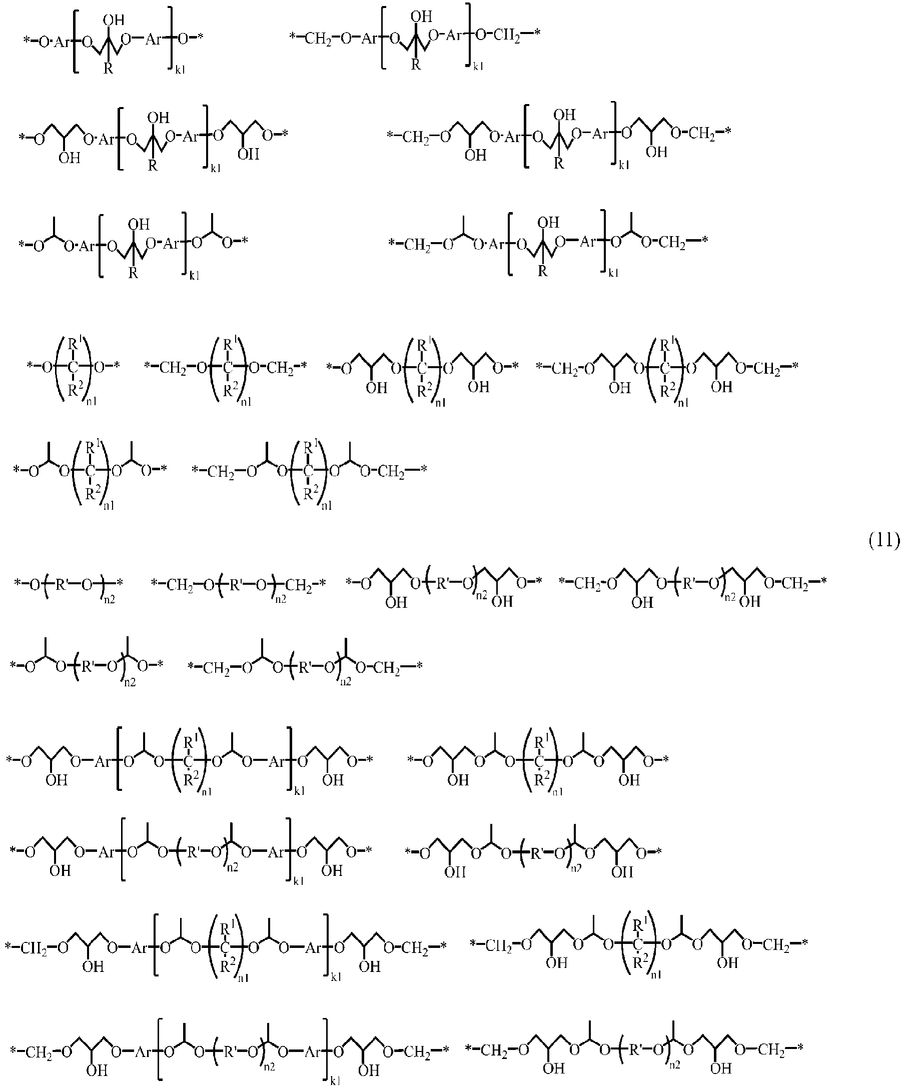
[in the formula (11),
Ar is the same as above,
Ar's are each independently a structure containing an unsubstituted aromatic ring or an aromatic ring having a substituent,
R¹ and R² are each independently a hydrogen atom, a methyl group, or an ethyl group,
R is a hydrogen atom or a methyl group,
R' is a divalent hydrocarbon group having 2 to 12 carbon atoms,
n1 is an integer of 2 to 16, and n2 is an average number of repeating units of 2 to 30,
k1 is an average value of repetition number, from 0.5 to 10,
p1 and p2 are each independently 0 to 5, and
X is a structural unit represented by the following formula (11-1), and Y is a structural unit represented by the following formula (11-2), 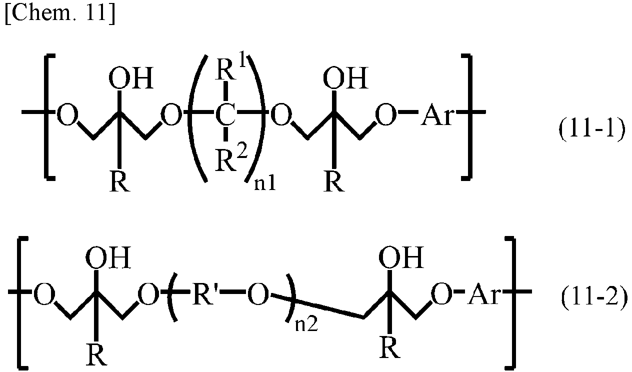
[in the formulas (11-1) and (11-2), Ar, R, R¹, R², R', n1, and n2 are the same as above],
m1 and m2 are an average value of repetition number, each independently 0 to 25, and m1 + m2 ≥ 1,
provided that a bond between the structural unit X represented by the formula (11-1) and the structural unit Y represented by the formula (11-2) may be random or blocked, and the respective total numbers of structural units X and Y present in one molecule are m1 and m2, respectively], 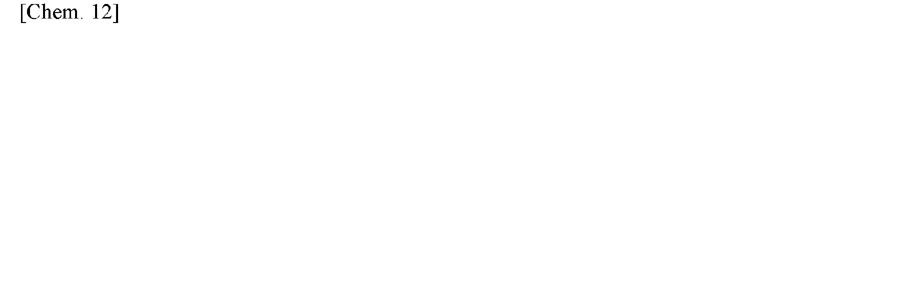 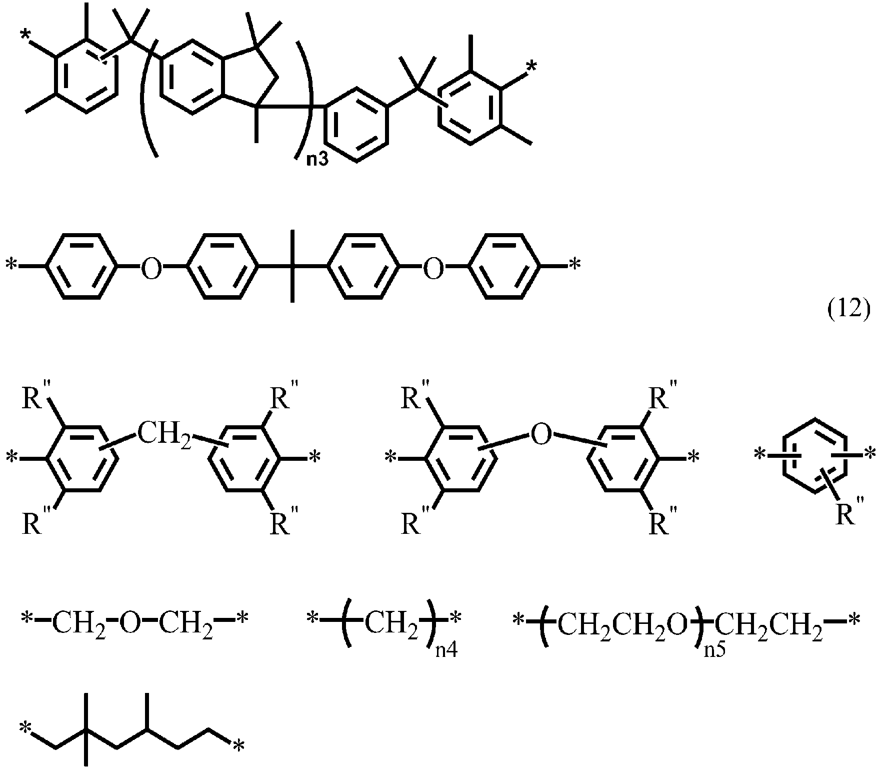
[in the formula (12), n3 and n5 are an average value of repetition number and are each 0.5 to 10, n4 is an integer of 1 to 16, and R"'s are each independently a hydrogen atom, a methyl group, or an ethyl group], 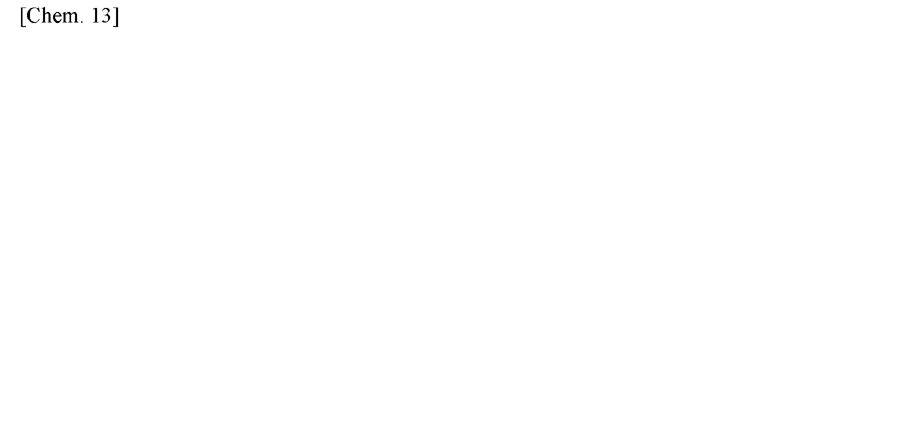 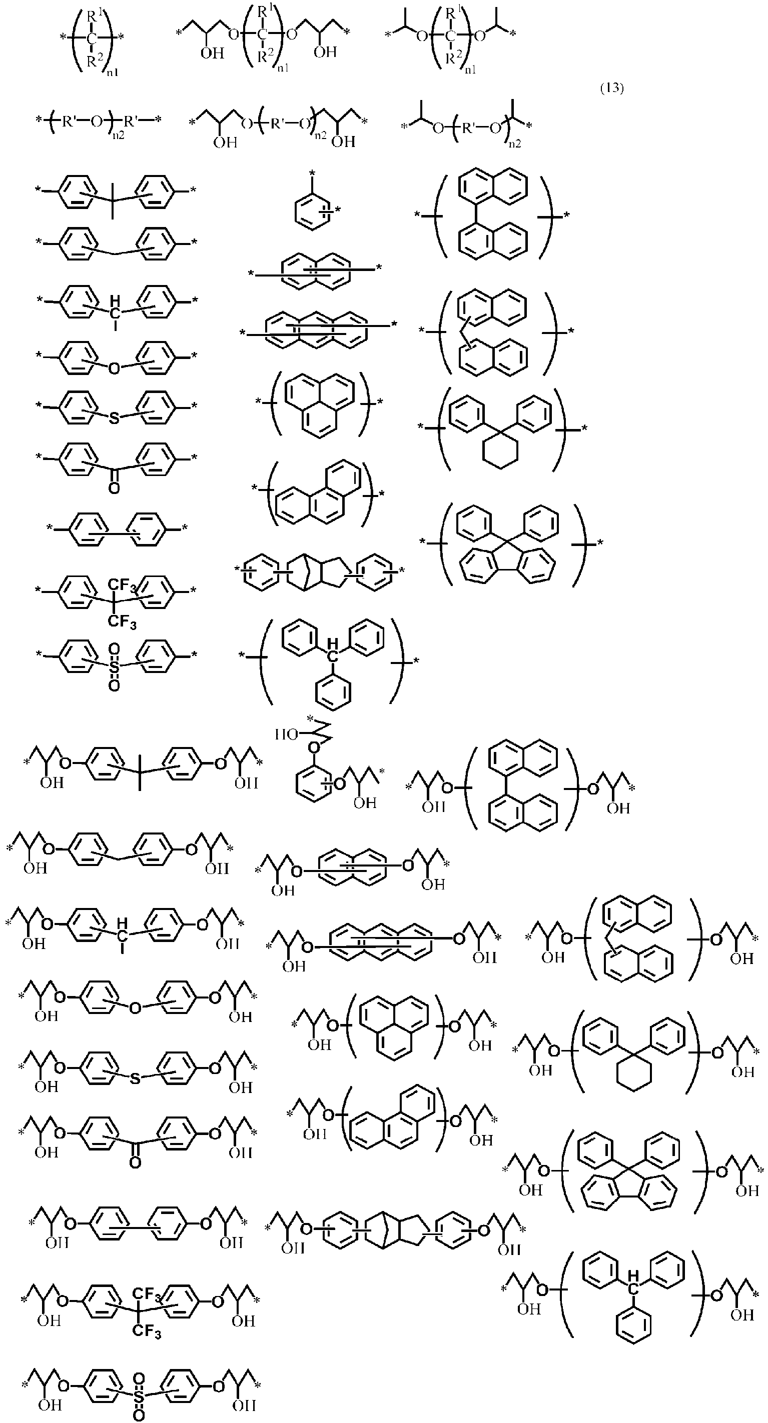 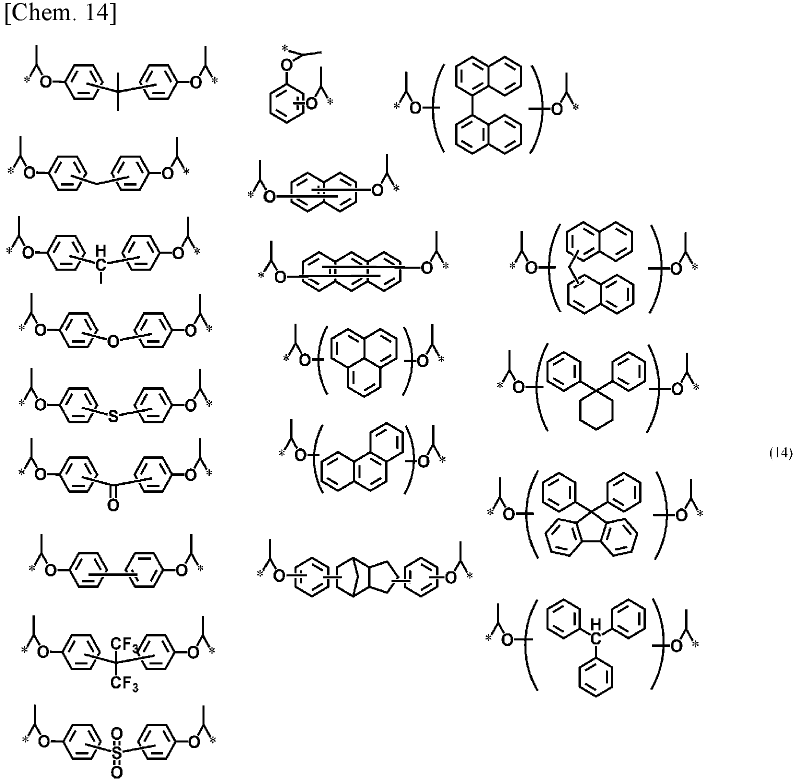
[in the formulas (13) and (14), R¹, R², R', n1, and n2 are the same as above].

13. The curable resin composition according to Claim 9, further comprising:
an epoxy resin with an epoxy equivalent weight of 100 to 10,000 g/eq, other than the glycidyl ether group-containing compound according to any one of Claims 1 to 8.

14. The curable resin composition according to Claim 13, wherein
the epoxy resin is represented by the following formula (15) and has an epoxy equivalent weight of 500 to 10000 g/eq: 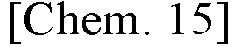 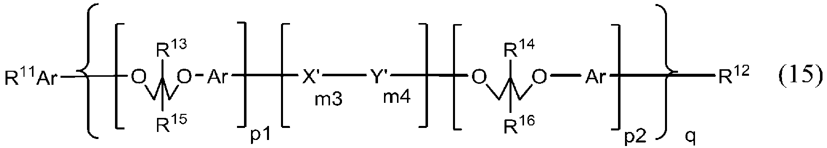
[in the formula (15),
Ar's are each independently a structure containing an unsubstituted aromatic ring or an aromatic ring having a substituent,
X' is a structural unit represented by the following formula (15-1), and Y' is a structural unit represented by the following formula (15-2), 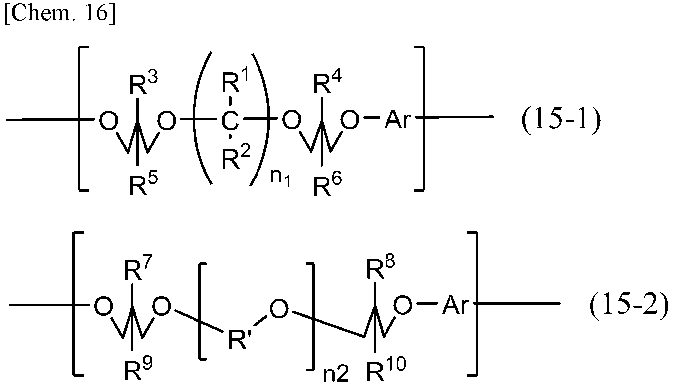
[in the formulas (15-1) and (15-2), Ar is the same as above,
R¹ and R² are each independently a hydrogen atom, a methyl group, or an ethyl group,
R' is a divalent hydrocarbon group having 2 to 12 carbon atoms,
R³, R⁴, R⁷, and R⁸ are each independently a hydroxyl group, a glycidyl ether group, or a 2-methylglycidyl ether group,
R⁵, R⁶, R⁹, and R¹⁰ are each independently a hydrogen atom or a methyl group,
n₁ is an integer of 4 to 16, and
n₂ is an average number of repeating units of 2 to 30]
R¹¹ and R¹² are each independently a glycidyl ether group or a 2-methyl glycidyl ether group,
R¹³ and R¹⁴ are each independently a hydroxyl group, a glycidyl ether group, or a 2-methyl glycidyl ether group,
R¹⁵ and R¹⁶ are hydrogen atoms or methyl groups,
m3, m4, p1, p2, and q are an average value of repetition number,
m3 and m4 are each independently 0 to 25 and m3 + m4 ≥ 1,
p1 and p2 are each independently 0 to 5,
q is 0.5 to 5,
provided that the bond between the structural unit X' represented by the formula (15-1) and the structural unit Y' represented by the formula (15-2) may be random or blocked, and
the respective total numbers of structural units X' and Y' present in one molecule are m3 and m4, respectively).

15. The curable resin composition according to Claim 9, wherein
a concentration of the reversible bond to a total mass of a curable component in the curable resin composition is 0.10 mmol/g or more.

16. The curable resin composition according to Claim 9, wherein
the curable resin composition is a self-healing composition or a composition for a remolding material.

17. A cured product obtained by curing the curable resin composition according to Claim 9.

18. A laminate comprising:
a base material; and
a layer containing the cured product according to Claim 17.

19. A heat-resistant member comprising:
the cured product according to Claim 17.

20. A method for producing a glycidyl ether group-containing compound, the method comprising:
synthesizing a glycidyl ether group-containing compound represented by the formulas (1-1), (1-2) in situ during a process of curing a compound (I) having reactivity with the glycidyl ether group-containing compound, by using an intermediate of a conjugated diene or a parent diene intermediate represented by the following general formula (1-1)', (1-2)': 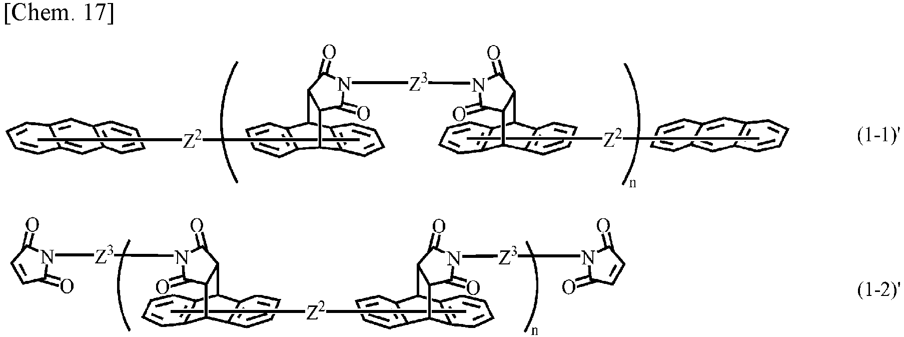
[in the formula, n, Z², and Z³ are the same as above].

21. A cured product obtained by subjecting, to a curing reaction, the formula (1-1)', a maleimide having a glycidyl ether group, and a compound (I) having reactivity with the glycidyl ether group-containing compound as essential raw materials.

22. A cured product obtained by subjecting, to a curing reaction, the formula (1-2)', an anthracene having a glycidyl ether group, and a compound (I) having reactivity with the glycidyl ether group-containing compound as essential raw materials.
